# EUROPEAN PATENT APPLICATION

(11) **EP 4 063 359 A1**
(43) Date of publication of application: **28.09.2022**
(21) Application number: 20889890.8
(22) Date of filing: 17.11.2020
(51) Int. Cl.: C07D 405/14, C07D 409/14, H01L 51/00, H01L 51/50, C09K 11/06

(54) **HETEROCYCLIC COMPOUND, ORGANIC LIGHT EMITTING DEVICE COMPRISING SAME, COMPOSITION FOR ORGANIC LAYER OF ORGANIC LIGHT EMITTING DEVICE, AND METHOD FOR MANUFACTURING ORGANIC LIGHT EMITTING DEVICE**

(30) Priority: 21.11.2019 KR 20190150714
(71) Applicant: LT Materials Co., Ltd., Yongin-si, Gyeonggi-do 17118 (KR)
(72) Inventor: LEE, Young-Jin, Yongin-si, Gyeonggi-do 17118 (KR); MO, Jun-Tae, Yongin-si, Gyeonggi-do 17118 (KR); KIM, Dong-Jun, Yongin-si, Gyeonggi-do 17118 (KR); OH, Han-Kook, Yongin-si, Gyeonggi-do 17118 (KR); BYUN, Ji-Yoon, Yongin-si, Gyeonggi-do 17118 (KR)
(74) Representative: Goddar, Heinz J.
(86) International application number: PCT/KR2020/016200
(87) International publication number: WO 2021/101220

(57) **Abstract**

The present specification provides a heterocyclic compound represented by Chemical Formula 1, an organic light emitting device comprising the same, a composition for an organic material layer of an organic light emitting device, and a method for manufacturing an organic light emitting device.

## Description

### [Technical Field]

This application claims priority to and the benefits of Korean Patent Application No. 10-2019-0150714, filed with the Korean Intellectual Property Office on November 21, 2019, the entire contents of which are incorporated herein by reference.

The present specification relates to a heterocyclic compound, an organic light emitting device comprising the same, a composition for an organic material layer of an organic light emitting device, and a method for manufacturing an organic light emitting device.

### [Background Art]

An organic electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

Studies on an organic light emitting device comprising a compound capable of satisfying conditions required for materials usable in an organic light emitting device, for example, satisfying proper energy level, electrochemical stability, thermal stability and the like, and having a chemical structure capable of performing various roles required in an organic light emitting device depending on substituents have been required.

### Prior Art Documents

### Patent Documents

US Patent No. 4,356,429

### [Disclosure]

### [Technical Problem]

The present application relates to a heterocyclic compound, an organic light emitting device comprising the same, a composition for an organic material layer of an organic light emitting device, and a method for manufacturing an organic light emitting device.

### [Technical Solution]

One embodiment of the present application provides a heterocyclic compound represented by the following Chemical Formula 1.

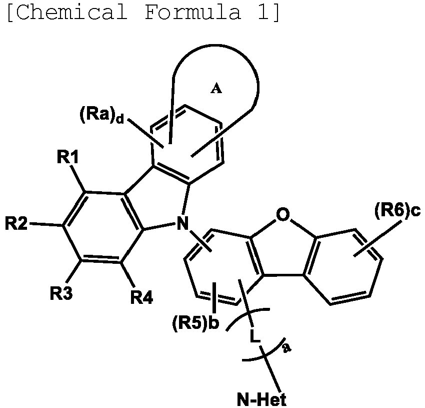

In Chemical Formula 1,
N-Het is a C2 to C60 monocyclic or polycyclic heterocyclic group substituted or unsubstituted, and comprising one or more Ns,
L is a direct bond; a substituted or unsubstituted C6 to C60 arylene group; or a substituted or unsubstituted C2 to C60 heteroarylene group, a is an integer of 1 to 3, and when a is 2 or greater, Ls are the same as or different from each other,
A is a substituted or unsubstituted C6 to C60 aryl ring; or a substituted or unsubstituted C2 to C60 heteroaryl ring,
Ra is selected from the group consisting of hydrogen; deuterium; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, d is an integer of 0 to 2, and when d is 2, the two Ras are the same as or different from each other, and
R1 to R6 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted phosphine oxide group; a substituted or unsubstituted silyl group; and a substituted or unsubstituted amine group, or two or more groups adjacent to each other bond to each other to form a substituted or unsubstituted C6 to C60 aromatic hydrocarbon ring or a substituted or unsubstituted C2 to C60 heteroring, b is an integer of 0 to 2, c is an integer of 0 to 4, and when b is 2, R5s are the same as or different from each other, and when c is 2 or greater, R6s are the same as or different from each other.

In addition, one embodiment of embodiment of the present application provides an organic light emitting device comprising a first electrode; a second electrode provided opposite to the first electrode; and one or more organic material layers provided between the first electrode and the second electrode, wherein one or more layers of the organic material layers comprise one or more of the heterocyclic compound represented by Chemical Formula 1.

In addition, one embodiment of the present application provides a composition for an organic material layer of an organic light emitting device, the composition comprising the heterocyclic compound represented by Chemical Formula 1 and a heterocyclic compound represented by the following Chemical Formula 2.

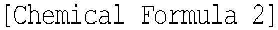

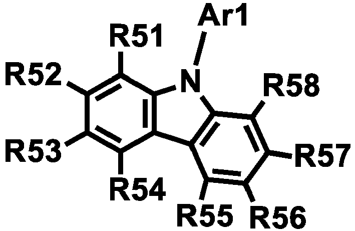

In Chemical Formula 2,
Ar1 is hydrogen; deuterium; a substituted or unsubstituted C1 to C60 alkyl group; a substituted or unsubstituted C6 to C60 aryl group; or a substituted or unsubstituted C2 to C60 heteroaryl group, and
R51 to R58 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted phosphine oxide group; and a substituted or unsubstituted amine group, or two or more groups adjacent to each other bond to each other to form a substituted or unsubstituted C6 to C60 aromatic hydrocarbon ring or a substituted or unsubstituted C2 to C60 heteroring.

Lastly, one embodiment of the present application provides a method for manufacturing an organic light emitting device, the method comprising preparing a substrate; forming a first electrode on the substrate; forming one or more organic material layers on the first electrode; and forming a second electrode on the organic material layer, wherein the forming of organic material layers comprises forming one or more organic material layers using the composition for an organic material layer according to one embodiment of the present application.

### [Advantageous Effects]

A compound described in the present specification can be used as a material of an organic material layer of an organic light emitting device. In the organic light emitting device, the compound is capable of performing a role of a hole injection material, a hole transfer material, a light emitting material, an electron transfer material, an electron injection material or the like. Particularly, the compound can be used as a light emitting material of the organic light emitting device. For example, the compound can be used alone as a light emitting material, or two of the compounds can be used together as a light emitting material, and can be used as a host material of a light emitting layer.

Particularly, the compound of Chemical Formula 1 has an O atom with high electronegativity in the center of the core structure and thereby has an excellent electron transfer ability, and has properties suitable for exciton blocking as well. In addition, when No. 1 and No. 3 positions of one side benzene ring of the dibenzofuran structure are substituted with a fused carbazole structure, the HOMO orbital and the LUMO orbital can be separated due to steric, which facilitates electron transfer.

Meanwhile, when No. 1 and No. 4 positions of one side benzene ring of the dibenzofuran are substituted with a fused carbazole structure, intermolecular Π-Π stacking effectively occurs resulting in effective intermolecular electron transfer, and thermal stability suitable for a device can be obtained. Accordingly, by using the heterocyclic compound of Chemical Formula 1, an organic light emitting device with improved lifetime, driving stability and efficiency can be manufactured.

### [Description of Drawings]

FIG. 1 to FIG. 3 are diagrams each schematically illustrating a lamination structure of an organic light emitting device according to one embodiment of the present application.
FIG. 4 is a diagram explaining an exciplex phenomenon.
FIG. 5 shows data measuring photoluminescence (PL) of each of a first host and a second host according to Example 71 of the present application.
FIG. 6 shows data measuring photoluminescence (PL) when comprising both a first host and a second host according to Example 71 of the present application.

### [Reference Numeral]

100: Substrate
200: Anode
300: Organic Material Layer
301: Hole Injection Layer
302: Hole Transfer Layer
303: Light Emitting Layer
304: Hole Blocking Layer
305: Electron Transfer Layer
306: Electron Injection Layer
400: Cathode

### [Mode for Disclosure]

Hereinafter, the present application will be described in detail.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0% or a hydrogen content being 100%. In other words, an expression of "substituent X is hydrogen" does not exclude deuterium such as a hydrogen content being 100% or a deuterium content being 0%, and therefore, may mean a state in which hydrogen and deuterium are mixed.

In one embodiment of the present application, deuterium is one of isotopes of hydrogen, is an element having deuteron formed with one proton and one neutron as a nucleus, and may be expressed as hydrogen-2, and the elemental symbol may also be written as D or 2H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 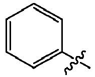 means that the total number of substituents that the phenyl group may have is 5 (T1 in the formula), and the number of deuterium among these is 1 (T2 in the formula) . In other words, having a deuterium content of 20% in a phenyl group may be represented by the following structural formulae.

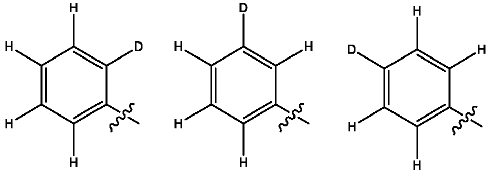

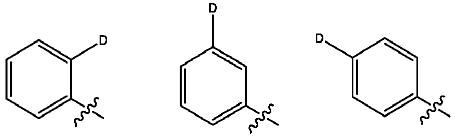

In addition, in one embodiment of the present application, "a phenyl group having a deuterium content of 0%" may mean a phenyl group that does not comprise a deuterium atom, that is, a phenyl group that has 5 hydrogen atoms.

In the present specification, the halogen may be fluorine, chlorine, bromine or iodine.

In the present specification, the alkyl group comprises linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may comprise a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group comprises linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may comprise a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group comprises linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may comprise methoxy, ethoxy, n-propoxy, i-propyloxy, n-butoxy, isobutoxy, tert-butoxy, secbutoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group comprises monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may comprise a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group comprises O, S, Se, N or Si as a heteroatom, comprises monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group comprises monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group comprises a spiro group. The number of carbon atoms of the aryl group may be from 6 to 60, specifically from 6 to 40 and more specifically from 6 to 25. Specific examples of the aryl group may comprise a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the fluorenyl group may be substituted, and adjacent substituents may bond to each other to form a ring.

When the fluorenyl group is substituted, 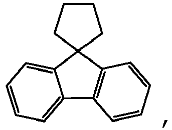 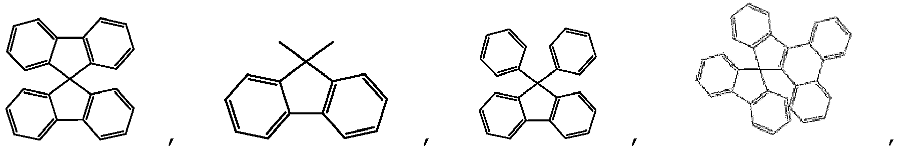 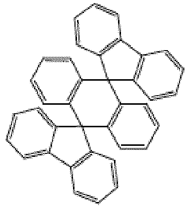 and the like may be included, however, the structure is not limited thereto.

In the present specification, the heteroaryl group comprises S, O, Se, N or Si as a heteroatom, comprises monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may comprise a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, a 5,10-dihydrobenzo[b,e][1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may comprise a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide may comprise a diphenylphosphine oxide group, a dinaphthylphosphine oxide group and the like, but are not limited thereto.

In the present specification, the silyl group is a substituent comprising Si, having the Si atom directly linked as a radical, and is represented by -SiR104R105R106. R104 to R106 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may comprise a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

As the aliphatic or aromatic hydrocarbon ring or heteroring that adjacent groups may form, the structures illustrated as the cycloalkyl group, the cycloheteroalkyl group, the aryl group and the heteroaryl group described above may be used except for those that are not a monovalent group.

In the present specification, the term "substitution" means a hydrogen atom bonding to a carbon atom of a compound being changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, "substituted or unsubstituted" means being substituted with one or more substituents selected from the group consisting of C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; -P(=O)RR'; C1 to C20 alkylamine; C6 to C60 monocyclic or polycyclic arylamine; and C2 to C60 monocyclic or polycyclic heteroarylamine, or being unsubstituted, or being substituted with a substituent linking two or more substituents selected from among the substituents illustrated above, or being unsubstituted.

In one embodiment of the present application, R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

In another embodiment, R, R' and R" are the same as or different from each other, and each independently a substituted or unsubstituted C1 to C40 alkyl group; a substituted or unsubstituted C6 to C40 aryl group; or a substituted or unsubstituted C2 to C40 heteroaryl group.

In another embodiment, R, R' and R" are the same as or different from each other, and each independently a substituted or unsubstituted C1 to C40 alkyl group; or a substituted or unsubstituted C6 to C40 aryl group.

In another embodiment, R, R' and R" are the same as or different from each other, and each independently a substituted or unsubstituted C1 to C20 alkyl group; or a substituted or unsubstituted C6 to C20 aryl group.

In another embodiment, R, R' and R" are the same as or different from each other, and each independently a C1 to C20 alkyl group; or a C6 to C20 aryl group.

In another embodiment, R, R' and R" are the same as or different from each other, and each independently a C1 to C10 alkyl group; or a C6 to C10 aryl group.

In another embodiment, R, R' and R" are the same as or different from each other, and each independently a linear C1 to C10 alkyl group; or a monocyclic C6 to C10 aryl group.

In another embodiment, R, R' and R" are the same as or different from each other, and each independently a methyl group; or a phenyl group.

One embodiment of the present application provides a heterocyclic compound represented by Chemical Formula 1.

In one embodiment of the present application, Chemical Formula 1 may be represented by one of the following Chemical Formulae 3 to 6.

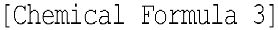

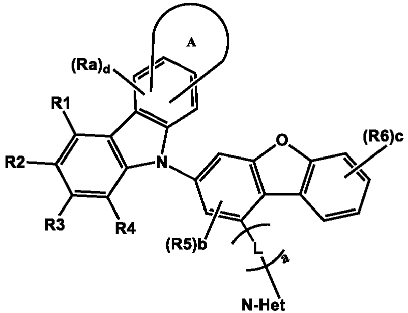

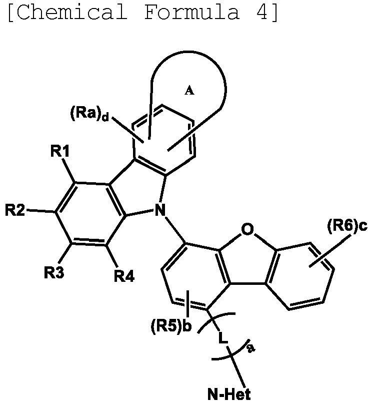

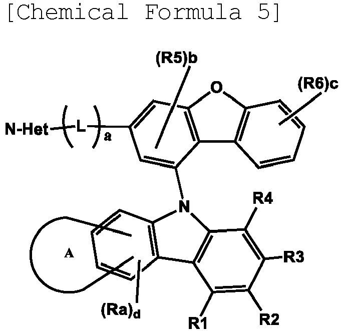

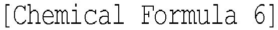

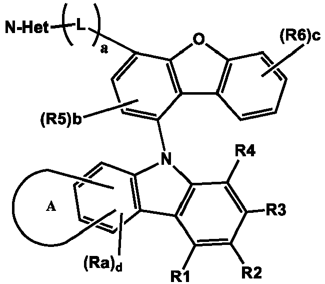

In Chemical Formulae 3 to 6,
N-Het, L, A, Ra, R1 to R6, a, b, c and d have the same definitions as in Chemical Formula 1.

Particularly, when No.1 and No. 3 positions of the dibenzofuran are substituted with the substituents as in Chemical Formula 5, excellent lifetime properties are obtained, and device efficiency and driving are also superior.

In one embodiment of the present application, 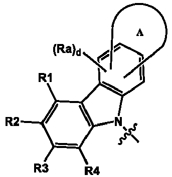 may be represented by any one of the following Chemical Formulae 1-1 to 1-6.

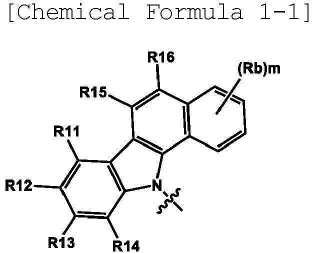

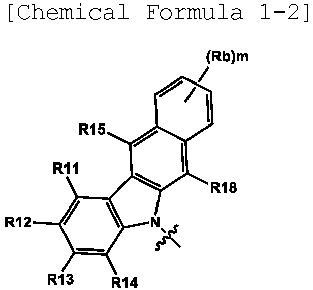

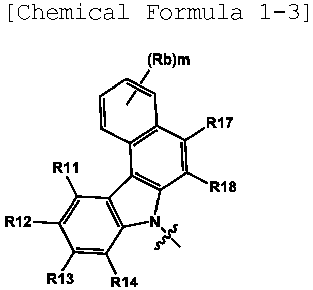

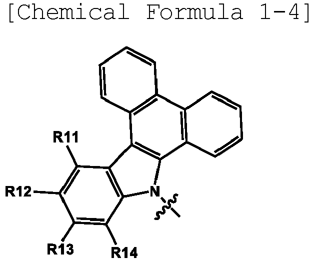

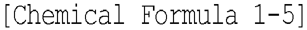

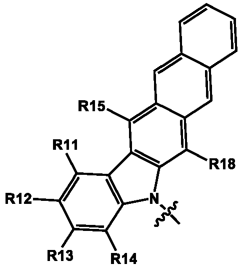

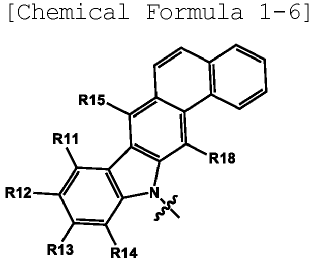

In Chemical Formulae 1-1 to 1-6,
R11 to R14 are the same as or different from each other, and each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring,
R15 to R18 are the same as or different from each other, and each independently hydrogen; deuterium; a substituted or unsubstituted C1 to C60 alkyl group; a substituted or unsubstituted C6 to C60 aryl group; or a substituted or unsubstituted C2 to C60 heteroaryl group, and
Rb is hydrogen; deuterium; or a substituted or unsubstituted C6 to C60 aryl group, m is an integer of 0 to 4, and when m is 2 or greater, Rbs are the same as or different from each other.

In Chemical Formulae 1-1 to 1-6, 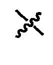 means a position linked to Chemical Formula 1.

When R12 and R13 bond to each other to form an unsubstituted C6 to C60 aromatic hydrocarbon ring or an unsubstituted C2 to C60 heteroring in Chemical Formula 1-1 and Chemical Formula 1-3 of the present application, at least one of R11, R14, R15 to R18 and Rb of Chemical Formula 1-1 and Chemical Formula 1-3 may be selected from the group consisting of deuterium; a substituted or unsubstituted C1 to C60 alkyl group; a substituted or unsubstituted C6 to C60 aryl group; and a substituted or unsubstituted C2 to C60 heteroaryl group.

When R12 and R13 bond to each other to form an unsubstituted C6 to C40 aromatic hydrocarbon ring in Chemical Formula 1-1 and Chemical Formula 1-3 of the present application, at least one of R11, R14, R15 to R18 and Rb of Chemical Formula 1-1 and Chemical Formula 1-3 may be selected from the group consisting of deuterium; a substituted or unsubstituted C6 to C60 aryl group; and a substituted or unsubstituted C2 to C60 heteroaryl group.

When R12 and R13 bond to each other to form an unsubstituted benzene ring in Chemical Formula 1-1 and Chemical Formula 1-3 of the present application, at least one of R11, R14, R15 to R18 and Rb of Chemical Formula 1-1 and Chemical Formula 1-3 may be selected from the group consisting of deuterium; a phenyl group; a biphenyl group; and a naphthyl group.

In one embodiment of the present application, in Chemical Formula 1-1 and Chemical Formula 1-3, R11 to R14 are the same as or different from each other, and each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group, or R11 and R12; or R13 and R14 may bond to each other to form a substituted or unsubstituted C6 to C60 aromatic hydrocarbon ring or a substituted or unsubstituted C2 to C60 heteroring.

In another embodiment, in Chemical Formula 1-1 and Chemical Formula 1-3, R11 to R14 are the same as or different from each other, and each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group, or R11 and R12; or R13 and R14 may bond to each other to form a substituted or unsubstituted C6 to C60 aromatic hydrocarbon ring.

In another embodiment, in Chemical Formula 1-1 and Chemical Formula 1-3, R11 to R14 are each independently selected from the group consisting of hydrogen; deuterium; a phenyl group; a biphenyl group; and a naphthyl group, or R11 and R12; or R13 and R14 may bond to each other to form a benzene ring.

In one embodiment of the present application, N-Het is a C2 to C60 monocyclic or polycyclic heteroring substituted or unsubstituted, and comprising one or more Ns.

In another embodiment, N-Het is a C2 to C60 monocyclic or polycyclic heteroring unsubstituted or substituted with one or more substituents selected from the group consisting of a C6 to C60 aryl group and a C2 to C60 heteroaryl group, and comprising one or more Ns.

In another embodiment, N-Het is a C2 to C40 monocyclic or polycyclic heteroring unsubstituted or substituted with one or more substituents selected from the group consisting of a C6 to C40 aryl group and a C2 to C40 heteroaryl group, and comprising one or more Ns.

In another embodiment, N-Het is a C2 to C30 monocyclic or polycyclic heteroring unsubstituted or substituted with one or more substituents selected from the group consisting of a C6 to C30 aryl group and a C2 to C30 heteroaryl group, and comprising one or more Ns.

In another embodiment, N-Het is a C2 to C30 monocyclic or polycyclic heteroring unsubstituted or substituted with one or more substituents selected from the group consisting of a phenyl group, a biphenyl group, a naphthyl group, a dibenzofuran group, a dibenzothiophene group and a dimethylfluorenyl group, and comprising one or more Ns.

In another embodiment, N-Het is a C2 to C30 monocyclic or polycyclic heteroring unsubstituted or substituted with one or more substituents selected from the group consisting of a phenyl group, a biphenyl group, a naphthyl group, a dibenzofuran group, a dibenzothiophene group and a dimethylfluorenyl group, and comprising one or more and three or less Ns.

In another embodiment, N-Het may be a triazine group unsubstituted or substituted with one or more substituents selected from the group consisting of a phenyl group, a biphenyl group, a naphthyl group, a dibenzofuran group, a dibenzothiophene group and a dimethylfluorenyl group.

In another embodiment, N-Het may be a triazine group unsubstituted or substituted with one or more substituents selected from the group consisting of a phenyl group, a biphenyl group, a naphthyl group, a dibenzofuran group, a dibenzothiophene group and a dimethylfluorenyl group; a pyrimidine group unsubstituted or substituted with one or more substituents selected from the group consisting of a phenyl group and a naphthyl group; a quinazoline group unsubstituted or substituted with a phenyl group, a biphenyl group or a naphthyl group; a quinoline group unsubstituted or substituted with a phenyl group, a biphenyl group or a naphthyl group; a quinoxaline group unsubstituted or substituted with a phenyl group or a naphthyl group; a benzofuro[3,2-d]pyrimidine group unsubstituted or substituted with a phenyl group, a biphenyl group or a naphthyl group; a benzofuro[2,3-d]pyrimidine group unsubstituted or substituted with a phenyl group, a biphenyl group or a naphthyl group; a benzo[4,5]thieno[3,2-d]pyrimidine group unsubstituted or substituted with a phenyl group, a biphenyl group or a naphthyl group; or a benzo[4,5]thieno[2,3-d]pyrimidine group unsubstituted or substituted with a phenyl group, a biphenyl group or a naphthyl group.

In one embodiment of the present application, the benzofuro[3,2-d]pyrimidine group may have the following structure.

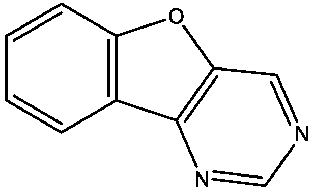

In one embodiment of the present application, the benzofuro[2,3-d]pyrimidine group may have the following structure.

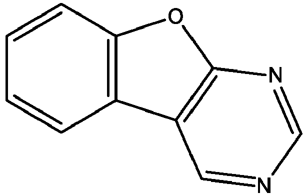

In one embodiment of the present application, the benzo[4,5]thieno[3,2-d]pyrimidine group may have the following structure.

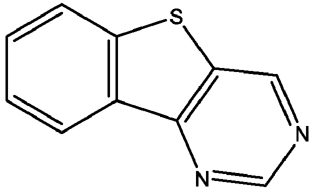

In one embodiment of the present application, the benzo[4,5]thieno[2,3-d]pyrimidine group may have the following structure.

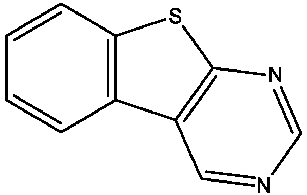

In one embodiment of the present application, N-Het may be further substituted with a C6 to C20 aryl group; or deuterium.

In another embodiment, N-Het may be further substituted with deuterium; a phenyl group; or a naphthyl group.

In one embodiment of the present application, N-Het may be represented by the following Chemical Formula 2-1.

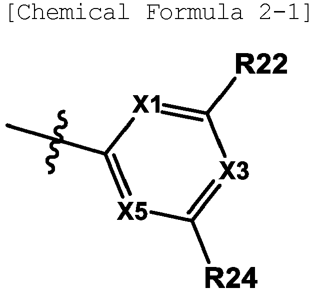

In Chemical Formula 2-1,
X1 is N or CR21, X3 is N or CR23, and X5 is N or CR25,
at least one of X1, X3 and X5 is N, and
R21 to R25 are the same as or different from each other, and each independently hydrogen; deuterium; a substituted or unsubstituted C1 to C60 alkyl group; a substituted or unsubstituted C6 to C60 aryl group; or a substituted or unsubstituted C2 to C60 heteroaryl group.

In Chemical Formula 2-1, 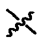 means a position linked to L of Chemical Formula 1.

In one embodiment of the present application, Chemical Formula 2-1 may be selected from among the following structural formulae.

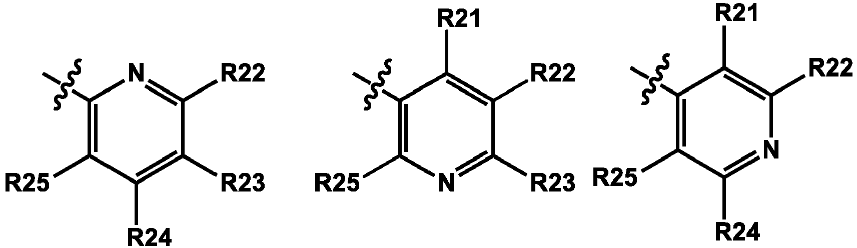

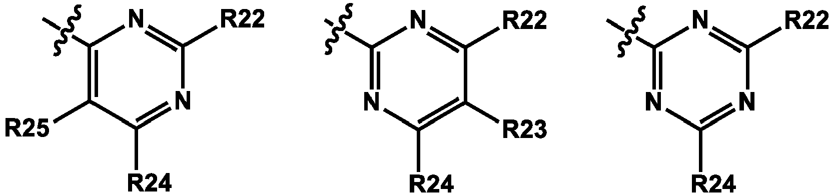

In the structural formulae,
R21 to R25 have the same definitions as in Chemical Formula 2-1.

In one embodiment of the present application, L may be a direct bond; a substituted or unsubstituted C6 to C60 arylene group; or a substituted or unsubstituted C2 to C60 heteroarylene group.

In another embodiment, L may be a direct bond; a substituted or unsubstituted C6 to C40 arylene group; or a substituted or unsubstituted C2 to C40 heteroarylene group.

In another embodiment, L may be a direct bond; a C6 to C40 arylene group; or a C2 to C40 heteroarylene group.

In another embodiment, L may be a direct bond; a C6 to C40 monocyclic arylene group; or a C2 to C40 monocyclic heteroarylene group.

In another embodiment, L may be a direct bond; or a phenylene group.

In another embodiment, L may be a direct bond.

In another embodiment, L may be a phenylene group.

In one embodiment of the present application, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, N-Het of Chemical Formula 1 may be a C2 to C60 monocyclic or polycyclic heteroring substituted or unsubstituted, and comprising one or more and two or less Ns.

In another embodiment, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, N-Het of Chemical Formula 1 may be a C2 to C40 monocyclic or polycyclic heteroring substituted or unsubstituted, and comprising one or more and two or less Ns.

In another embodiment, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, N-Het of Chemical Formula 1 may be a C2 to C20 monocyclic or polycyclic heteroring substituted or unsubstituted, and comprising one or more and two or less Ns.

In another embodiment, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, N-Het of Chemical Formula 1 may be a C2 to C20 monocyclic or polycyclic heteroring unsubstituted or substituted with a C6 to C20 aryl group, and comprising one or more and two or less Ns.

In another embodiment, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, N-Het of Chemical Formula 1 may be a pyrimidine group unsubstituted or substituted with one or more substituents selected from the group consisting of a phenyl group and a naphthyl group; a quinazoline group unsubstituted or substituted with a phenyl group, a biphenyl group or a naphthyl group; a quinoline group unsubstituted or substituted with a phenyl group, a biphenyl group or a naphthyl group; a quinoxaline group unsubstituted or substituted with a phenyl group or a naphthyl group; a benzofuro[3,2-d]pyrimidine group unsubstituted or substituted with a phenyl group, a biphenyl group or a naphthyl group; a benzofuro[2,3-d]pyrimidine group unsubstituted or substituted with a phenyl group, a biphenyl group or a naphthyl group; a benzo[4,5]thieno[3,2-d]pyrimidine group unsubstituted or substituted with a phenyl group, a biphenyl group or a naphthyl group; or a benzo[4,5]thieno[2,3-d]pyrimidine group unsubstituted or substituted with a phenyl group, a biphenyl group or a naphthyl group.

In one embodiment of the present application, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, a deuterium content in Chemical Formula 1 may be greater than or equal to 10% and less than or equal to 100%.

In another embodiment, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, a deuterium content in Chemical Formula 1 may be greater than or equal to 15% and less than or equal to 90%.

In another embodiment, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, a deuterium content in Chemical Formula 1 may be greater than or equal to 20% and less than or equal to 80%.

In another embodiment, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, a deuterium content in Chemical Formula 1 may be greater than or equal to 20% and less than or equal to 40%.

In the present application, the deuterium content in Chemical Formula 1 may mean a ratio of substitution by deuterium among positions of Chemical Formula 1 to which substituents may be introduced. In other words, when there are a total of 40 positions that may be substituted by substituents in Chemical Formula 1 and 20 of them are substituted with deuterium, the deuterium content in Chemical Formula 1 may be represented as 50%.

In one embodiment of the present application, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, L of Chemical Formula 1 may be a substituted or unsubstituted C6 to C60 arylene group; or a substituted or unsubstituted C2 to C60 heteroarylene group.

In another embodiment, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, L of Chemical Formula 1 may be a substituted or unsubstituted C6 to C40 arylene group; or a substituted or unsubstituted C2 to C40 heteroarylene group.

In another embodiment, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, L of Chemical Formula 1 may be a substituted or unsubstituted C6 to C20 arylene group; or a substituted or unsubstituted C2 to C20 heteroarylene group.

In another embodiment, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, L of Chemical Formula 1 may be a C6 to C20 arylene group; or a C2 to C20 heteroarylene group.

In another embodiment, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, L of Chemical Formula 1 may be a C6 to C20 arylene group.

In another embodiment, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, L of Chemical Formula 1 may be a phenylene group.

In one embodiment of the present application, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, N-Het of Chemical Formula 1 may be a C2 to C60 monocyclic or polycyclic heteroring substituted or unsubstituted and comprising one or more and two or less Ns, or the deuterium content in Chemical Formula 1 is greater than or equal to 10% and less than or equal to 100%, or L of Chemical Formula 1 may be a substituted or unsubstituted C6 to C60 arylene group; or a substituted or unsubstituted C2 to C60 heteroarylene group.

In one embodiment of the present application, A may be a substituted or unsubstituted C6 to C60 aryl ring; or a substituted or unsubstituted C2 to C60 heteroaryl ring.

In another embodiment, A may be a substituted or unsubstituted C6 to C40 aryl ring; or a substituted or unsubstituted C2 to C40 heteroaryl ring.

In another embodiment, A may be a substituted or unsubstituted C6 to C40 aryl ring.

In another embodiment, A may be a substituted or unsubstituted benzene ring or a substituted or unsubstituted naphthyl ring.

In another embodiment, A may be a benzene ring unsubstituted or substituted with a C6 to C30 aryl group, or a naphthyl ring unsubstituted or substituted with a C6 to C30 aryl group.

In another embodiment, A may be a benzene ring or naphthyl ring unsubstituted or substituted with a phenyl group.

In one embodiment of the present application, A may be further substituted with deuterium.

In one embodiment of the present application, Ra is selected from the group consisting of hydrogen; deuterium; a substituted or unsubstituted C6 to C60 aryl group; and a substituted or unsubstituted C2 to C60 heteroaryl group, or two or more groups adjacent to each other may bond to each other to form a substituted or unsubstituted C6 to C60 aromatic hydrocarbon ring or a substituted or unsubstituted C2 to C60 heteroring.

In another embodiment, Ra is selected from the group consisting of hydrogen; deuterium; a substituted or unsubstituted C6 to C40 aryl group; and a substituted or unsubstituted C2 to C40 heteroaryl group, or two or more groups adjacent to each other may bond to each other to form a substituted or unsubstituted C6 to C40 aromatic hydrocarbon ring or a substituted or unsubstituted C2 to C40 heteroring.

In another embodiment, Ra is selected from the group consisting of hydrogen; deuterium; and a C6 to C40 aryl group, or two or more groups adjacent to each other may bond to each other to form a C6 to C40 aromatic hydrocarbon ring.

In another embodiment, Ra is selected from the group consisting of hydrogen; deuterium; and a C6 to C40 monocyclic or polycyclic aryl group, or two or more groups adjacent to each other may bond to each other to form a C6 to C40 monocyclic or polycyclic aromatic hydrocarbon ring.

In another embodiment, Ra is selected from the group consisting of hydrogen; deuterium; and a phenyl group, or two or more groups adjacent to each other may bond to each other to form a benzene ring.

In one embodiment of the present application, R1 to R4 are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted phosphine oxide group; a substituted or unsubstituted silyl group; and a substituted or unsubstituted amine group, or two or more groups adjacent to each other may bond to each other to form a substituted or unsubstituted C6 to C60 aromatic hydrocarbon ring or a substituted or unsubstituted C2 to C60 heteroring.

In another embodiment, R1 to R4 are each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group, or two or more groups adjacent to each other may bond to each other to form a substituted or unsubstituted C6 to C60 aromatic hydrocarbon ring or a substituted or unsubstituted C2 to C60 heteroring.

In another embodiment, R1 to R4 are each independently selected from the group consisting of hydrogen; deuterium; and a substituted or unsubstituted C6 to C40 aryl group, or two or more groups adjacent to each other may bond to each other to form a substituted or unsubstituted C6 to C60 aromatic hydrocarbon ring.

In another embodiment, R1 to R4 are each independently selected from the group consisting of hydrogen; deuterium; and a monocyclic or polycyclic C6 to C40 aryl group, or two or more groups adjacent to each other may bond to each other to form a monocyclic or polycyclic C6 to C60 aromatic hydrocarbon ring.

In another embodiment, R1 to R4 are each independently selected from the group consisting of hydrogen; deuterium; and a monocyclic or polycyclic C6 to C20 aryl group, or two or more groups adjacent to each other may bond to each other to form a monocyclic or polycyclic C6 to C30 aromatic hydrocarbon ring.

In another embodiment, R1 to R4 are each independently selected from the group consisting of hydrogen; deuterium; a phenyl group; a biphenyl group; and a naphthyl group, or two or more groups adjacent to each other may bond to each other to form a benzene ring.

In one embodiment of the present application, R5 and R6 may be hydrogen.

In one embodiment of the present application, R5 and R6 may be deuterium.

In one embodiment of the present application, R5 and R6 may be hydrogen; or deuterium.

In one embodiment of the present application, R5 and R6 may all be hydrogen.

In one embodiment of the present application, R5 and R6 may all be deuterium.

In one embodiment of the present application, the heterocyclic compound of Chemical Formula 1 may be further substituted with deuterium. Herein, the heterocyclic compound of Chemical Formula 1 may be substituted with deuterium by greater than or equal to 10% and less than or equal to 100%.

In one embodiment of the present application, R11 to R14 are the same as or different from each other, and each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group, or two or more groups adjacent to each other may bond to each other to form a substituted or unsubstituted C6 to C60 aromatic hydrocarbon ring or a substituted or unsubstituted C2 to C60 heteroring.

In another embodiment, R11 to R14 are each independently selected from the group consisting of hydrogen; deuterium; and a substituted or unsubstituted C6 to C40 aryl group, or two or more groups adjacent to each other may bond to each other to form a substituted or unsubstituted C6 to C60 aromatic hydrocarbon ring.

In another embodiment, R11 to R14 are each independently selected from the group consisting of hydrogen; deuterium; and a monocyclic or polycyclic C6 to C40 aryl group, or two or more groups adjacent to each other may bond to each other to form a monocyclic or polycyclic C6 to C60 aromatic hydrocarbon ring.

In another embodiment, R11 to R14 are each independently selected from the group consisting of hydrogen; deuterium; and a monocyclic or polycyclic C6 to C20 aryl group, or two or more groups adjacent to each other may bond to each other to form a monocyclic or polycyclic C6 to C30 aromatic hydrocarbon ring.

In another embodiment, R11 to R14 are each independently selected from the group consisting of hydrogen; deuterium; a phenyl group; a biphenyl group; and a naphthyl group, or two or more groups adjacent to each other may bond to each other to form a benzene ring.

In one embodiment of the present application, R15 to R18 are the same as or different from each other, and may be each independently hydrogen; deuterium; a substituted or unsubstituted C1 to C60 alkyl group; a substituted or unsubstituted C6 to C60 aryl group; or a substituted or unsubstituted C2 to C60 heteroaryl group.

In another embodiment, R15 to R18 are the same as or different from each other, and may be each independently hydrogen; deuterium; a substituted or unsubstituted C6 to C60 aryl group; or a substituted or unsubstituted C2 to C60 heteroaryl group.

In another embodiment, R15 to R18 are the same as or different from each other, and may be each independently hydrogen; deuterium; a substituted or unsubstituted C6 to C40 aryl group; or a substituted or unsubstituted C2 to C40 heteroaryl group.

In another embodiment, R15 to R18 are the same as or different from each other, and may be each independently hydrogen; deuterium; a C6 to C40 aryl group; or a C2 to C40 heteroaryl group.

In another embodiment, R15 to R18 are the same as or different from each other, and may be each independently hydrogen; deuterium; or a monocyclic or polycyclic C6 to C40 aryl group.

In another embodiment, R15 to R18 are the same as or different from each other, and may be each independently hydrogen; deuterium; or a phenyl group.

In one embodiment of the present application, Rb may be hydrogen; deuterium; or a substituted or unsubstituted C6 to C60 aryl group.

In another embodiment, Rb may be hydrogen; deuterium; or a substituted or unsubstituted C6 to C40 aryl group.

In another embodiment, Rb may be hydrogen; deuterium; or a substituted or unsubstituted C6 to C20 aryl group.

In another embodiment, Rb may be hydrogen; deuterium; or a C6 to C20 aryl group.

In another embodiment, Rb may be hydrogen; deuterium; or a C6 to C20 monocyclic or polycyclic aryl group.

In another embodiment, Rb may be hydrogen; deuterium; or a phenyl group.

In one embodiment of the present application, R21 to R25 are the same as or different from each other, and may be each independently hydrogen; deuterium; a substituted or unsubstituted C1 to C60 alkyl group; a substituted or unsubstituted C6 to C60 aryl group; or a substituted or unsubstituted C2 to C60 heteroaryl group.

In another embodiment, R21 to R25 are the same as or different from each other, and may be each independently hydrogen; deuterium; a substituted or unsubstituted C6 to C60 aryl group; or a substituted or unsubstituted C2 to C60 heteroaryl group.

In another embodiment, R21 to R25 are the same as or different from each other, and may be each independently hydrogen; deuterium; a C6 to C60 aryl group unsubstituted or substituted with deuterium, a C1 to C60 alkyl group or a C6 to C60 aryl group; or a C2 to C60 heteroaryl group.

In another embodiment, R21 to R25 are the same as or different from each other, and may be each independently hydrogen; deuterium; a C6 to C40 aryl group unsubstituted or substituted with deuterium, a C1 to C10 alkyl group or a C6 to C20 aryl group; or a C2 to C40 heteroaryl group.

In another embodiment, R21 to R25 are the same as or different from each other, and may be each independently hydrogen; deuterium; a phenyl group unsubstituted or substituted with deuterium or a naphthyl group; a naphthyl group unsubstituted or substituted with a phenyl group; a biphenyl group; a dibenzofuran group; a dibenzothiophene group; or a dimethylfluorenyl group.

In one embodiment of the present application, X1, X3 and X5 may be N.

In one embodiment of the present application, at least two of X1, X3 and X5 may be N.

According to one embodiment of the present application, Chemical Formula 1 may be represented by any one of the following compounds, but is not limited thereto.

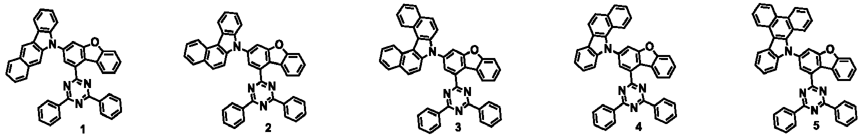

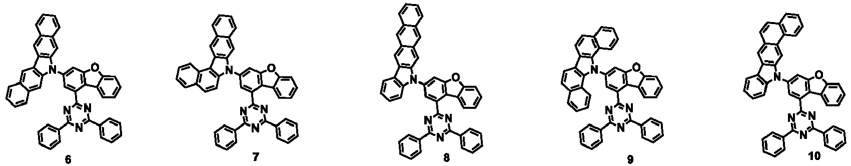

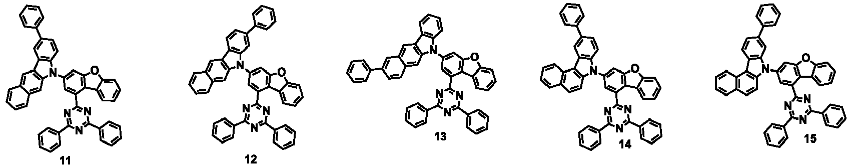

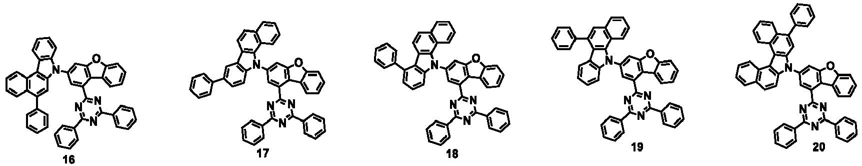

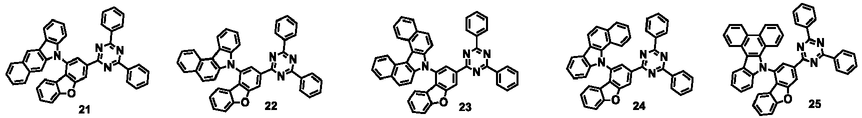

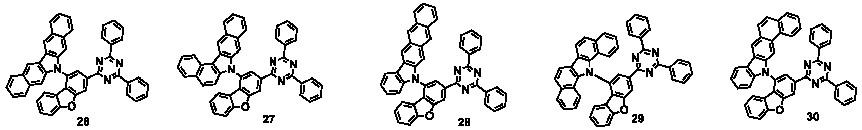

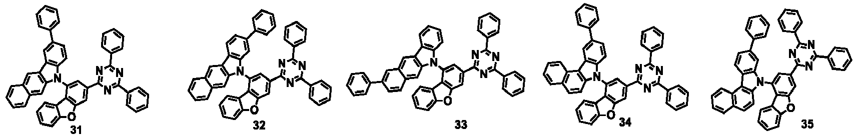

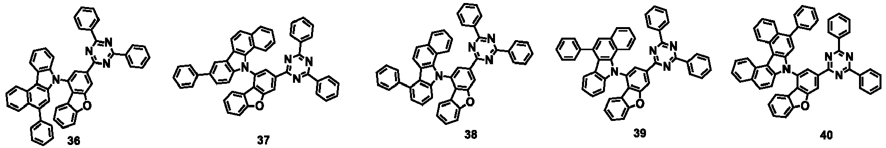

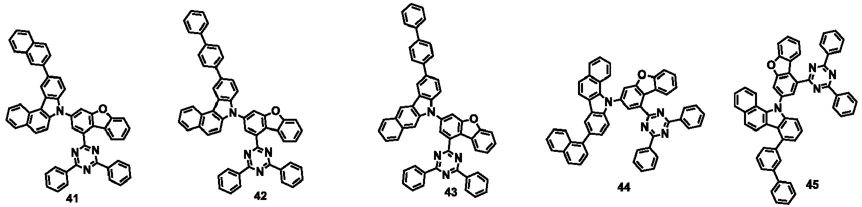

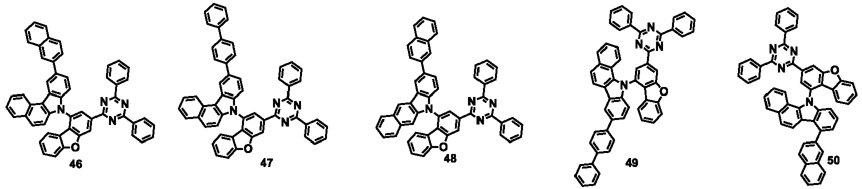

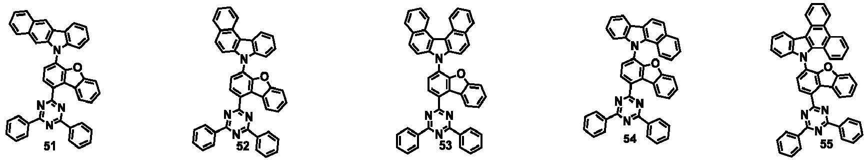

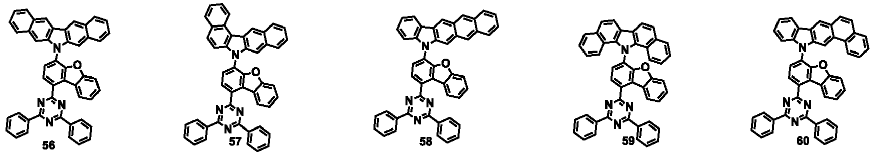

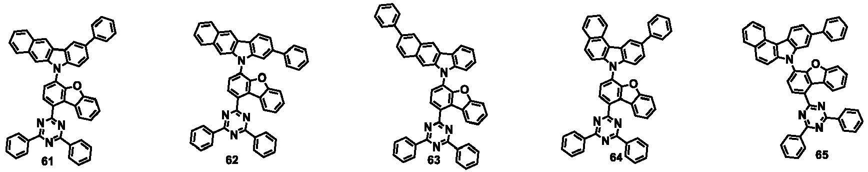

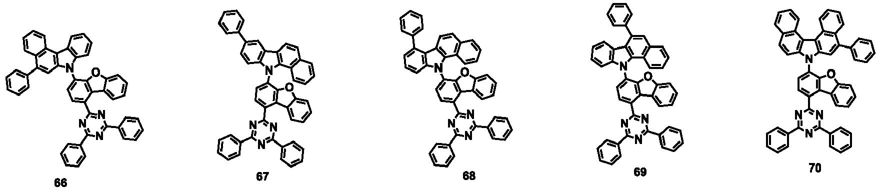

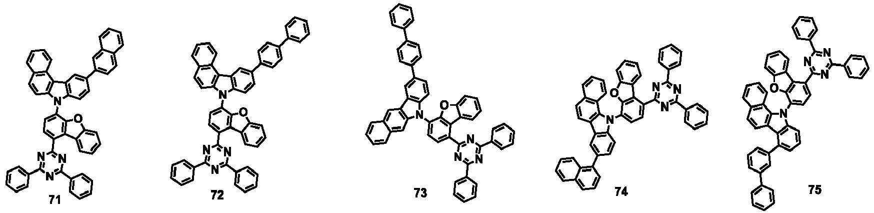

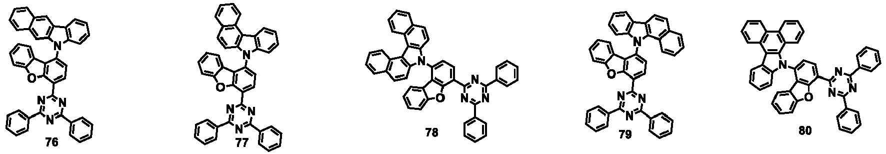

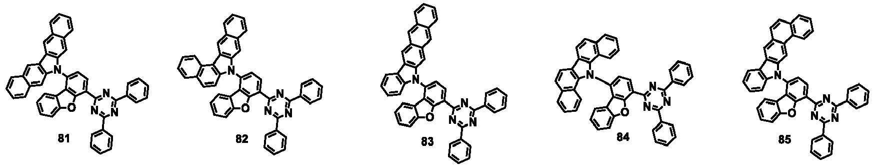

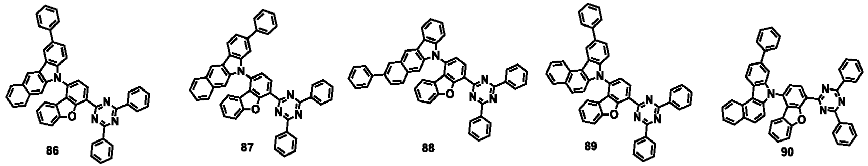

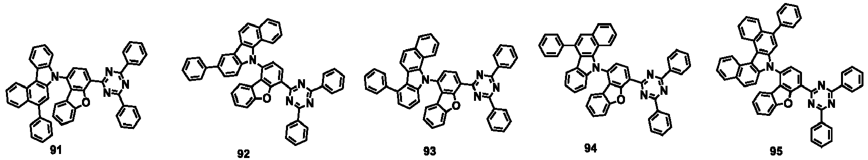

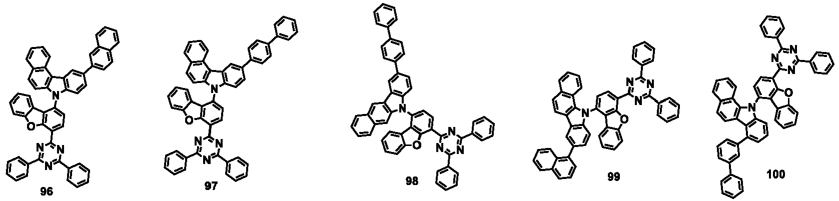

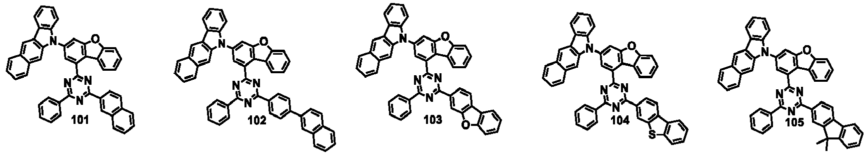

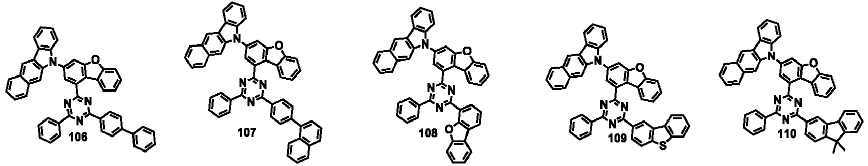

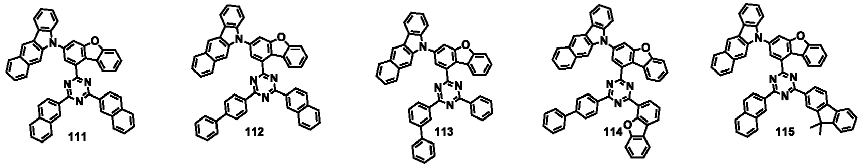

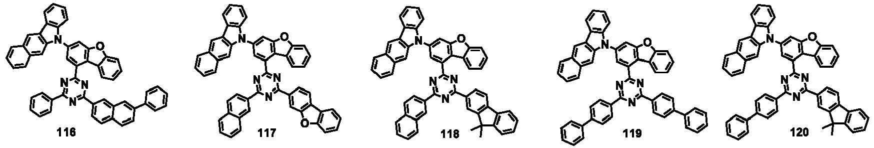

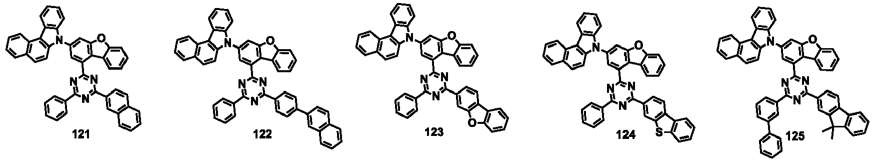

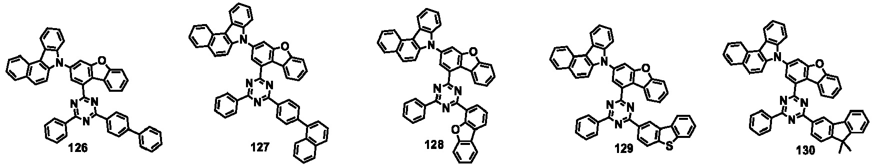

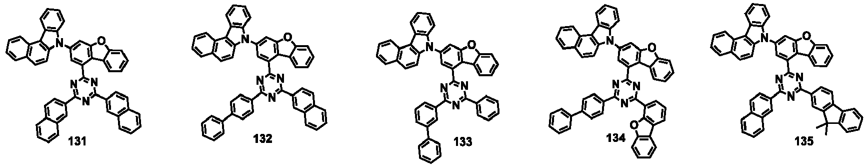

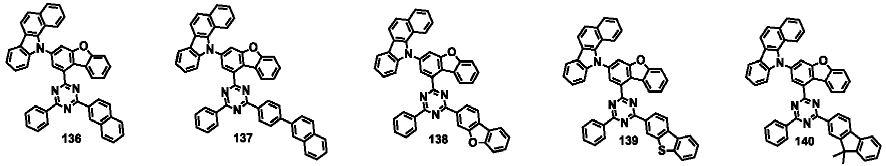

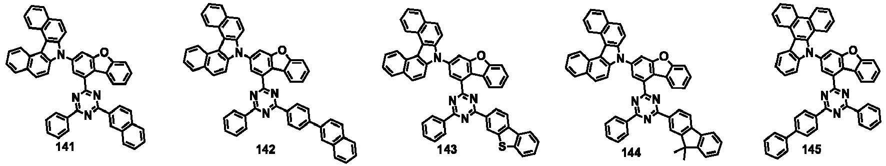

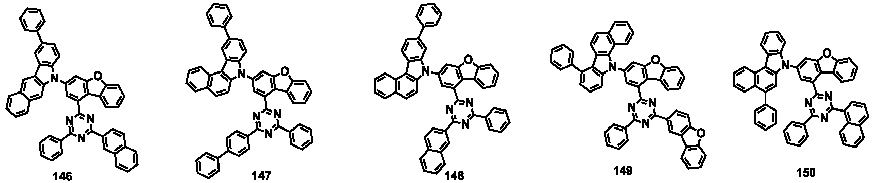

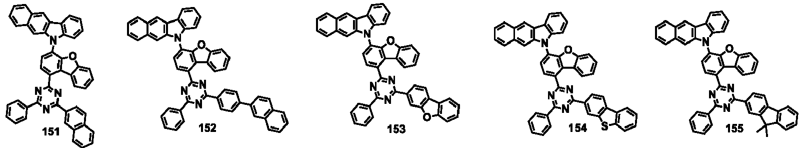

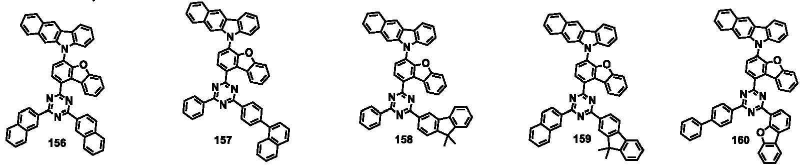

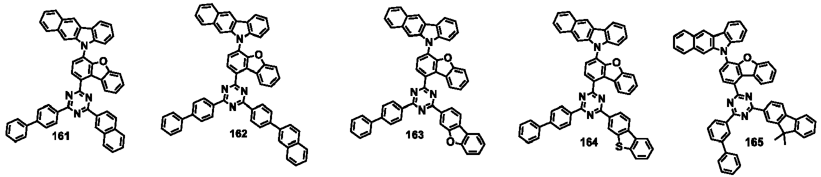

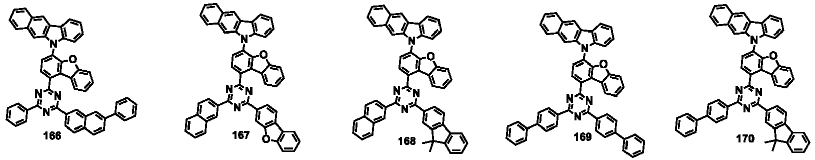

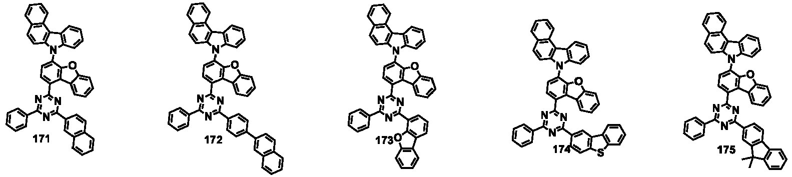

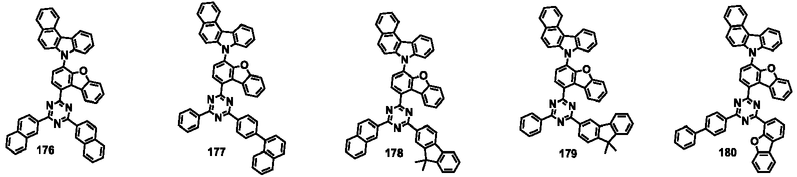

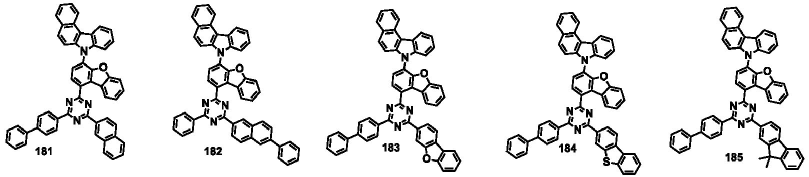

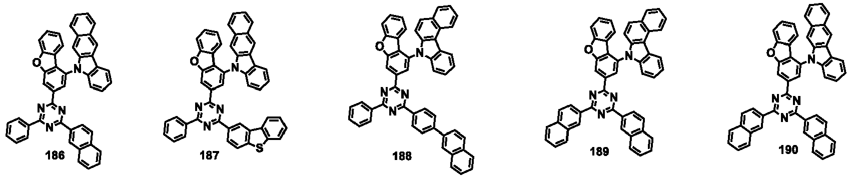

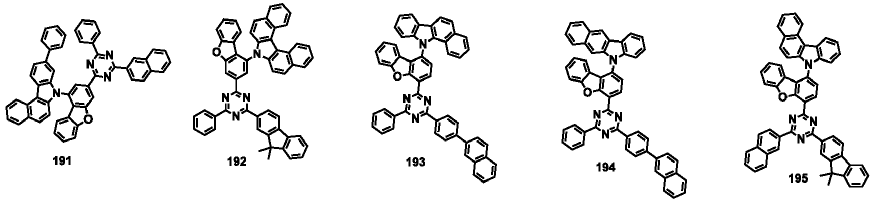

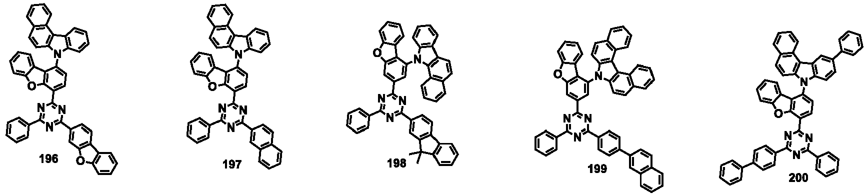

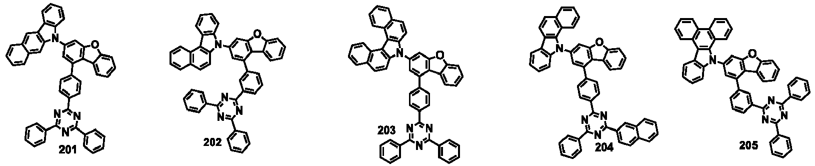

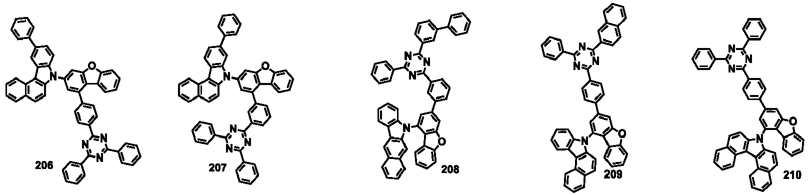

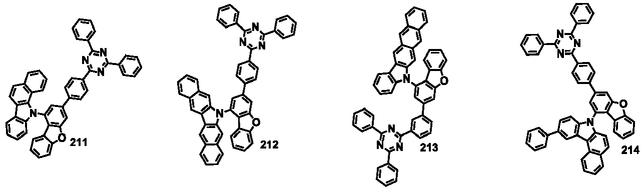

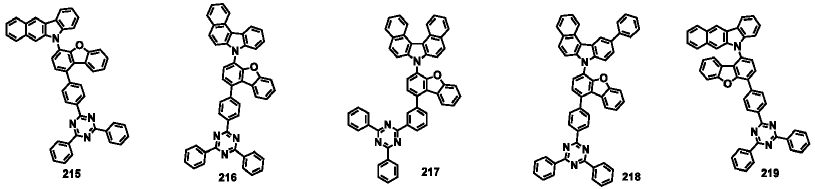

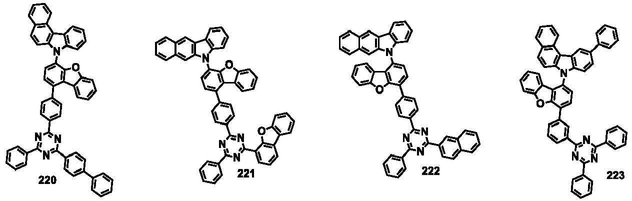

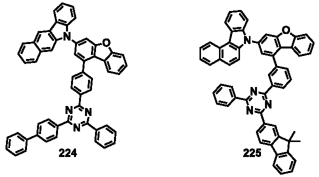

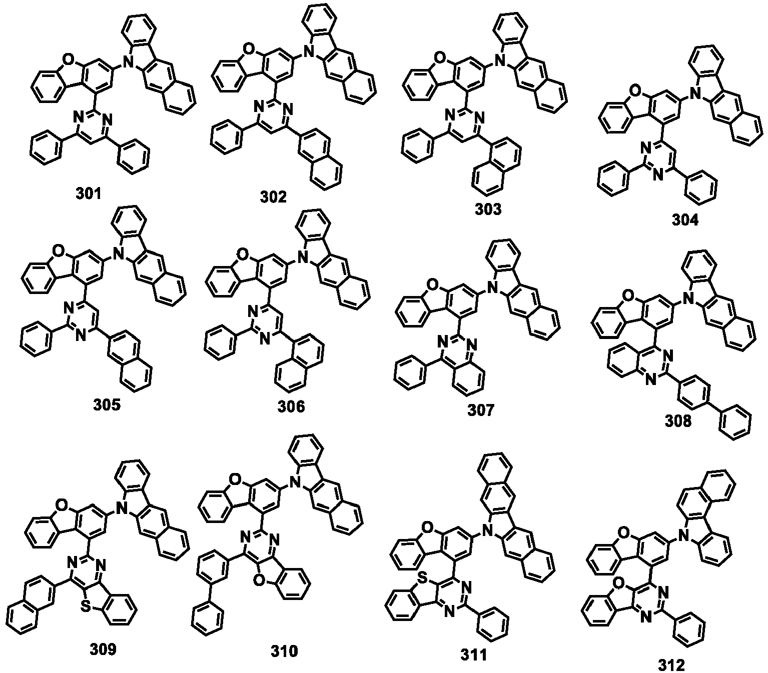

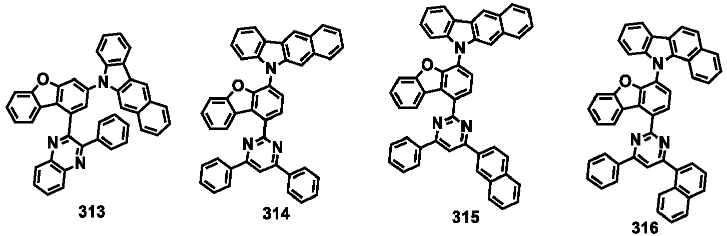

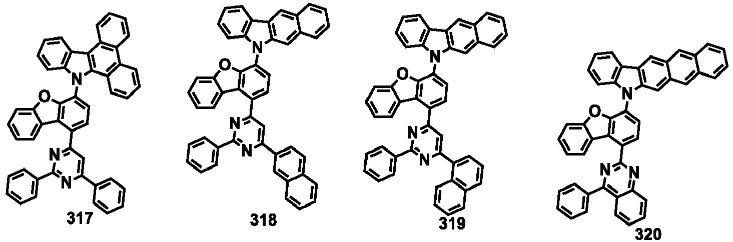

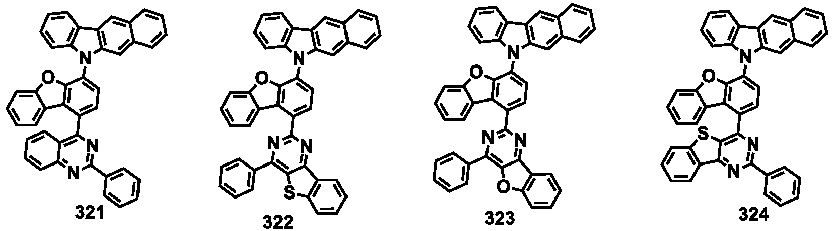

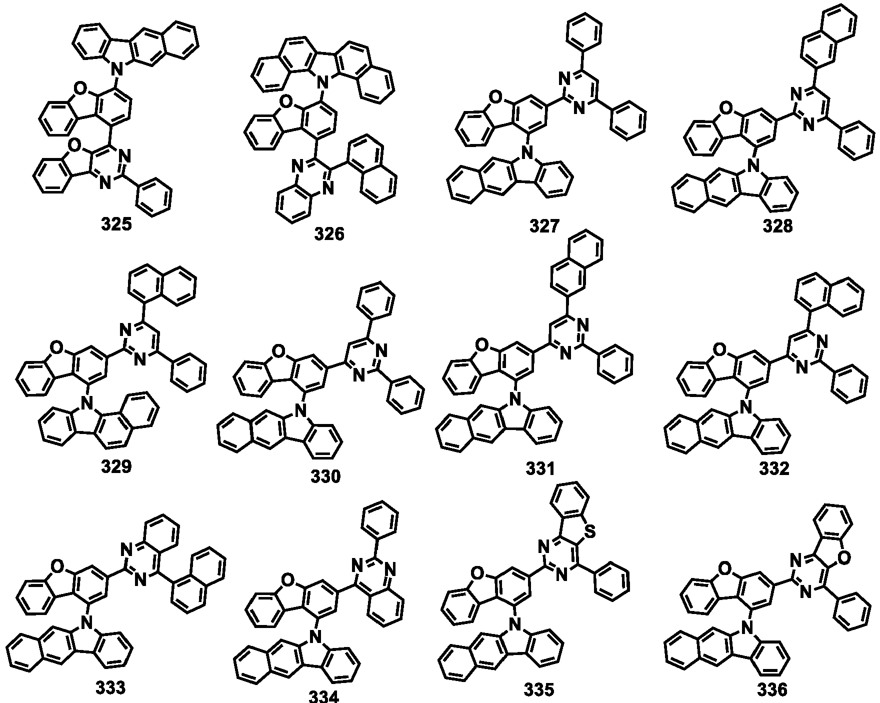

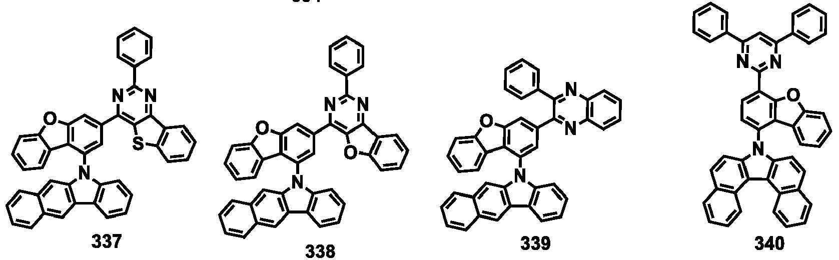

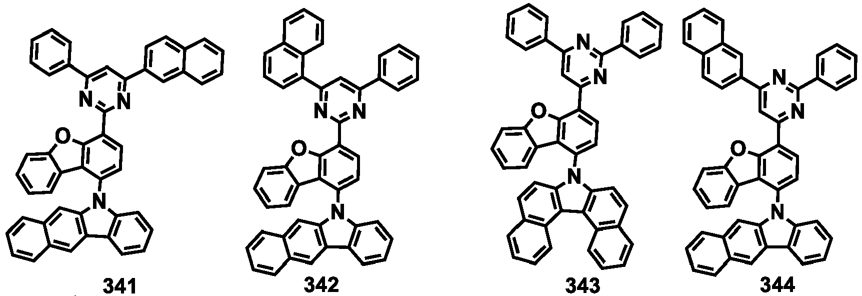

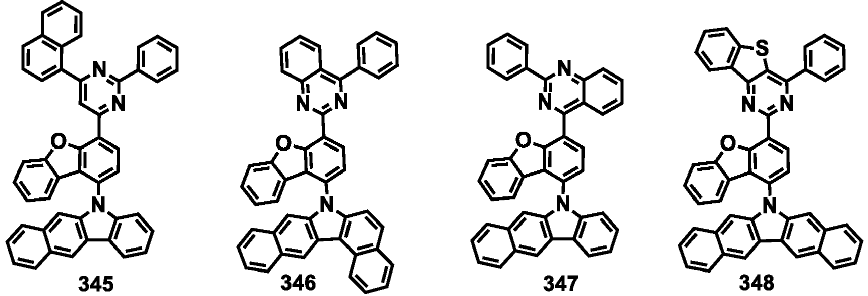

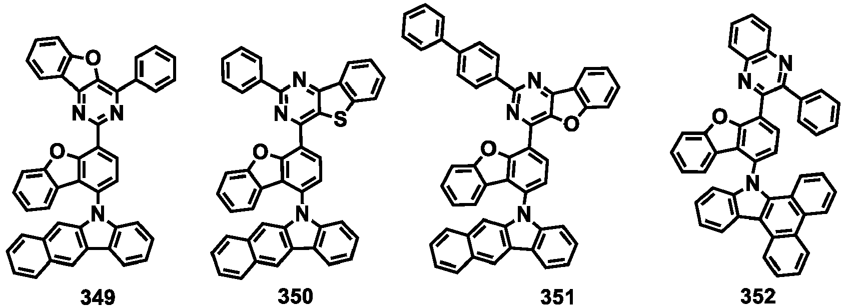

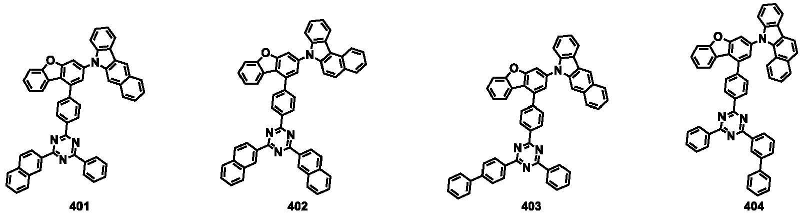

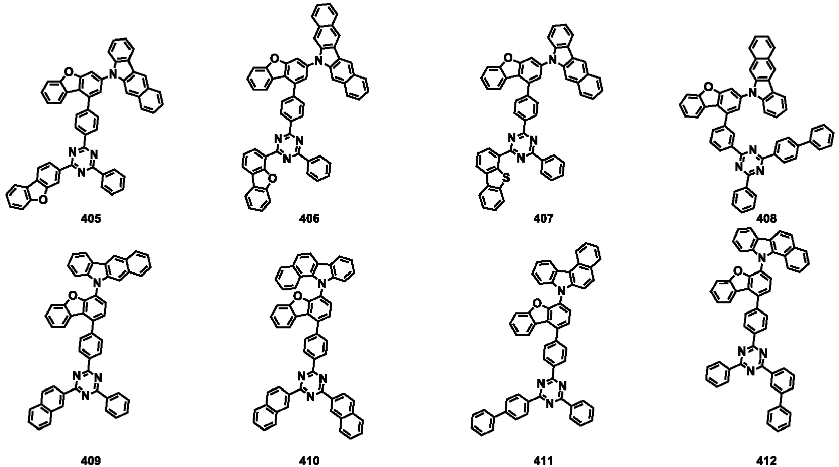

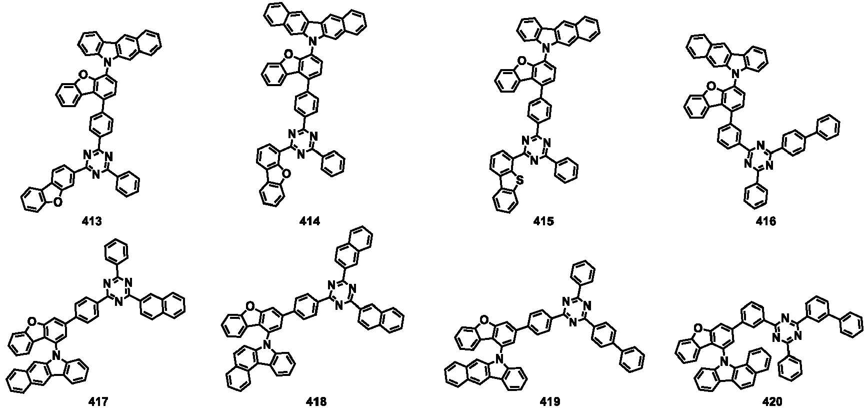

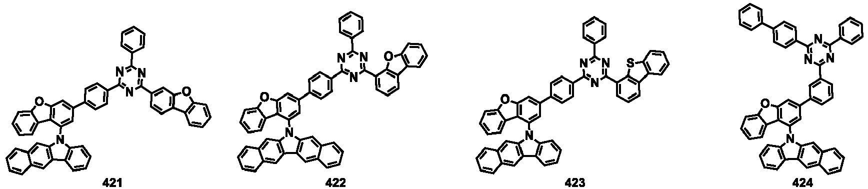

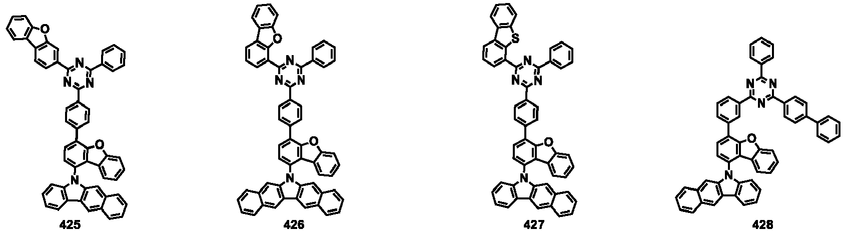

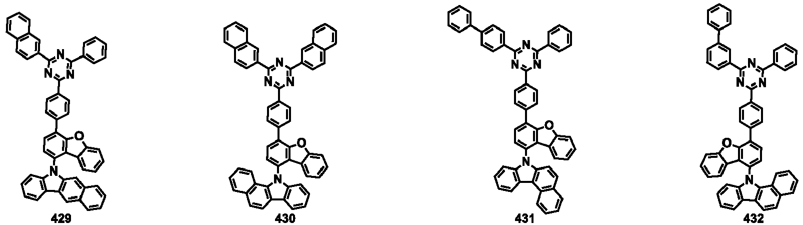

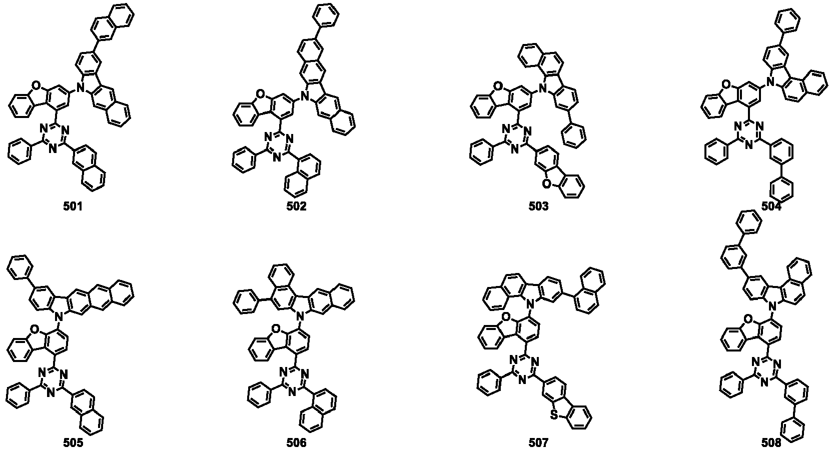

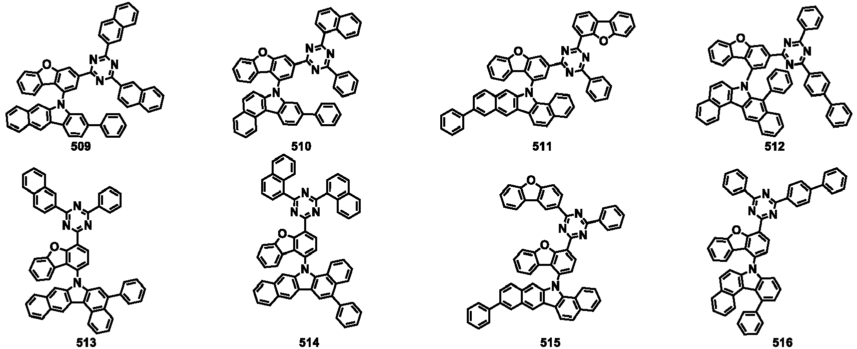

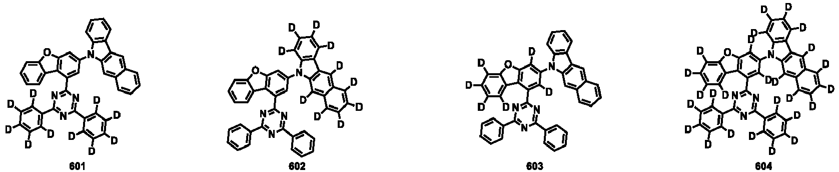

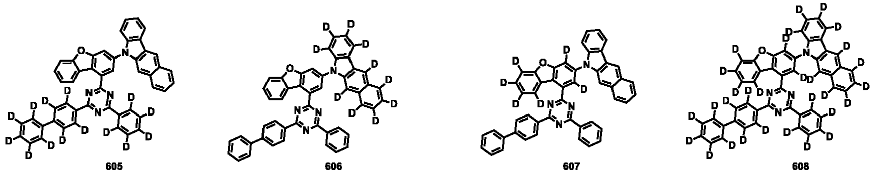

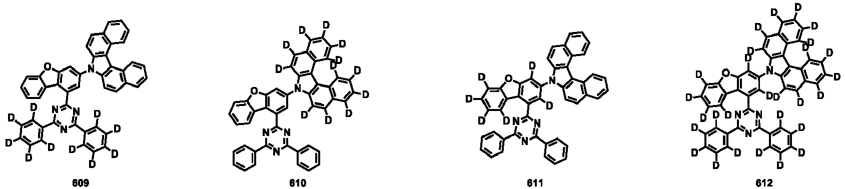

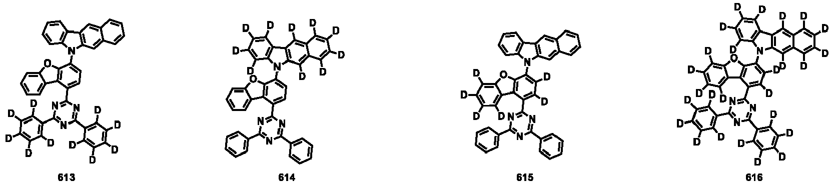

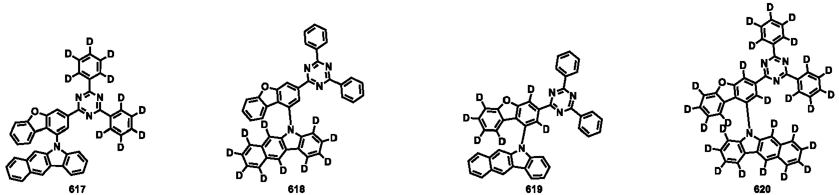

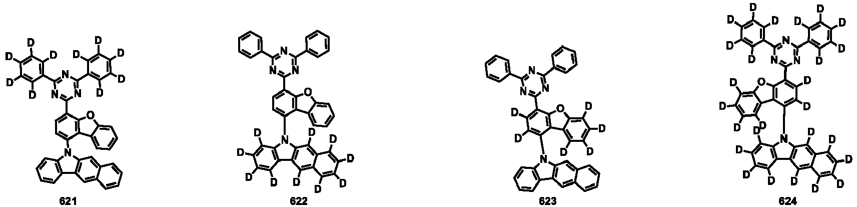

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

In addition, one embodiment of the present application provides an organic light emitting device comprising a first electrode; a second electrode provided opposite to the first electrode; and one or more organic material layers provided between the first electrode and the second electrode, wherein one or more layers of the organic material layers comprise one or more of the heterocyclic compound according to Chemical Formula 1.

Another embodiment of the present application provides an organic light emitting device comprising a first electrode; a second electrode provided opposite to the first electrode; and one or more organic material layers provided between the first electrode and the second electrode, wherein one or more layers of the organic material layers comprise one heterocyclic compound according to Chemical Formula 1.

Another embodiment of the present application provides an organic light emitting device comprising a first electrode; a second electrode provided opposite to the first electrode; and one or more organic material layers provided between the first electrode and the second electrode, wherein one or more layers of the organic material layers comprise two heterocyclic compounds according to Chemical Formula 1.

In the organic light emitting device, when two or more of the heterocyclic compound are included, types of the heterocyclic compound may be the same as or different from each other.

Specific descriptions on the heterocyclic compound represented by Chemical Formula 1 are the same as the descriptions provided above.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a blue organic light emitting device, and the heterocyclic compound according to Chemical Formula 1 may be used as a material of the blue organic light emitting device. For example, the heterocyclic compound according to Chemical Formula 1 may be included in a host material of a blue light emitting layer of the blue organic light emitting device.

In one embodiment of the present application, the organic light emitting device may be a green organic light emitting device, and the heterocyclic compound according to Chemical Formula 1 may be used as a material of the green organic light emitting device. For example, the heterocyclic compound according to Chemical Formula 1 may be included in a host material of a green light emitting layer of the green organic light emitting device.

In one embodiment of the present application, the organic light emitting device may be a red organic light emitting device, and the heterocyclic compound according to Chemical Formula 1 may be used as a material of the red organic light emitting device. For example, the heterocyclic compound according to Chemical Formula 1 may be included in a host material of a red light emitting layer of the red organic light emitting device.

The organic light emitting device of the present disclosure may be manufactured using common organic light emitting device manufacturing methods and materials except that one or more of the organic material layers are formed using the heterocyclic compound described above.

The heterocyclic compound may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, but may be formed in a multilayer structure in which two or more organic material layers are laminated. For example, the organic light emitting device of the present disclosure may have a structure comprising a hole injection layer, a hole transfer layer, a light emitting layer, an electron transfer layer, an electron injection layer and the like as the organic material layer. However, the structure of the organic light emitting device is not limited thereto, and may comprise a smaller number of organic material layers.

In the organic light emitting device of the present disclosure, the organic material layer may comprise a light emitting layer, and the light emitting layer may comprise the heterocyclic compound.

In another organic light emitting device, the organic material layer comprises a light emitting layer, the light emitting layer comprises a host material, and the host material may comprise the heterocyclic compound.

As another example, the organic material layer comprising the heterocyclic compound comprises the heterocyclic compound represented by Chemical Formula 1 as a host, and an iridium-based dopant may be used therewith.

In the organic light emitting device of the present disclosure, the organic material layer comprises an electron injection layer or an electron transfer layer, and the electron transfer layer or the electron injection layer may comprise the heterocyclic compound.

In another organic light emitting device, the organic material layer comprises an electron blocking layer or a hole blocking layer, and the electron blocking layer or the hole blocking layer may comprise the heterocyclic compound.

The organic light emitting device of the present disclosure may further comprise one, two or more layers selected from the group consisting of a light emitting layer, a hole injection layer, a hole transfer layer, an electron injection layer, an electron transfer layer, an electron blocking layer and a hole blocking layer.

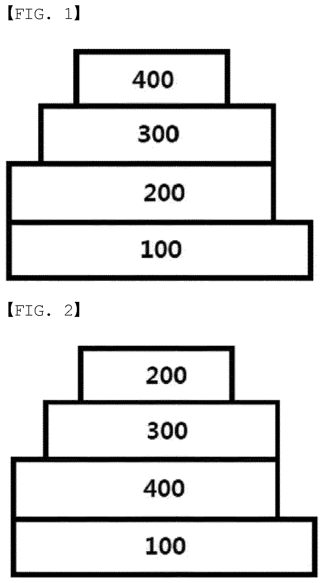

FIG 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

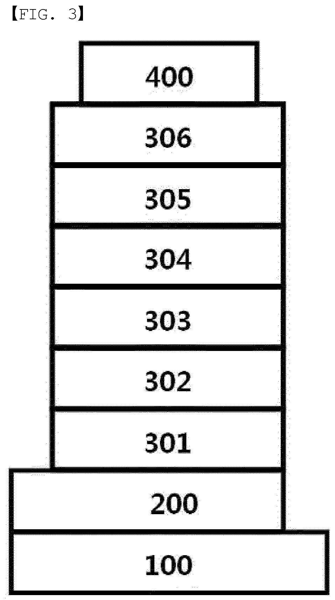

FIG. 3 illustrates a case of the organic material layer being a multilayer. The organic light emitting device according to FIG. 3 comprises a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

The organic material layer comprising the compound of Chemical Formula 1 may further comprise other materials as necessary.

In the organic light emitting device according to one embodiment of the present application, the organic material layer may further comprise a heterocyclic compound of the following Chemical Formula 2.

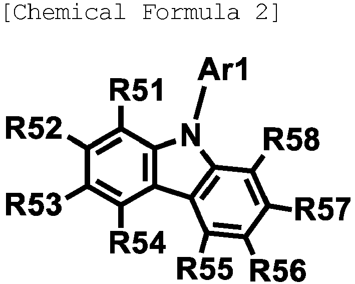

In Chemical Formula 2,
Ar1 is hydrogen; deuterium; a substituted or unsubstituted C1 to C60 alkyl group; a substituted or unsubstituted C6 to C60 aryl group; or a substituted or unsubstituted C2 to C60 heteroaryl group, and
R51 to R58 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted phosphine oxide group; and a substituted or unsubstituted amine group, or two or more groups adjacent to each other bond to each other to form a substituted or unsubstituted C6 to C60 aromatic hydrocarbon ring or a substituted or unsubstituted C2 to C60 heteroring.

Particularly, when further comprising the heterocyclic compound of Chemical Formula 2, an exciplex phenomenon occurs. The exciplex phenomenon refers to forming a bicomplex in an excited state due to electron exchanges between a molecule having strong donor properties and a molecule having strong acceptor properties.

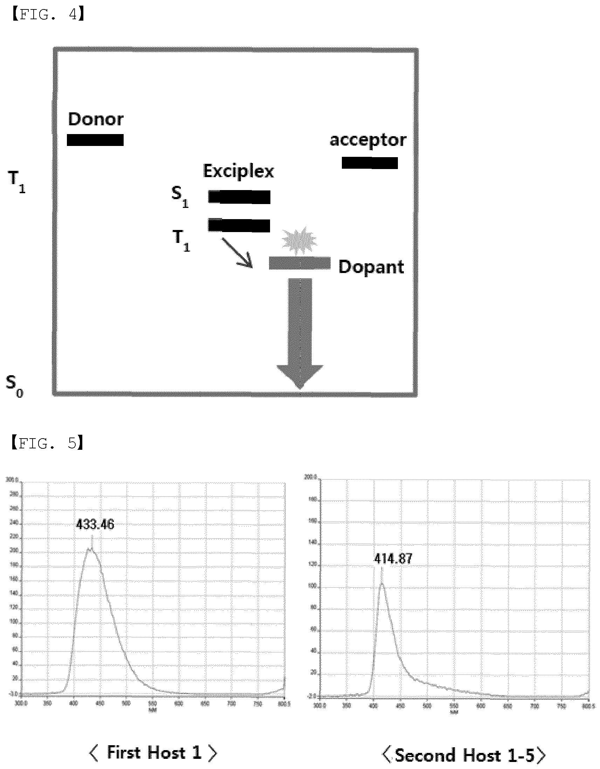

FIG. 4 is a diagram explaining the exciplex phenomenon. When the exciplex phenomenon occurs as in FIG. 4, new S₁ energy level and T₁ energy level are formed, and red shifted changes in PL may be identified compared to in each of the molecules.

In other words, when exciplex that is a bicomplex form in an excited state is formed between donor and acceptor molecules, a new energy level different from energy levels of the donor and the acceptor is obtained, and herein, light emitting at this energy level emits red shifted light compared to light emitted by each of the donor and the acceptor, and PL is measured in order to identify this phenomenon. Accordingly, by comparing the emission wavelength of the single host and the emission wavelength of the mixed host from the PL data, the occurrence of exciplex in the molecules may be identified.

When the exciplex phenomenon occurs between two molecules as above, reverse intersystem crossing (RISC) occurs, and as a result, internal quantum efficiency may increase up to 100%.

Particularly, the compound of Chemical Formula 1 is a bipolar compound and does not have a strong acceptor ability, however, by introducing a donor (p-host) that is the heterocyclic compound of Chemical Formula 2 having a favorable hole transfer ability, exciplex may be formed based on the observation of red shifted changes in the PL, which resultantly helps with enhancement in light emission properties. In addition, by introducing the compound (donor(p-host)) corresponding to Chemical Formula 2 of the present application having a favorable hole transfer ability, the lifetime may be significantly improved due to a proper movement of a light emitting zone in a light emitting layer.

In one embodiment of the present application, Chemical Formula 2 may be represented by any one of the following Chemical Formulae 10 to 12.

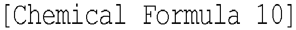

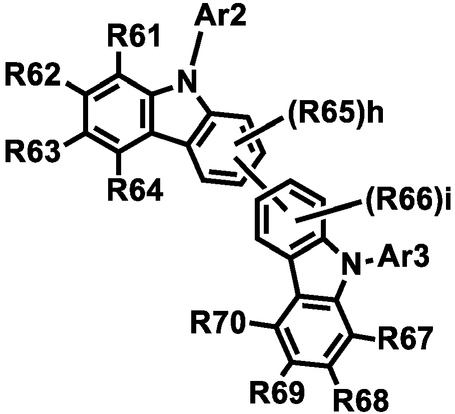

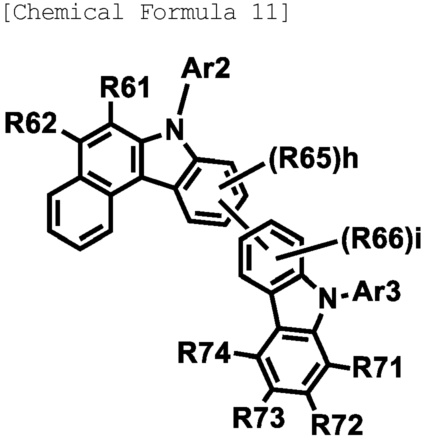

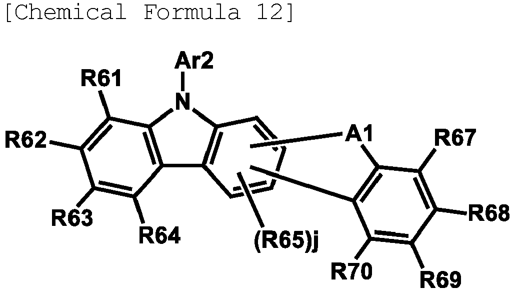

In Chemical Formulae 10 to 12,
R61 to R70 are the same as or different from each other, and each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group,
R71 to R74 are the same as or different from each other, and each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring,
Ar2 and Ar3 are the same as or different from each other, and each independently hydrogen; deuterium; a substituted or unsubstituted C1 to C60 alkyl group; a substituted or unsubstituted C6 to C60 aryl group; or a substituted or unsubstituted C2 to C60 heteroaryl group,
A1 is O; S; NAr4; or CRdRe,
Rd and Re are the same as or different from each other, and each independently a substituted or unsubstituted C1 to C60 alkyl group; or a substituted or unsubstituted C6 to C60 aryl group,
Ar4 is hydrogen; deuterium; a substituted or unsubstituted C1 to C60 alkyl group; a substituted or unsubstituted C6 to C60 aryl group; or a substituted or unsubstituted C2 to C60 heteroaryl group,
h and i are an integer of 0 to 3, and
j is an integer of 0 to 2.

In one embodiment of the present application, Chemical Formula 10 may be represented by the following Chemical Formula 10-1 or 10-2.

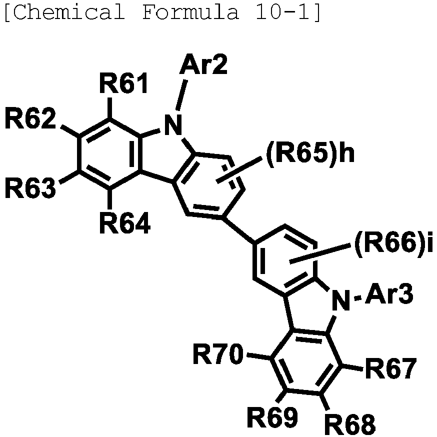

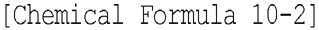

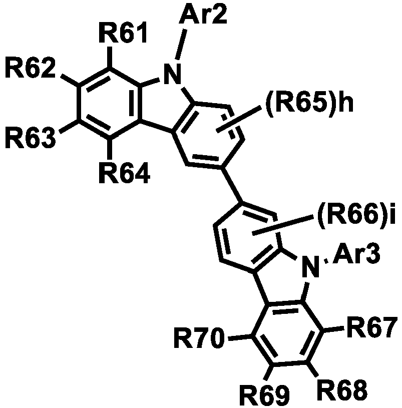

In Chemical Formulae 10-1 and 10-2,
each substituent has the same definition as in Chemical Formula 10.

In one embodiment of the present application, Chemical Formula 11 may be represented by the following Chemical Formula 11-1 or 11-2.

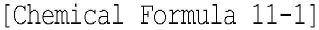

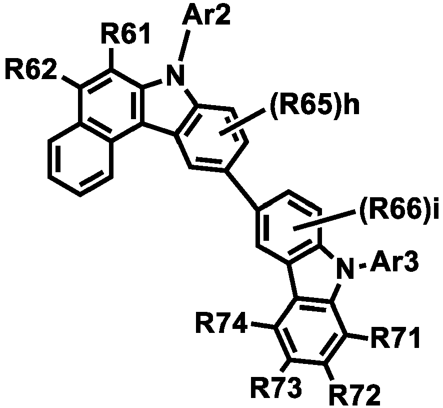

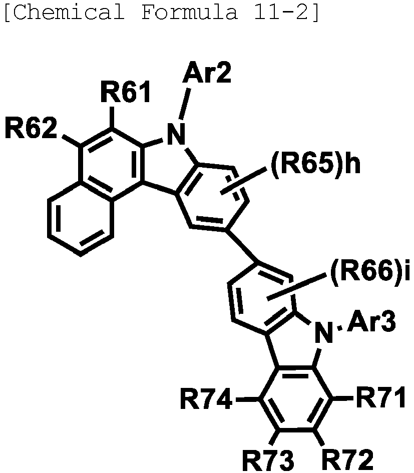

In Chemical Formulae 11-1 and 11-2,
each substituent has the same definition as in Chemical Formula 11.

In one embodiment of the present application, Chemical Formula 12 may be represented by any one of the following Chemical Formulae 12-1 to 12-4.

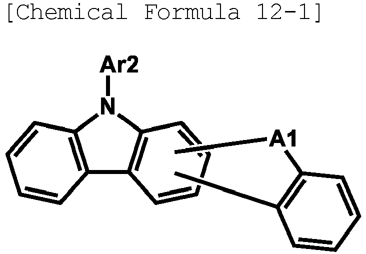

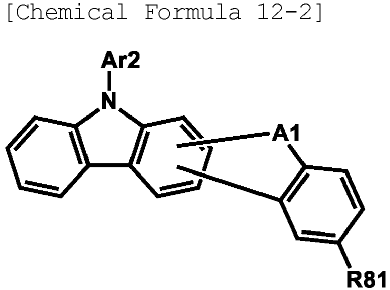

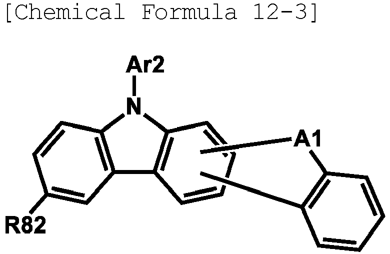

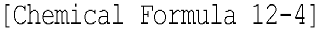

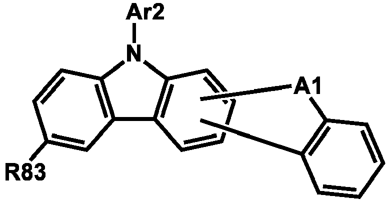

In Chemical Formulae 12-1 to 12-4,
Ar2 and Al have the same definitions as in Chemical Formula 12,
R81 and R82 are the same as or different from each other, and each independently a substituted or unsubstituted C10 or higher aryl group; or a substituted or unsubstituted C2 to C60 heteroaryl group, and
R83 is a substituted or unsubstituted lower than C10 aryl group.

In one embodiment of the present application, Ar2 to Ar4 are the same as or different from each other, and may be each independently hydrogen; deuterium; a substituted or unsubstituted C1 to C60 alkyl group; a substituted or unsubstituted C6 to C60 aryl group; or a substituted or unsubstituted C2 to C60 heteroaryl group.

In another embodiment, Ar2 to Ar4 are the same as or different from each other, and may be each independently hydrogen; deuterium; a substituted or unsubstituted C1 to C40 alkyl group; a substituted or unsubstituted C6 to C40 aryl group; or a substituted or unsubstituted C2 to C40 heteroaryl group.

In another embodiment, Ar2 to Ar4 are the same as or different from each other, and may be each independently hydrogen; deuterium; a C6 to C40 aryl group unsubstituted or substituted with one or more substituents selected from the group consisting of a halogen group, a C1 to C20 alkyl group, a C6 to C40 aryl group and a C2 to C40 heteroaryl group; or a C2 to C40 heteroaryl group unsubstituted or substituted with one or more substituents selected from the group consisting of deuterium, a C6 to C40 aryl group and a C2 to C40 heteroaryl group.

In one embodiment of the present application, R61 to R70 may be hydrogen; or deuterium.

In one embodiment of the present application, R71 to R74 are the same as or different from each other, and each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group, or two or more groups adjacent to each other may bond to each other to form a substituted or unsubstituted C6 to C60 aromatic hydrocarbon ring or a substituted or unsubstituted C2 to C60 heteroring.

In another embodiment, R71 to R74 are the same as or different from each other, and each independently hydrogen; or deuterium, or two or more groups adjacent to each other may bond to each other to form a substituted or unsubstituted C6 to C60 aromatic hydrocarbon ring or a substituted or unsubstituted C2 to C60 heteroring.

In another embodiment, R71 to R74 are the same as or different from each other, and each independently hydrogen; or deuterium, or two or more groups adjacent to each other may bond to each other to form a substituted or unsubstituted C6 to C60 aromatic hydrocarbon ring.

In another embodiment, R71 to R74 are the same as or different from each other, and each independently hydrogen; or deuterium, or two or more groups adjacent to each other may bond to each other to form a C6 to C60 aromatic hydrocarbon ring.

In another embodiment, R71 to R74 are the same as or different from each other, and each independently hydrogen; or deuterium, or two or more groups adjacent to each other may bond to each other to form a C6 to C40 aromatic hydrocarbon ring.

In another embodiment, R71 to R74 are the same as or different from each other, and each independently hydrogen; or deuterium, or two or more groups adjacent to each other may bond to each other to form a benzene ring.

In one embodiment of the present application, Rd and Re are the same as or different from each other, and may be each independently a substituted or unsubstituted C1 to C60 alkyl group; or a substituted or unsubstituted C6 to C60 aryl group.

In another embodiment, Rd and Re are the same as or different from each other, and may be each independently a substituted or unsubstituted C1 to C60 alkyl group.

In another embodiment, Rd and Re are the same as or different from each other, and may be each independently a C1 to C40 alkyl group.

In another embodiment, Rd and Re may be a methyl group.

In one embodiment of the present application, R81 and R82 are the same as or different from each other, and may be each independently a substituted or unsubstituted C10 or higher aryl group; or a substituted or unsubstituted C2 to C60 heteroaryl group.

In another embodiment, R81 and R82 are the same as or different from each other, and may be each independently a substituted or unsubstituted C10 or higher and C60 or lower aryl group; or a substituted or unsubstituted C2 to C60 heteroaryl group.

In another embodiment, R81 and R82 are the same as or different from each other, and may be each independently a substituted or unsubstituted C10 or higher and C40 or lower aryl group; or a substituted or unsubstituted C2 to C40 heteroaryl group.

In another embodiment, R81 and R82 are the same as or different from each other, and may be each independently a C10 or higher and C40 or lower aryl group unsubstituted or substituted with a C1 to C20 alkyl group; or a C2 to C40 heteroaryl group unsubstituted or substituted with one or more substituents selected from the group consisting of a C6 to C40 aryl group and a C2 to C40 heteroaryl group.

In another embodiment, R81 and R82 may be represented by the following Chemical Formula 13.

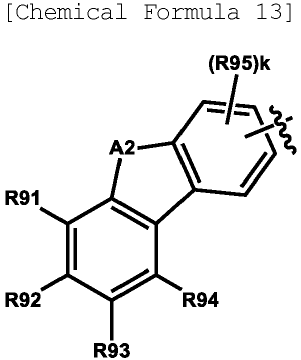

In Chemical Formula 13,
A2 is NR96; O; S; or CR97R98,
R91 to R95 are the same as or different from each other, and each independently hydrogen; deuterium; a substituted or unsubstituted C6 to C60 aryl group; or a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring,
R96 to R98 are the same as or different from each other, and each independently hydrogen; deuterium; a substituted or unsubstituted C1 to C60 alkyl group; a substituted or unsubstituted C6 to C60 aryl group; or a substituted or unsubstituted C2 to C60 heteroaryl group, and
k is an integer of 0 to 3.

In one embodiment of the present application, R83 is a substituted or unsubstituted lower than C10 aryl group.

In another embodiment, R83 is a lower than C10 aryl group unsubstituted or substituted with a C6 to C10 aryl group or a C2 to C20 heteroaryl group.

In another embodiment, R83 may be a phenyl group unsubstituted or substituted with a phenyl group or a dibenzofuran group; a biphenyl group; or a naphthyl group.

In one embodiment of the present application, R91 to R95 are the same as or different from each other, and each independently hydrogen; deuterium; or a substituted or unsubstituted C6 to C60 aryl group, or two or more groups adjacent to each other may bond to each other to form a substituted or unsubstituted C6 to C60 aromatic hydrocarbon ring.

In one embodiment of the present application, R91 to R95 are the same as or different from each other, and each independently hydrogen; or deuterium, or two or more groups adjacent to each other may bond to each other to form a benzene ring.

In one embodiment of the present application, R96 to R98 are the same as or different from each other, and may be each independently hydrogen; deuterium; a substituted or unsubstituted C1 to C60 alkyl group; a substituted or unsubstituted C6 to C60 aryl group; or a substituted or unsubstituted C2 to C60 heteroaryl group.

In another embodiment, R96 to R98 are the same as or different from each other, and may be each independently hydrogen; deuterium; a C1 to C20 alkyl group; or a C6 to C40 aryl group unsubstituted or substituted with one or more substituents selected from the group consisting of a C1 to C20 alkyl group, a C6 to C40 aryl group and a C2 to C40 heteroaryl group.

In one embodiment of the present application, the heterocyclic compound represented by Chemical Formula 2 may be any one of the following compounds.

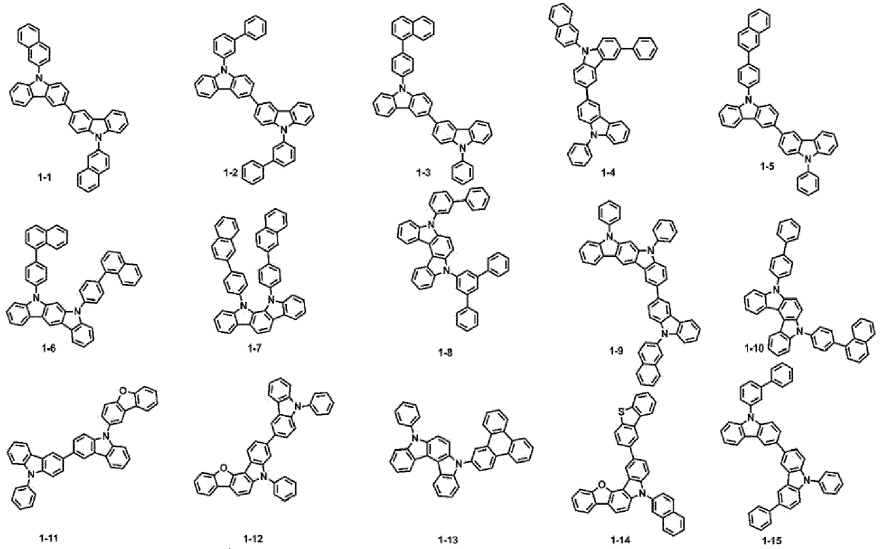

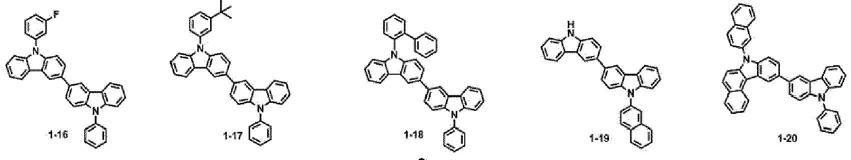

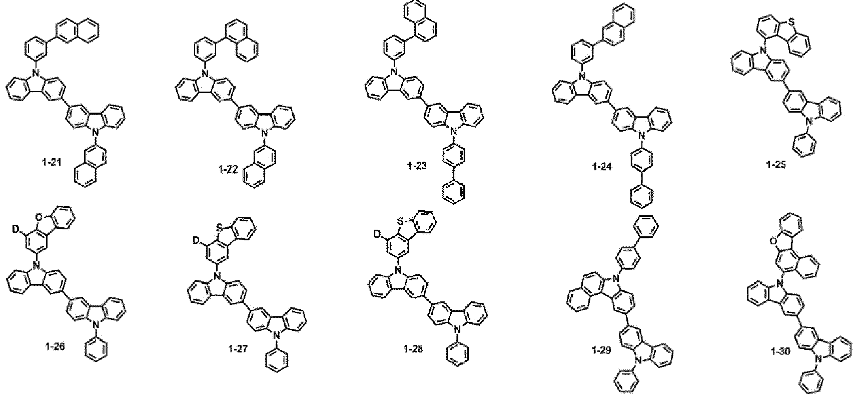

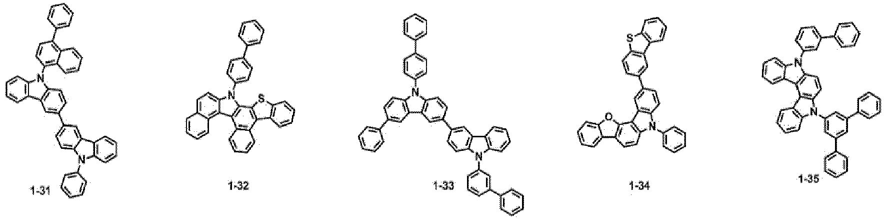

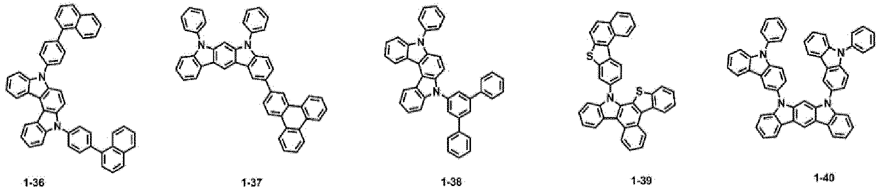

In the organic light emitting device according to one embodiment of the present application, Chemical Formula 2 may be included in a light emitting layer of the organic material layer.

In the organic light emitting device according to one embodiment of the present application, Chemical Formula 2 may be included in a light emitting layer of the organic material layer, and may be specifically used as a host material of the light emitting layer.

In one embodiment of the present application, the host material of the light emitting layer of the organic light emitting device may comprise the heterocyclic compound of Chemical Formula 1 and the compound of Chemical Formula 2 at the same time.

One embodiment of the present application provides a composition for an organic material layer of an organic light emitting device, the composition comprising the heterocyclic compound represented by Chemical Formula 1 and the compound represented by Chemical Formula 2.

In the composition, the heterocyclic compound represented by Chemical Formula 1: the heterocyclic compound represented by Chemical Formula 2 may have a weight ratio of 1:10 to 10:1, and the weight ratio may be from 1:8 to 8:1, 1:5 to 5:1 or 1:2 to 2:1, but is not limited thereto.

One embodiment of the present application provides a method for manufacturing an organic light emitting device, the method comprising preparing a substrate; forming a first electrode on the substrate; forming one or more organic material layers on the first electrode; and forming a second electrode on the organic material layer, wherein the forming of organic material layers comprises forming one or more organic material layers using the composition for an organic material layer according to one embodiment of the present application.

In the method for manufacturing an organic light emitting device provided in one embodiment of the present application, the forming of organic material layers is forming the heterocyclic compound represented by Chemical Formula 1 and the compound represented by Chemical Formula 2 using a thermal vacuum deposition method after pre-mixing.

The pre-mixing means first mixing the heterocyclic compound represented by Chemical Formula 1 and the compound represented by Chemical Formula 2 in one source of supply before depositing on the organic material layer.

The premixed material may be referred to as the composition for an organic material layer according to one embodiment of the present application.

In the organic light emitting device according to one embodiment of the present application, materials other than the compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material comprise metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material comprise metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present application may be a top-emission type, a bottom-emission type or a dual-emission type depending on the materials used.

The heterocyclic compound according to one embodiment of the present application may also be used in an organic electronic device comprising an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example>

### <Preparation Example 1> Preparation of Compound 1

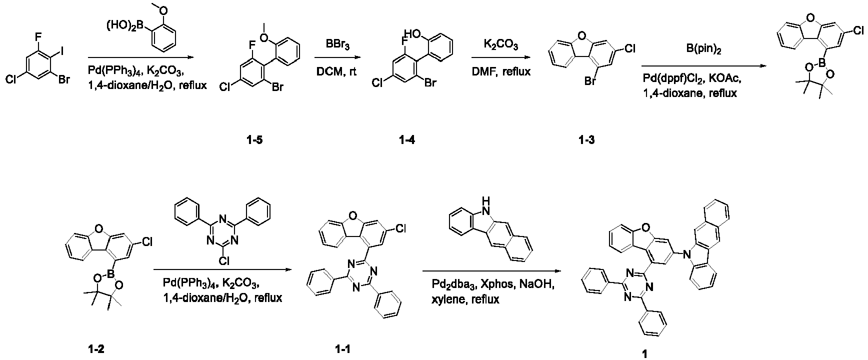

### 1) Preparation of Compound 1-5

After dissolving 1-bromo-5-chloro-3-fluoro-2-iodobenzene (200.0 g, 596.4 mmol), (2-methoxyphenyl)boronic acid (82.4 g, 542.2 mmol), Pd(PPh)₄ (31.3 g, 27.1 mmol) and K₂CO₃ (150.0 g, 1084.4 mmol) in 1,4-dioxane/H₂O (1 L/200 mL), the mixture was refluxed for 24 hours. After the reaction was completed, the result was extracted by introducing distilled water and dichloromethane (DCM) thereto at room temperature, and after drying the organic layer with MgSO₄, the solvent was removed using a rotary evaporator. The reaction material was purified by column chromatography (DCM:Hex=1:10) to obtain target Compound 1-5 (137 g, 80%).

### 2) Preparation of Compound 1-4

After dissolving Compound 1-5 (82 g, 259.8 mmol) and BBr₃ (49 mL, 519.7 mol) in DCM (800 mL), the mixture was refluxed for 1 hour. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:1) to obtain target Compound 1-4 (65.3 g, 83%).

### 3) Preparation of Compound 1-3

After dissolving Compound 1-4 (65.3 g, 216.5 mM) and K₂CO₃ (59.9 g, 433.1 mmol) in dimethylformamide (DMF) (300 mL), the mixture was refluxed for 4 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:5), and recrystallized with methanol to obtain target Compound 1-3 (54.8 g, 90%).

### 4) Preparation of Compound 1-2

After dissolving Compound 1-3 (20.0 g, 70 mmol), bis(pinacolato)diboron (23 g, 92 mmol), Pd(dppf)Cl₂ (2.6 g, 3.55 mmol) and KOAc (20.9 g, 213 mmol) in 1,4-dioxane (350 mL), the mixture was refluxed for 2 hours. After the reaction was completed, the result was extracted by introducing distilled water and DCM thereto at room temperature, and after drying the organic layer with MgSO₄, the solvent was removed using a rotary evaporator. The reaction material was purified by silica, and recrystallized with hexane to obtain target Compound 1-2 (20.7 g, 89%).

### 5) Preparation of Compound 1-1

After dissolving Compound 1-2 (10.7 g, 32.6 mmol), 2-chloro-4,6-diphenyl-1,3,5-triazine (8.7 g, 32.6 mmol), Pd(PPh)₄ (1.88 g, 1.63 mmol) and K₂CO₃ (13.5 g, 98 mmol) in 1,4-dioxane/H₂O (160 mL/30 mL), the mixture was refluxed for 6 hours. After the reaction was completed, produced solids were filtered, and washed with dioxane/distilled water/acetone to obtain target Compound 1-1 (12.4 g, 88%).

### 6) Preparation of Compound 1

After dissolving Compound 1-1 (6 g, 13.8 mmol), 5H-benzo[b]carbazole (3 g, 13.8 mmol), Pd₂(dba)₃ (1.2 g, 1.38 mmol), Xphos (1.3 g, 2.8 mmol) and NaPtBu (4 g, 41.4 mmol) in xylene (70 mL), the mixture was refluxed for 12 hours. After the reaction was completed, produced solids were filtered, washed with distilled water, and dried. The dried solids were boiled and dissolved in DCB, and purified by silica, and then the solvent was removed using a rotary evaporator. The result was recrystallized with acetone to obtain target Compound 1 (7.8 g, 86%) .

Target Compound A was synthesized in the same manner as in Preparation Example 1 except that Intermediate A of the following Table 1 was used instead of 2-chloro-4,6-diphenyl-1,3,5-triazine, and Intermediate B of the following Table 1 was used instead of 5H-benzo[b]carbazole.

**[Table 1]**

| Compou nd No. | Intermediate A | Intermediate B | Target Compound A | Yield |
|---|---|---|---|---|
| 2 | 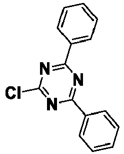 | 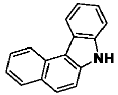 | 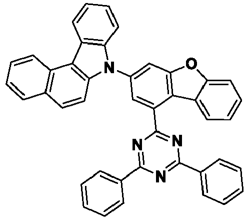 | 70% |
| 3 | | 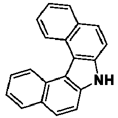 | 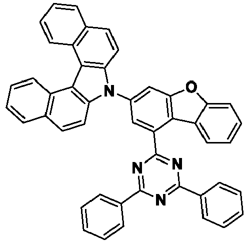 | 73% |
| 6 | | 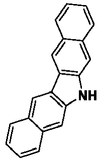 | 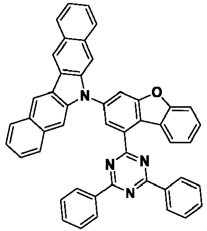 | 60% |
| 11 | | 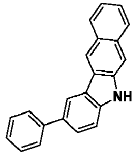 | 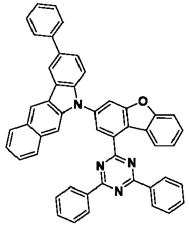 | 52% |
| 14 | | 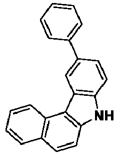 | 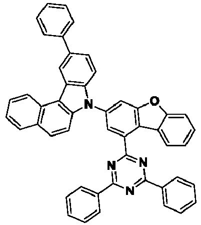 | 73% |
| 41 | | 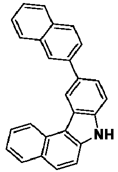 | 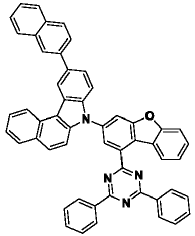 | 80% |
| 102 | 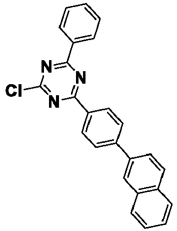 | 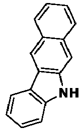 | 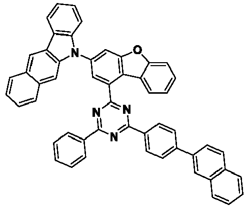 | 82% |
| 103 | 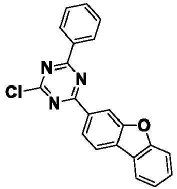 | | 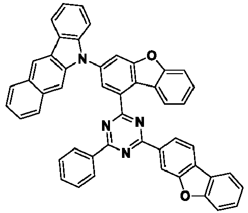 | 78% |
| 105 | 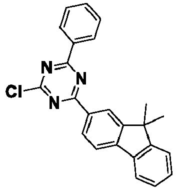 | | 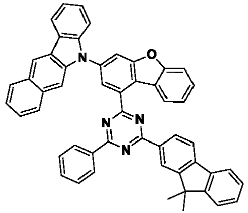 | 80% |
| 106 | 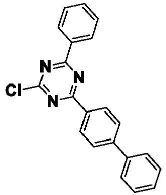 | | 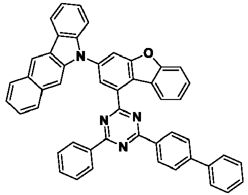 | 77% |
| 108 | 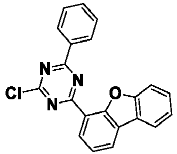 | | 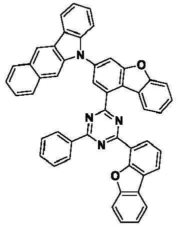 | 67% |
| 113 | 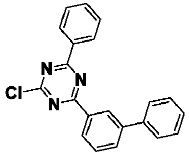 | | 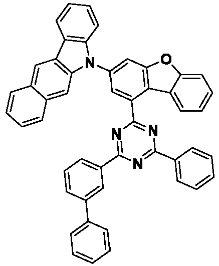 | 86% |
| 115 | 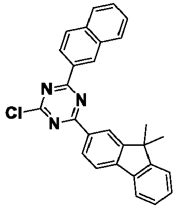 | | 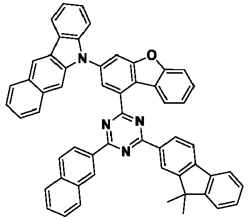 | 75% |
| 119 | 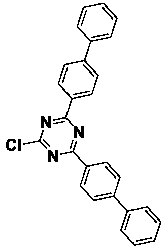 | | 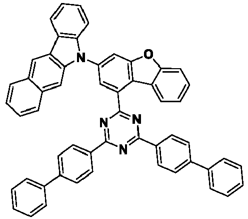 | 81% |
| 120 | 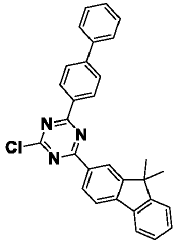 | | 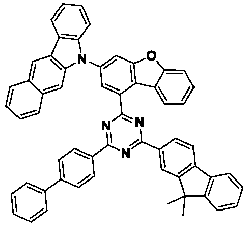 | 72% |
| 121 | 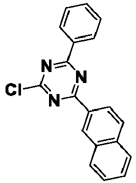 | 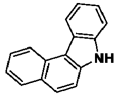 | 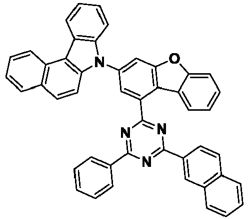 | 68% |
| 124 | 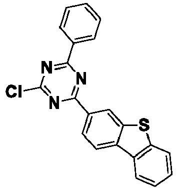 | | 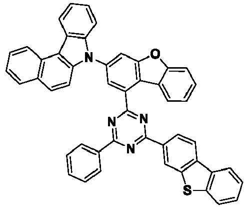 | 88% |
| 125 | 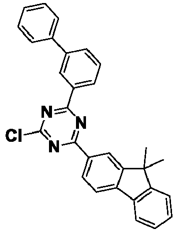 | | 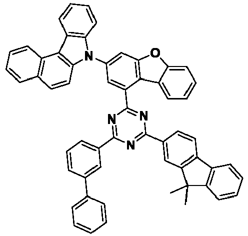 | 87% |
| 126 | 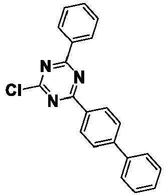 | | 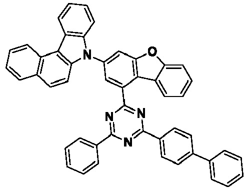 | 79% |
| 135 | 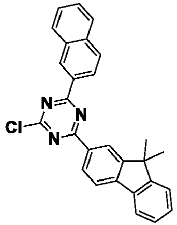 | | 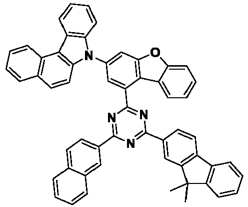 | 77% |
| 140 | 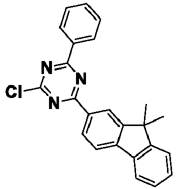 | 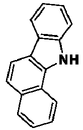 | 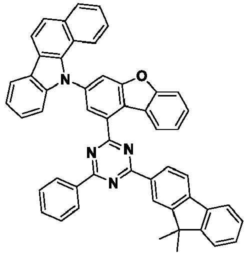 | 81% |
| 145 | 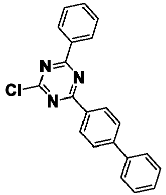 | 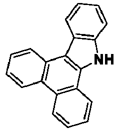 | 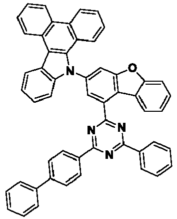 | 80% |
| 147 | 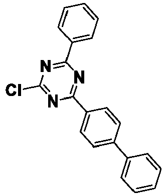 | 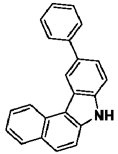 | 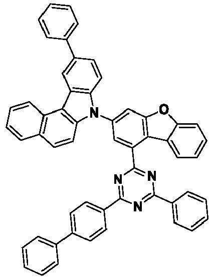 | 69% |
| 201 | 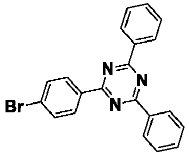 | 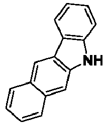 | 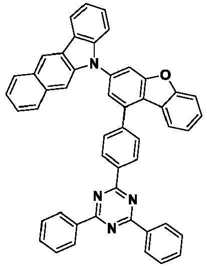 | 79% |
| 202 | 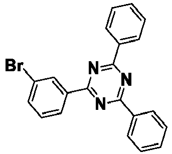 | 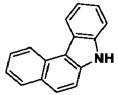 | 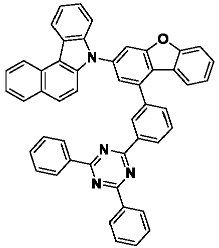 | 74% |
| 204 | 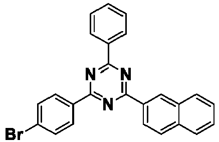 | 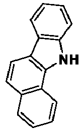 | 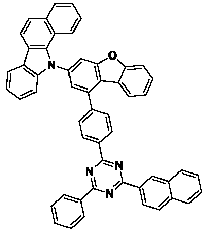 | 80% |
| 206 | 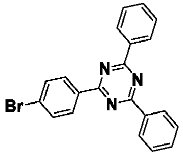 | 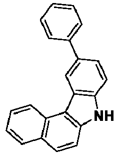 | 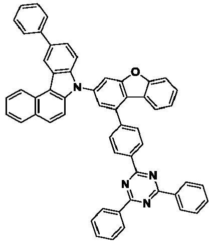 | 82% |
| 224 | 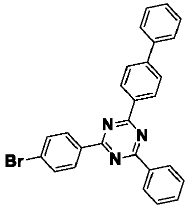 | 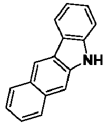 | 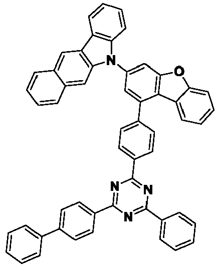 | 75% |
| 301 | 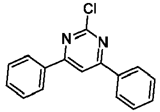 | 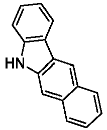 | 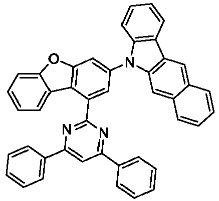 | 72% |
| 304 | 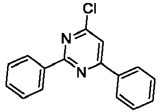 | 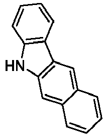 | 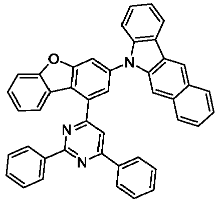 | 76% |
| 307 | 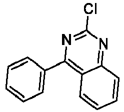 | 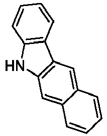 | 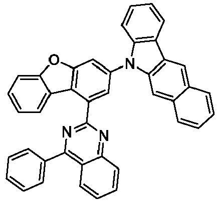 | 80% |
| 309 | 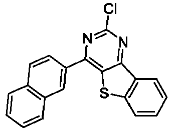 | 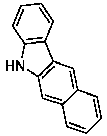 | 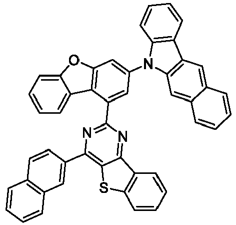 | 71% |
| 402 | 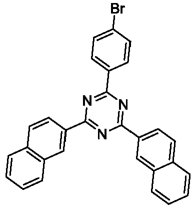 | 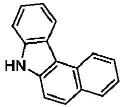 | 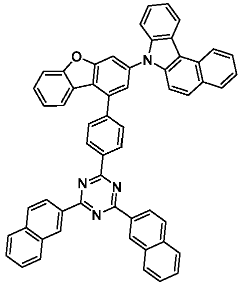 | 73% |
| 501 | 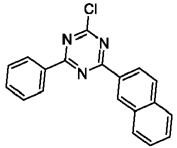 | 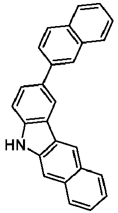 | 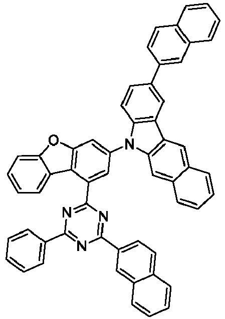 | 75% |
| 504 | 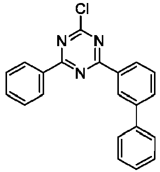 | 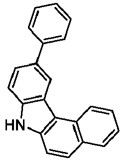 | 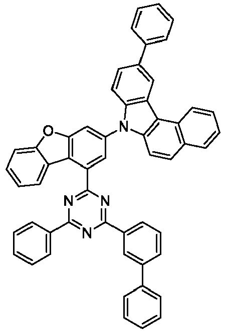 | 72% |
| 601 | 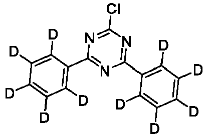 | 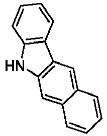 | 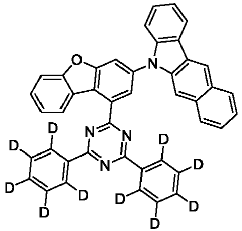 | 57% |

### <Preparation Example 2> Preparation of Compound 21

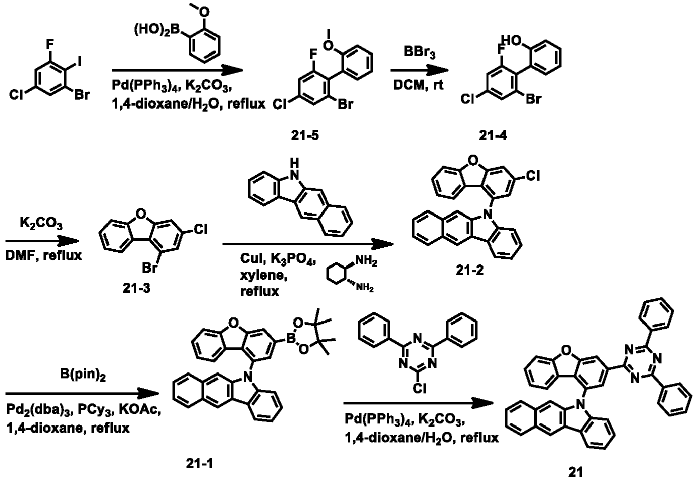

### 1) Preparation of Compound 21-2

After dissolving Compound 21-3 (11.0 g, 39.1 mmol), 5H-benzo[b]carbazole (8.49 g, 39.1 mmol), CuI (7.44 g, 39.1 mmol), trans-1,2-cyclohexanediamine (4.46 g, 39.1 mmol) and K₃PO₄ (16.59 g, 78.14 mM) in xylene (200 mL), the mixture was refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:4), and recrystallized with methanol to obtain target Compound 21-2 (12.4 g, 76%).

### 2) Preparation of Compound 21-1

After dissolving Compound 21-2 (12.4 g, 29.7 mmol), bis(pinacolato)diboron (8.29 g, 32.64 mmol), Pd₂(dba)₃ (1.36 g, 1.48 mmol), PCy₃ (1.25 g, 4.45 mmol) and KOAc (5.82 g, 59.3 mM) in 1,4-dioxane (200 mL), the mixture was refluxed for 4 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain target Compound 21-1 (13.5 g, 89%).

### 3) Preparation of Compound 21

After dissolving Compound 21-1 (13.5 g, 26.4 mmol), 2-chloro-4,6-diphenyl-1,3,5-triazine (7.8 g, 29.0 mM), Pd(PPh)₄ (1.53 g, 1.3 mM) and K₂CO₃ (7.33 g, 53.0 mM) in 1,4-dioxane/H₂O (150 mL/30mL), the mixture was refluxed for 4 hours. After the reaction was completed, produced solids were washed with distilled water and acetone, and dried. The dried solids were boiled and dissolved in DCB, and purified by silica, and then the solvent was removed using a rotary evaporator. The result was recrystallized with methanol to obtain target Compound 21 (13.4 g, 82%).

Target Compound A was synthesized in the same manner as in Preparation Example 2 except that Intermediate A of the following Table 2 was used instead of 5H-benzo[b]carbazole, and Intermediate B of the following Table 2 was used instead of 2-chloro-4,6-diphenyl-1,3,5-triazine.

**[Table 2]**

| Compou nd No. | Intermediate A | Intermediate B | Target Compound A | Yield |
|---|---|---|---|---|
| 22 | 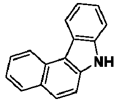 | 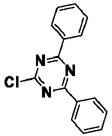 | 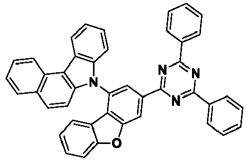 | 45% |
| 34 | 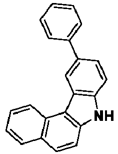 | | 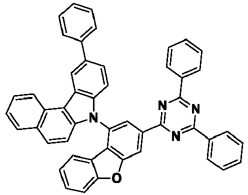 | 55% |
| 37 | 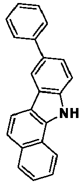 | | 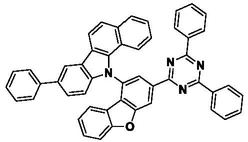 | 50% |
| 187 | 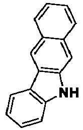 | 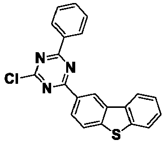 | 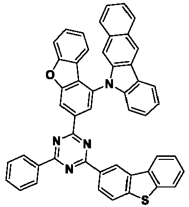 | 60% |
| 198 | 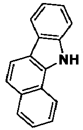 | 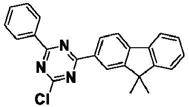 | 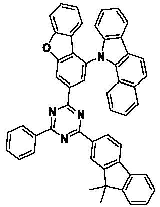 | 44% |
| 208 | 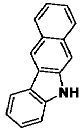 | 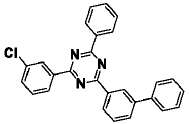 | 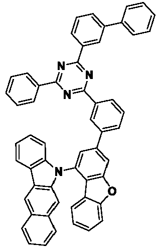 | 46% |
| 212 | 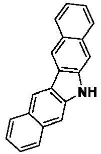 | 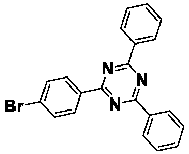 | 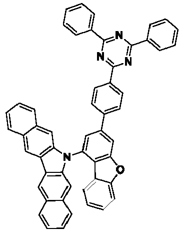 | 51% |
| 333 | 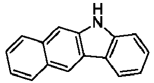 | 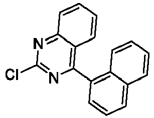 | 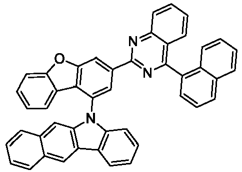 | 72% |
| 335 | 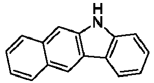 | 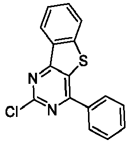 | 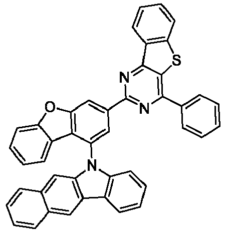 | 67% |
| 419 | 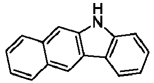 | 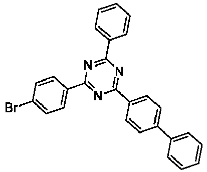 | 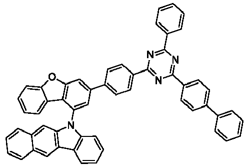 | 65% |
| 509 | 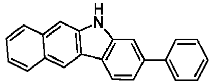 | 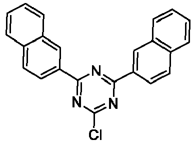 | 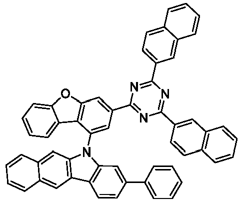 | 71% |
| 510 | 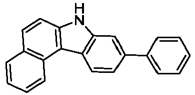 | 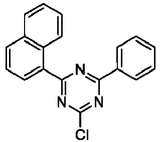 | 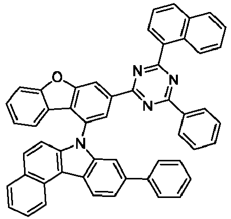 | 80% |
| 617 | 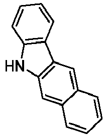 | 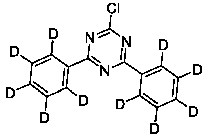 | 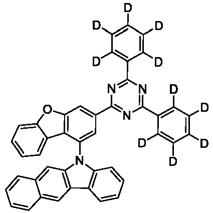 | 59% |

### <Preparation Example 3> Preparation of Compound 51

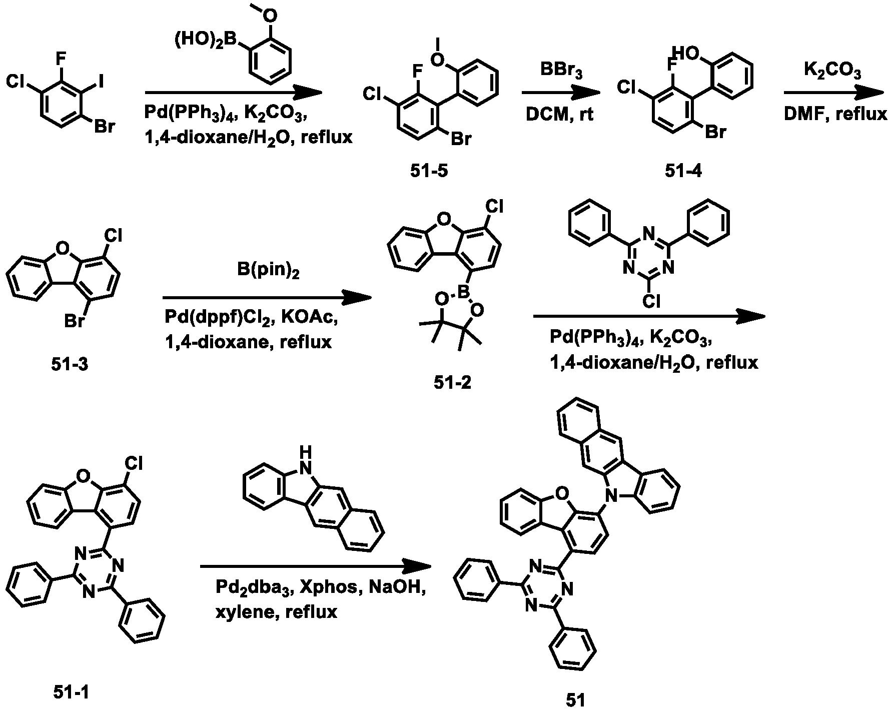

Compound 51 was synthesized in the same manner as in Preparation Example 1 except that 1-bromo-4-chloro-3-fluoro-2-iodobenzene was used instead of 1-bromo-5-chloro-3-fluoro-2-iodobenzene.

Target compounds were synthesized in the same manner as in Preparation Example 3 except that Intermediate A and Intermediate B of the following Table 3 were used.

**[Table 3]**

| Compo und No. | Intermediate A | Intermediate B | Target Compound A | Yield |
|---|---|---|---|---|
| 52 | 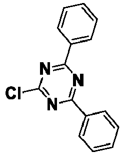 | 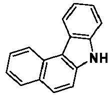 | 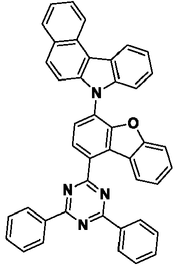 | 55% |
| 53 | | 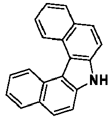 | 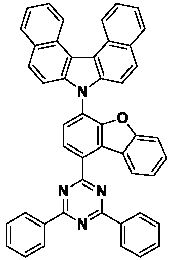 | 60% |
| 57 | | 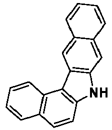 | 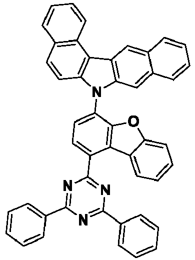 | 57% |
| 62 | | 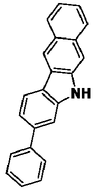 | 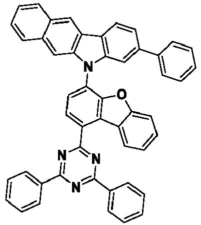 | 61% |
| 65 | | 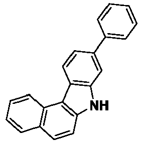 | 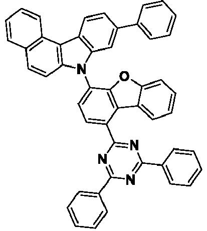 | 53% |
| 72 | | 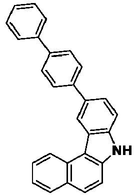 | 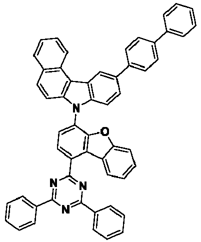 | 64% |
| 151 | 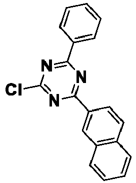 | 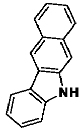 | 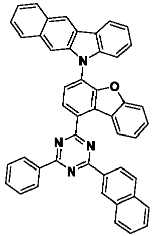 | 51% |
| 155 | 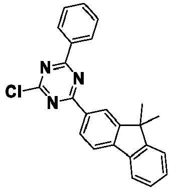 | | 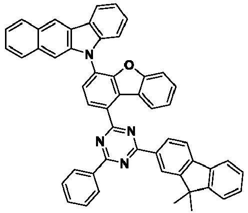 | 49% |
| 157 | 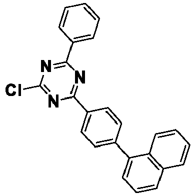 | | 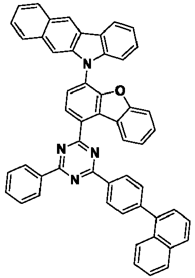 | 45% |
| 158 | 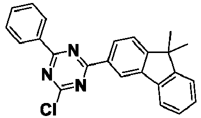 | | 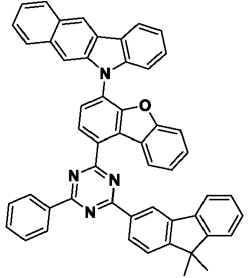 | 67% |
| 160 | 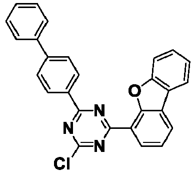 | | 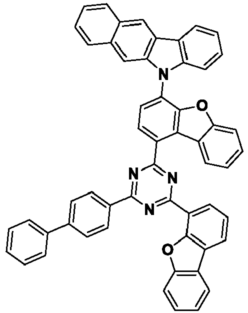 | 57% |
| 161 | 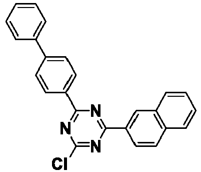 | | 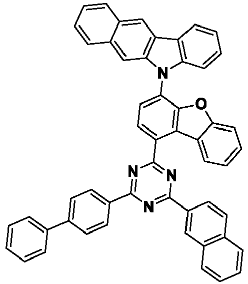 | 56% |
| 162 | 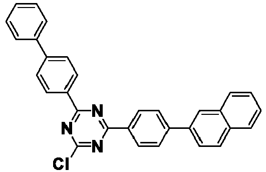 | | 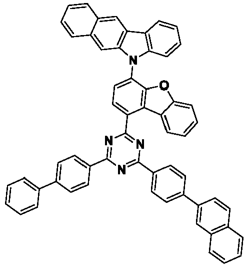 | 50% |
| 163 | 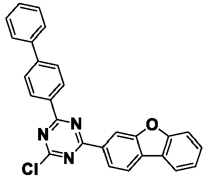 | | 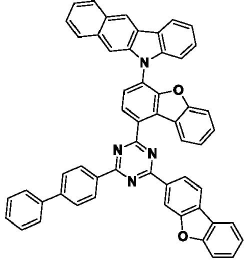 | 49% |
| 169 | 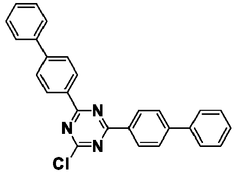 | | 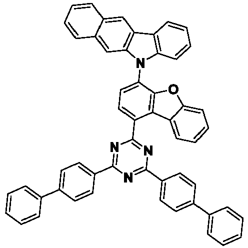 | 61% |
| 170 | 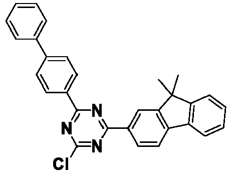 | | 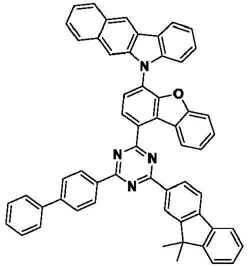 | 62% |
| 172 | 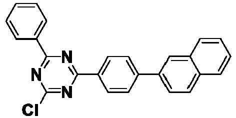 | 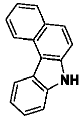 | 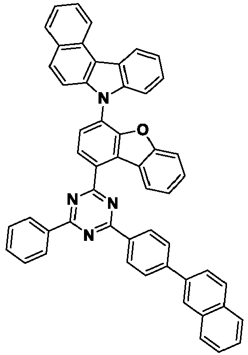 | 58% |
| 175 | 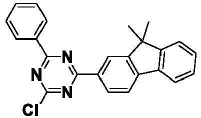 | | 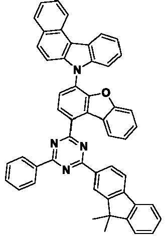 | 45% |
| 177 | 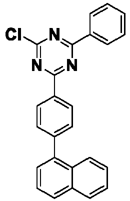 | | 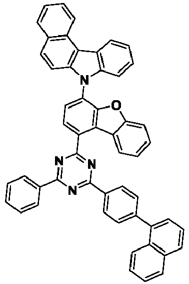 | 66% |
| 179 | 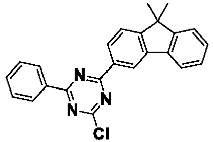 | | 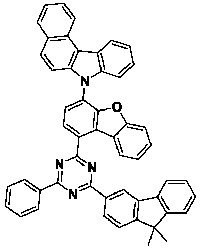 | 54% |
| 215 | 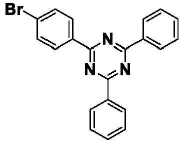 | 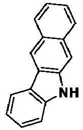 | 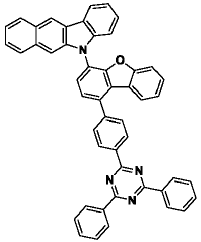 | 42% |
| 220 | 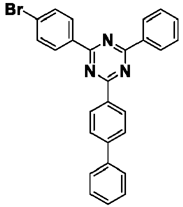 | 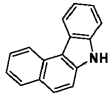 | 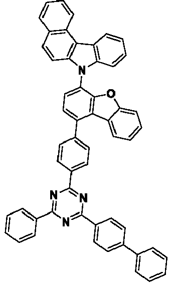 | 67% |
| 319 | 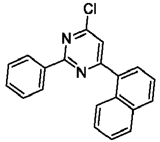 | 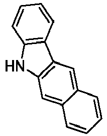 | 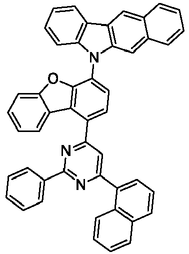 | 71% |
| 323 | 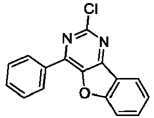 | 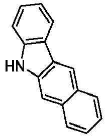 | 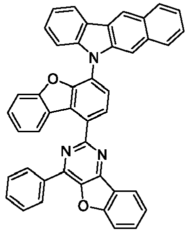 | 65% |
| 411 | 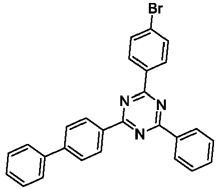 | 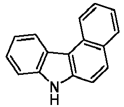 | 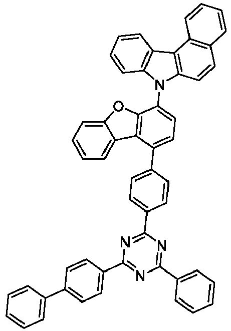 | 73% |
| 413 | 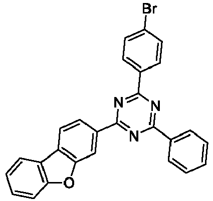 | 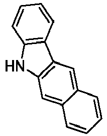 | 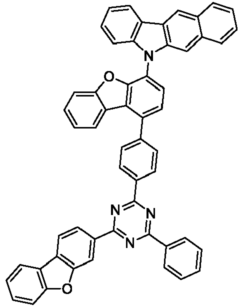 | 69% |
| 506 | 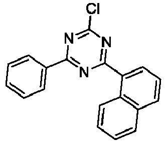 | 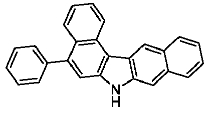 | 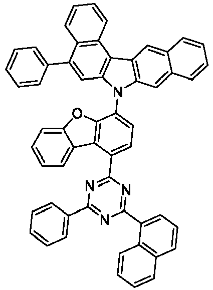 | 81% |
| 613 | 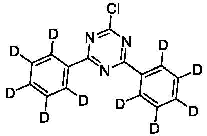 | 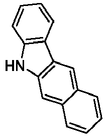 | 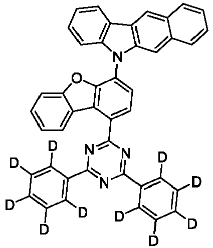 | 61% |

### <Preparation Example 4> Preparation of Compound 76

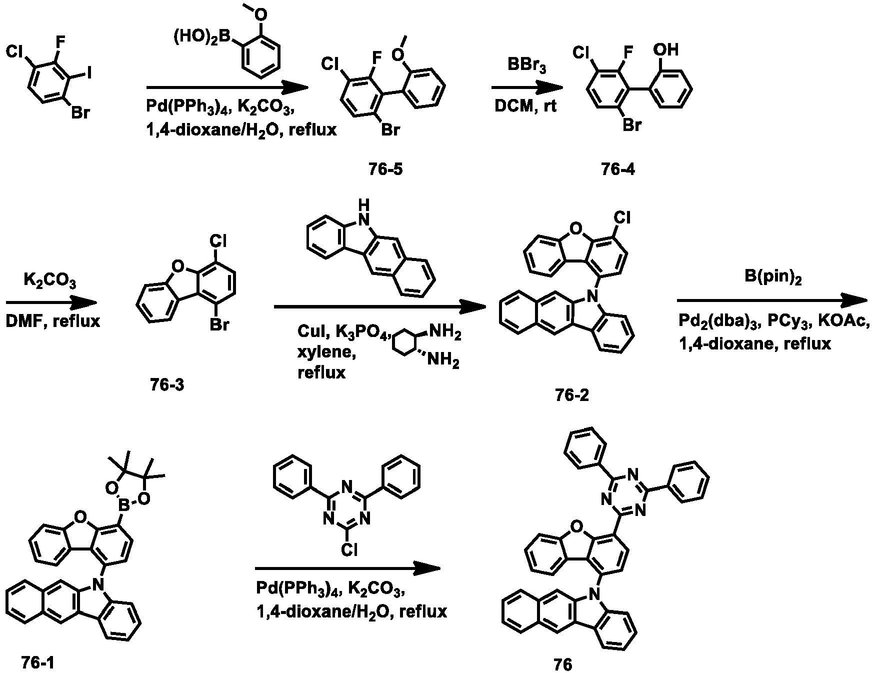

Compound 76 was synthesized in the same manner as in Preparation Example 2 except that 1-bromo-4-chloro-3-fluoro-2-iodobenzene was used instead of 1-bromo-5-chloro-3-fluoro-2-iodobenzene.

Target compounds were synthesized in the same manner as in Preparation Example 4 except that Intermediate A and Intermediate B of the following Table 4 were used.

**[Table 4]**

| Compo und No. | Intermediate A | Intermediate B | Target Compound A | Yield |
|---|---|---|---|---|
| 77 | 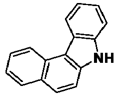 | | 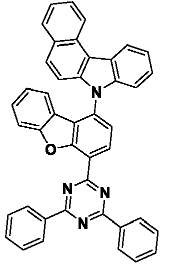 | 36% |
| 79 | 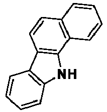 | | 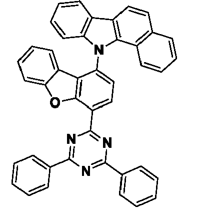 | 38% |
| 81 | 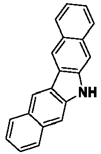 | 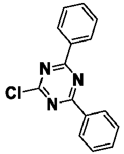 | 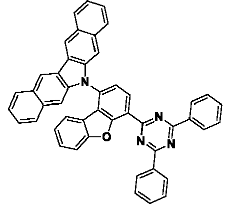 | 40% |
| 88 | 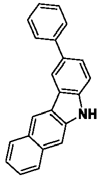 | | 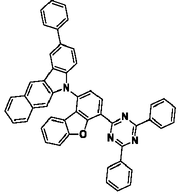 | 37% |
| 95 | 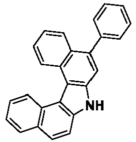 | | 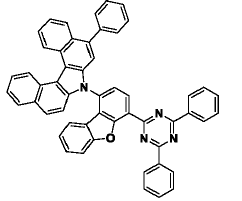 | 42% |
| 96 | 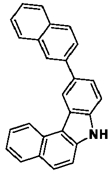 | | 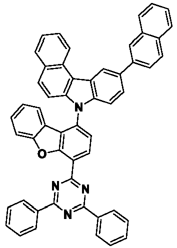 | 55% |
| 222 | 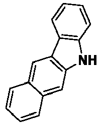 | 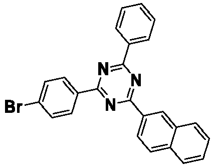 | 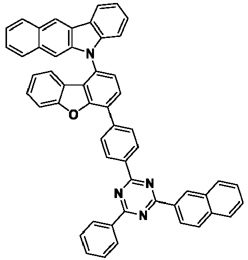 | 41% |
| 340 | 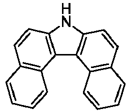 | 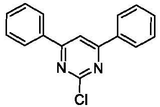 | 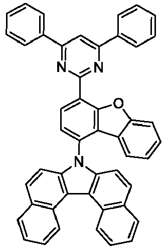 | 56% |
| 342 | 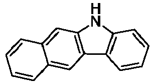 | 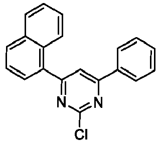 | 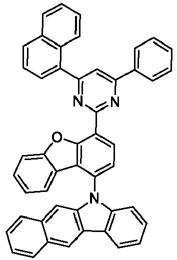 | 47% |
| 346 | 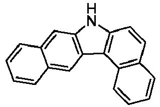 | 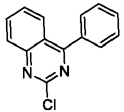 | 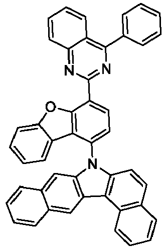 | 51% |
| 430 | 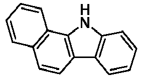 | 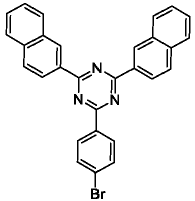 | 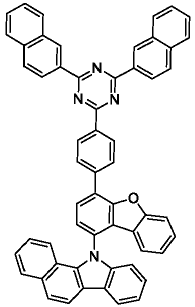 | 61% |
| 516 | 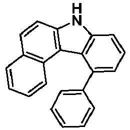 | 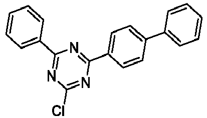 | 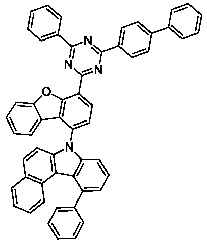 | 54% |
| 621 | 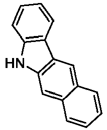 | 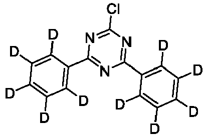 | 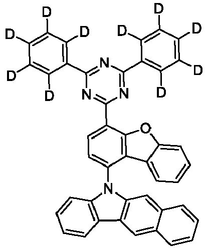 | 47% |

Compounds 1 to 225 and 301 to 624 other than the compounds described in Preparation Examples 1 to 4 and Tables 1 to 4 were also prepared in the same manner as in the preparation examples described above.

Synthesis identification data of the compounds prepared above are as described in the following [Table 5] and [Table 6].

**[Table 5]**

| Com pou nd | FD-Mass | Com pou nd | FD-Mass |
|---|---|---|---|
| 1 | m/z=614.21(C43H26N4O, 614.71) | 2 | m/z=614.21(C43H26N4O, 614.71) |
| 3 | m/z=664.23(C47H28N4O, 664.77) | 4 | m/z=614.21(C₄₃H₂₆N₄O, 614.71) |
| 5 | m/z=664.23(C47H28N4O, 664.77) | 6 | m/z=664.23(C₄₇H₂₈N₄O, 664.77) |
| 7 | m/z=664.23 (C₄₇H₂₈N₄O, 664.77) | 8 | m/z=664.23 (C₄₇H₂₈N₄O, 664.77) |
| 9 | m/z=664.23 (C₄₇H₂₈N₄O, 664.77) | 10 | m/z=664.23 (C₄₇H₂₈N₄O, 664.77) |
| 11 | m/z=690.24(C49H30N4O, 690.81) | 12 | m/z=690.24(C49H30N4O, 690.81) |
| 13 | m/z=690.24(C49H30N4O, 690.81) | 14 | m/z=690.24(C49H30N4O, 690.81) |
| 15 | m/z=690.24(C49H30N4O, 690.81) | 16 | m/z=690.24(C49H30N4O, 690.81) |
| 17 | m/z=690.24(C49H30N4O, 690.81) | 18 | m/z=690.24(C49H30N4O, 690.81) |
| 19 | m/z=690.24(C49H30N4O, 690.81) | 20 | m/z=740.26 (C53H32N4O, 740.87) |
| 21 | m/z=614.21(C43H26N4O, 614.71) | 22 | m/z=614.21(C43H26N4O, 614.71) |
| 23 | m/z=664.23(C47H28N4O, 664.77) | 24 | m/z=614.21(C₄₃H₂₆N₄O, 614.71) |
| 25 | m/z=664.23(C47H28N4O, 664.77) | 26 | m/z=664.23(C₄₇H₂₈N₄O, 664.77) |
| 27 | m/z=664.23 (C₄₇H₂₈N₄O, 664.77) | 28 | m/z=664.23 (C₄₇H₂₈N₄O, 664.77) |
| 29 | m/z=664.23 (C₄₇H₂₈N₄O, 664.77) | 30 | m/z=664.23 (C₄₇H₂₈N₄O, 664.77) |
| 31 | m/z=690.24(C49H30N4O, 690.81) | 32 | m/z=690.24(C49H30N4O, 690.81) |
| 33 | m/z=690.24(C49H30N4O, 690.81) | 34 | m/z=690.24(C49H30N4O, 690.81) |
| 35 | m/z=690.24(C49H30N4O, 690.81) | 36 | m/z=690.24(C49H30N4O, 690.81) |
| 37 | m/z=690.24(C49H30N4O, 690.81) | 38 | m/z=690.24(C49H30N4O, 690.81) |
| 39 | m/z=690.24(C49H30N4O, 690.81) | 40 | m/z=740.26 (C53H32N4O, 740.87) |
| 41 | m/z=740.26(C53H32N4O, 740.87) | 42 | m/z=766.27(C55H34N4O, 766.90) |
| 43 | m/z=766.27(C55H34N4O, 766.90) | 44 | m/z=766.27(C55H34N4O, 766.90) |
| 45 | m/z=766.27(C55H34N4O, 766.90) | 46 | m/z=740.26(C53H32N4O, 740.87) |
| 47 | m/z=766.27(C55H34N4O, 766.90) | 48 | m/z=740.26(C53H32N4O, 740.87) |
| 49 | m/z=766.27(C55H34N4O, 766.90) | 50 | m/z=740.26(C53H32N4O, 740.87) |
| 51 | m/z=614.21(C43H26N4O, 614.71) | 52 | m/z=614.21(C43H26N4O, 614.71) |
| 53 | m/z=664.23(C47H28N4O, 664.77) | 54 | m/z=614.21(C₄₃H₂₆N₄O, 614.71) |
| 55 | m/z=664.23(C47H28N4O, 664.77) | 56 | m/z=664.23(C₄₇H₂₈N₄O, 664.77) |
| 57 | m/z=664.23 (C₄₇H₂₈N₄O, 664.77) | 58 | m/z=664.23 (C₄₇H₂₈N₄O, 664.77) |
| 59 | m/z=664.23 (C₄₇H₂₈N₄O, 664.77) | 60 | m/z=664.23 (C₄₇H₂₈N₄O, 664.77) |
| 61 | m/z=690.24(C49H30N4O, 690.81) | 62 | m/z=690.24(C49H30N4O, 690.81) |
| 63 | m/z=690.24(C49H30N4O, 690.81) | 64 | m/z=690.24(C49H30N4O, 690.81) |
| 65 | m/z=690.24(C49H30N4O, 690.81) | 66 | m/z=690.24(C49H30N4O, 690.81) |
| 67 | m/z=690.24(C49H30N4O, 690.81) | 68 | m/z=690.24(C49H30N4O, 690.81) |
| 69 | m/z=690.24(C49H30N4O, 690.81) | 70 | m/z=740.26 (C53H32N4O, 740.87) |
| 71 | m/z=740.26(C53H32N4O, 740.87) | 72 | m/z=766.27(C55H34N4O, 766.90) |
| 73 | m/z=766.27(C55H34N4O, 766.90) | 74 | m/z=740.26(C53H32N4O, 740.87) |
| 75 | m/z=766.27(C55H34N4O, 766.90) | 76 | m/z=614.21(C43H26N4O, 614.71) |
| 77 | m/z=614.21(C43H26N4O, 614.71) | 78 | m/z=664.23(C47H28N4O, 664.77) |
| 79 | m/z=614.21(C₄₃H₂₆N₄O, 614.71) | 80 | m/z=664.23(C47H28N4O, 664.77) |
| 81 | m/z=664.23 (C₄₇H₂₈N₄O, 664.77) | 82 | m/z=664.23 (C₄₇H₂₈N₄O, 664.77) |
| 83 | m/z=664.23 (C₄₇H₂₈N₄O, 664.77) | 84 | m/z=664.23 (C₄₇H₂₈N₄O, 664.77) |
| 85 | m/z=664.23(C₄₇H₂₈N₄O, 664.77) | 86 | m/z=690.24(C49H30N4O, 690.81) |
| 87 | m/z=690.24(C49H30N4O, 690.81) | 88 | m/z=690.24(C49H30N4O, 690.81) |
| 89 | m/z=690.24(C49H30N4O, 690.81) | 90 | m/z=690.24(C49H30N4O, 690.81) |
| 91 | m/z=690.24(C49H30N4O, 690.81) | 92 | m/z=690.24(C49H30N4O, 690.81) |
| 93 | m/z=690.24(C49H30N4O, 690.81) | 94 | m/z=690.24(C49H30N4O, 690.81) |
| 95 | m/z=740.26(C53H32N4O, 740.87) | 96 | m/z=740.26(C53H32N4O, 740.87) |
| 97 | m/z=766.27(C55H34N4O, 766.90) | 98 | m/z=766.27(C55H34N4O, 766.90) |
| 99 | m/z=740.26(C53H32N4O, 740.87) | 100 | m/z=766.27(C55H34N4O, 766.90) |
| 101 | m/z=664.23(C47H28N4O, 664.77) | 102 | m/z=740.26(C53H32N4O, 740.87) |
| 103 | m/z=704.22(C49H28N4O2, 704.79) | 104 | m/z=720.20(C49H28N4OS, 720.85) |
| 105 | m/z=730.27(C52H34N4O, 730.87) | 106 | m/z=690.24(C49H30N4O, 690.81) |
| 107 | m/z=740.26(C53H32N4O, 740.87) | 108 | m/z=704.22(C49H28N4O2, 704.79) |
| 109 | m/z=720.20(C49H28N4OS, 720.85) | 110 | m/z=730.27(C52H34N4O, 730.87) |
| 111 | m/z=714.24(C51H30N4O, 714.83) | 112 | m/z=740.26(C53H32N4O, 740.87) |
| 113 | m/z=690.24 (C49H30N4O, 690.81) | 114 | m/z=780.25(C55H32N4O2, 780.89) |
| 115 | m/z=780.29(C56H36N4O, 780.93) | 116 | m/z=740.26(C53H32N4O, 740.87) |
| 117 | m/z=754.24(C53H30N4O2, 754.85) | 118 | m/z=780.29(C56H36N4O, 780.93) |
| 119 | m/z=766.27(C55H34N4O, 766.90) | 120 | m/z=806.30(C58H38N4O, 806.97) |
| 121 | m/z=664.23(C47H28N4O, 664.77) | 122 | m/z=740.26(C53H32N4O, 740.87) |
| 123 | m/z=704.22(C49H28N4O2, 704.79) | 124 | m/z=720.20(C49H28N4OS, 720.85) |
| 125 | m/z=730.27(C52H34N4O, 730.87) | 126 | m/z=689.25(C50H31N3O, 689.82) |
| 127 | m/z=740.26(C53H32N4O, 740.87) | 128 | m/z=704.22(C49H28N4O2, 704.79) |
| 129 | m/z=720.20(C49H28N4OS, 720.85) | 130 | m/z=729.28(C53H35N3O, 729.88) |
| 131 | m/z=714.24(C51H30N4O, 714.83) | 132 | m/z=739.26(C54H33N3O, 739.88) |
| 133 | m/z=689.25(C50H31N3O, 689.82) | 134 | m/z=780.25(C55H32N4O2, 780.89) |
| 135 | m/z=780.29(C56H36N4O, 780.93) | 136 | m/z=664.23(C47H28N4O, 664.77) |
| 137 | m/z=740.26(C53H32N4O, 740.87) | 138 | m/z=704.22(C49H28N4O2, 704.79) |
| 139 | m/z=720.20(C49H28N4OS, 719.86) | 140 | m/z=730.27(C52H34N4O, 730.87) |
| 141 | m/z=790.27(C57H34N4O, 790.93) | 142 | m/z=789.28(C58H35N3O, 789.94) |
| 143 | m/z=770.21(C53H30N4OS, 770.91) | 144 | m/z=780.29(C56H36N4O, 780.93) |
| 145 | m/z=740.26(C53H32N4O, 740.87) | 146 | m/z=740.26(C53H32N4O, 740.87)) |
| 147 | m/z=766.27(C55H34N4O, 766.90) | 148 | m/z=740.26(C53H32N4O, 740.87) |
| 149 | m/z=780.25(C55H32N4O, 780.89) | 150 | m/z=740.26(C53H32N4O, 740.87) |
| 151 | m/z=664.23(C47H28N4O, 664.77) | 152 | m/z=740.26(C53H32N4O, 740.87) |
| 153 | m/z=704.22(C49H28N4O2, 704.79) | 154 | m/z=720.20(C49H28N4OS, 720.85) |
| 155 | m/z=730.27(C52H34N4O, 730.87) | 156 | m/z=714.24(C51H30N4O, 714.83) |
| 157 | m/z=740.26(C53H32N4O, 740.87) | 158 | m/z=730.27(C52H34N4O, 730.87) |
| 159 | m/z=780.29(C56H36N4O, 780.93) | 160 | m/z=780.29(C56H36N4O, 780.93) |
| 161 | m/z=740.26(C53H32N4O, 740.87) | 162 | m/z=816.29(C59H36N4O, 816.96) |
| 163 | m/z=780.25(C55H32N4O2, 780.89) | 164 | m/z=796.23(C55H32N4OS, 796.95) |
| 165 | m/z=806.30(C58H38N4O, 806.97) | 166 | m/z=740.26(C53H32N4O, 740.87) |
| 167 | m/z=754.24(C53H30N4O2, 754.85) | 168 | m/z=780.29(C56H36N4O, 780.93) |
| 169 | m/z=766.27(C55H34N4O, 766.90) | 170 | m/z=806.30(C58H38N4O, 806.97) |
| 171 | m/z=664.23 (C47H28N4O, 664.77) | 172 | m/z=730.27(C52H34N4O, 730.87) |
| 173 | m/z=704.22(C49H28N4O2, 704.79) | 174 | m/z=740.26(C53H32N4O, 740.87) |
| 175 | m/z=730.27(C52H34N4O, 730.87) | 176 | m/z=720.20(C49H28N4OS, 720.85) |
| 177 | m/z=740.26(C53H32N4O, 740.87) | 178 | m/z=714.24(C51H30N4O, 714.83) |
| 179 | m/z=730.27(C52H34N4O, 730.87) | 180 | m/z=780.29(C56H36N4O, 780.93) |
| 181 | m/z=740.26(C53H32N4O, 740.87) | 182 | m/z=740.26(C53H32N4O, 740.87) |
| 183 | m/z=780.25(C56H36N4O, 780.89) | 184 | m/z=796.23(C55H32N4OS, 796.95) |
| 185 | m/z=806.30(C58H38N4O, 806.97) | 186 | m/z=664.23(C47H28N4O, 664.77) |
| 187 | m/z=720.20(C49H28N4OS, 720.85) | 188 | m/z=740.26(C53H32N4O, 740.87) |
| 189 | m/z=714.24(C51H30N4O, 714.83) | 190 | m/z=714.24(C51H30N4O, 714.83) |
| 191 | m/z=740.26(C53H32N4O, 740.87) | 192 | m/z=780.29(C56H36N4O, 780.93) |
| 193 | m/z=740.26(C53H32N4O, 740.87) | 194 | m/z=740.26(C53H32N4O, 740.87) |
| 195 | m/z=780.29(C56H36N4O, 780.93) | 196 | m/z=704.22(C49H28N4O2, 704.79) |
| 197 | m/z=664.23(C47H28N4O, 664.77) | 198 | m/z=730.27(C52H34N4O, 730.87) |
| 199 | m/z=790.27(C57H34N4O, 790.93) | 200 | m/z=766.27(C55H34N4O, 766.90) |
| 201 | m/z=690.24(C49H30N4O, 690.81) | 202 | m/z=690.24(C49H30N4O, 690.81) |
| 203 | m/z=740.26(C53H32N4O, 740.87) | 204 | m/z=740.26(C53H32N4O, 740.87) |
| 205 | m/z=740.26(C53H32N4O, 740.87) | 206 | m/z=766.27(C55H34N4O, 766.90) |
| 207 | m/z=766.27(C55H34N4O, 766.90) | 208 | m/z=766.27(C55H34N4O, 766.90) |
| 209 | m/z=740.26(C53H32N4O, 740.87) | 210 | m/z=740.26(C53H32N4O, 740.87) |
| 211 | m/z=690.24(C49H30N4O, 690.81) | 212 | m/z=740.26(C53H32N4O, 740.87) |
| 213 | m/z=740.26(C53H32N4O, 740.87) | 214 | m/z=766.27(C55H34N4O, 766.90) |
| 215 | m/z=690.24(C49H30N4O, 690.81) | 216 | m/z=690.24(C49H30N4O, 690.81) |
| 217 | m/z=740.26(C53H32N4O, 740.87) | 218 | m/z=766.27(C55H34N4O, 766.90) |
| 219 | m/z=690.24(C49H30N4O, 690.81) | 220 | m/z=766.27(C55H34N4O, 766.90) |
| 221 | m/z=780.25(C55H32N4O2, 780.89) | 222 | m/z=740.26(C53H32N4O, 740.87) |
| 223 | m/z=766.27(C55H34N4O, 766.90) | 224 | m/z=766.27(C55H34N4O, 766.90) |
| 225 | m/z=806.30(C58H38N4O, 806.97) | | |
| 301 | m/z=613.22(C44H27N3O, 613.72) | 304 | m/z=613.22(C44H27N3O, 613.72) |
| 307 | m/z=587.20(C42H25N3O, 587.68) | 309 | m/z=693.19(C48H27N3OS, 693.82) |
| 319 | m/z=663.23(C48H29N3O, 663.78) | 323 | m/z=627.19(C44H25N3O2, 627.70) |
| 333 | m/z=637.22(C46H27N3O, 637.74) | 335 | m/z=643.17(C44H25N3OS, 643.76) |
| 340 | m/z=663.23(C48H29N3O, 663.78) | 342 | m/z=663.23(C48H29N3O, 663.78) |
| 346 | m/z=637.22(C46H27N3O, 637.74) | 402 | m/z=790.27(C57H34N4O, 790.93) |
| 411 | m/z=766.27(C55H34N4O, 766.90) | 413 | m/z=780.25(C55H32N4O2, 780.89) |
| 419 | m/z=766.27(C55H34N4O, 766.90) | 430 | m/z=790.27(C57H34N4O, 790.93) |
| 501 | m/z=790.27(C57H34N4O, 790.93) | 504 | m/z=766.27(C55H34N4O, 766.90) |
| 506 | m/z=790.27(C57H34N4O, 790.93) | 509 | m/z=790.27(C57H34N4O, 790.93) |
| 510 | m/z=740.26(C53H32N4O, 740.87) | 516 | m/z=766.27(C55H34N4O, 766.90) |
| 601 | m/z=624.27(C43H16D10N4O, 624.77) | 613 | m/z=624.27(C43H16D10N4O, 624.77) |
| 617 | m/z=624.27(C43H16D10N4O, 624.77) | 621 | m/z=624.27(C43H16D10N4O, 624.77) |

| Com pou nd | FD-Mass | Com pou nd | FD-Mass |
|---|---|---|---|
| 1-1 | m/z=584.23(C44H28N2, 584.72) | 1-2 | m/z=636.26(C48H32N2, 636.80) |
| 1-3 | m/z=610.24(C46H30N2, 610.76) | 1-4 | m/z=610.24(C46H30N2, 610.76) |
| 1-5 | m/z=610.24(C46H30N2, 610.76) | 1-6 | m/z=660.26(C50H32N2, 660.82) |
| 1-7 | m/z=660.26(C50H32N2, 660.82) | 1-8 | m/z=636.26(C48H32N2, 636.80) |
| 1-9 | m/z=699.27(C52H33N3, 699.86) | 1-10 | m/z=610.24(C46H30N2, 610.76) |
| 1-11 | m/z=574.20(C42H26N2O, 574.68) | 1-12 | m/z=574.20(C42H26N2O, 574.68) |
| 1-13 | m/z=558.21(C42H26N2, 558.68) | 1-14 | m/z=565.15(C40H23NOS, 565.69) |
| 1-15 | m/z=636.26(C48H32N2, 636.80) | 1-16 | m/z=502.18(C36H23FN2, 502.59) |
| 1-17 | m/z=540.26(C40H32N2, 540.71) | 1-18 | m/z=560.23(C42H28N2, 560.70) |
| 1-19 | m/z=458.18(C34H22N2, 458.56) | 1-20 | m/z=584.23(C44H28N2, 584.72) |

**[Table 6]**

| Compound | ¹H NMR (CDCl₃, 200 Mz) |
|---|---|
| 1 | δ=8.55(d, 1H), 8.36~8.28(d, 5H), 8.11(d, 1H), 7.96-7.94(d, 2H), 7.75-7.50(m, 12H), 7.40-7.35(m, 4H), 7.15(t, 1H) |
| 2 | δ=8.55(d, 2H), 8.36(d, 4H), 7.99~7.94(m, 4H), 7.64-7.50(m, 13H), 7.39-7.31(m, 3H), 7.16(t, 1H) |
| 3 | δ=8.54(d, 2H), 8.36(d, 4H), 7.99~7.94(m, 5H), 7.65-7.50(m, 15H), 7.39-7.31(m, 2H) |
| 6 | δ=8.36~8.28(m, 6H), 8.11(d, 2H), 7.98(d, 1H), 7.75-7.50(m, 15H), 7.40-7.31(m, 4H) |
| 11 | δ=8.36~8.28(m, 6H), 8.13~8.11(d, 2H), 7.98(d, 1H), 7.89(d, 1H), 7.75-7.31(m, 20H) |
| 14 | δ=8.54(d, 2H), 8.36(d, 4H), 8.30(d, 1H), 8.13(d, 1H), 8.7.99(d, 2H), 7.89(d, 1H), 7.75(d, 2H), 7.61~7.41(m, 18H) |
| 21 | δ=8.55(d, 1H), 8.36(d, 4H), 8.28(d, 1H), 8.31(d, 1H), 7.94(d, 1H),7.90(d, 1H), 7.75~7.69(m, 3H), 7.59~7.50(m, 9H), 7.39~7.30(m, 4H), 7.16(t, 1H) |
| 22 | δ=8.55(d, 1H), 8.54(d, 1H), 8.36(d, 4H), 7.99~7.94(m, 3H), 7.72(d, 1H), 7.65~7.50(m, 12H), 7.39~7.31(m, 2H), 7.16(t, 1H) |
| 34 | δ=8.54(d, 1H), 8.36~8.30(m, 5H), 8.13(d, 1H), 7.99~7.98(m, 2H), 7.89(d, 1H), 7.75~7.72(m, 3H), 7.61~7.31(m, 17H) |
| 37 | δ=8.51(d, 1H), 8.36~8.30(m, 5H), 8.13(d, 1H) 8.12~7.98(m, 3H), 7.89(d, 1H), 7.75-7.67(m, 5H), 7.59-7.31(m, 14H) |
| 41 | δ=8.54(d, 1H), 8.36~8.30(m, 5H), 8.13~7.98(m, 6H), 7.89(d, 1H), 7.63-7.50(m, 16H), 7.39-7.31(m, 3H) |
| 51 | δ=8.55(d, 1H), 8.36(d, 4H), 8.28(d, 1H), 8.11(d, 1H), 7.98~7.94(m, 3H), 7.75~7.69(m,2H), 7.55~7.50(m, 9H), 7.40~7.31(m, 4H), 7.16(t, 1H) |
| 52 | δ=8.55(d, 1H), 8.54(d, 1H), 8.36(d, 4H), 7.99~7.94(m, 4H), 7.65~7.50(m, 12H), 7.39~7.31(m, 3H), 7.16(t, 1H) |
| 53 | δ=8.55(d, 2H), 8.36(d, 4H), 7.99~7.98(m, 4H), 7.65~7.31(m, 20H) |
| 57 | δ=8.54(d, 2H), 8.36(d, 4H), 8.28(d, 1H), 8.11(d, 1H), 7.99~7.96(m, 3H), 7.69~7.50(m, 15H), 7.40~7.31(m, 3H) |
| 62 | δ=8.62(d, 1H), 8.36(d, 4H), 8.28~8.22(d, 2H), 8.11(d, 1H), 7.98~7.96(m, 2H), 7.75~7.69(m, 5H), 7.55~7.31(m, 15H) |
| 65 | δ=8.62(d, 1H), 8.54(d, 1H), 8.36(d, 4H), 8.22(d, 1H), 7.99~7.96(m, 3H), 7.75~7.74(m, 3H), 7.65~7.31(m, 17H) |
| 72 | δ=8.54(d, 1H), 8.36(d, 4H), 8.30(d, 1H), 8.13(d, 1H), 7.99~7.96(m, 3H), 7.89(d, 1H), 7.75(d, 2H), 7.65~7.25(m, 21H) |
| 76 | δ=8.55(d, 2H), 8.36(d, 4H), 8.13(d, 1H), 8.02~7.94(m, 3H), 7.75~7.69(m, 2H), 7.55~7.31(m, 11H), 7.16(t, 1H) |
| 77 | δ=8.55(d, 1H), 8.54(d, 1H), 8.36(d, 4H), 8.02~7.94(m, 4H), 7.65~7.31(m, 15H), 7.16(t, 1H) |
| 79 | δ=8.55(d, 1H), 8.54(d, 1H), 8.36(d, 4H), 8.12~8.11(m, 2H), 8.02~7.94(m, 3H), 7.72~7.67(m, 2H), 7.57~7.31(m, 12H), 7.16(t, 1H) |
| 81 | δ=8.36~8.28(m, 6H), 8.11(d, 2H), 8.02(d, 2H), 7.75~7.69(m, 4H), 7.55~7.31(m, 14H) |
| 88 | δ=8.55(d, 1H), 8.36(d, 4H), 8.02~7.94(m, 4H), 7.75(d, 2H), 7.55~7.31(m, 18H) |
| 95 | δ=8.97(d, 2H), 8.54(d, 1H), 8.36(d, 4H), 8.02~7.94(m, 5H), 7.79(d, 2H), 7.61~7.31(m, 18H) |
| 96 | δ=8.54(d, 1H), 8.36(d, 4H), 8.30(d, 1H), 8.09~7.98(m, 7H), 7.69~7.31(m, 18H) |
| 102 | δ=8.55(d, 1H), 8.36(d, 2H), 8.28(d, 1H), 8.11~7.94(m, 8H), 7.75~7.50(m, 12H), 7.40~7.16(m, 8H) |
| 103 | δ=8.55(d, 1H), 8.36(d, 2H), 8.28(d, 1H), 8.11(d, 1H), 8.03~7.94(m, 4H), 7.82~7.31(m, 18H), 7.16(t, 1H) |
| 105 | δ=8.55(d, 2H), 8.36(d, 2H), 8.28(d, 1H), 8.11~8.09(d, 2H), 7.96~7.89(m, 4H), 7.78~7.50(m, 11H), 7.40-7.28(m, 6H), 7.16(t, 1H) |
| 106 | δ=8.55(d, 1H), 8.36(d, 2H), 8.28(d, 1H), 8.11(d, 1H), 7.96~7.94(m, 4H), 7.75~7.25(m, 20H), 7.16(t, 1H) |
| 108 | δ=8.55(d, 1H), 8.36(d, 2H), 8.28(d, 1H), 8.11~8.08(d, 2H), 7.98~7.94(m, 4H), 7.75~7.31(m, 17H), 7.16(t, 1H) |
| 113 | δ=8.55(d, 1H), 8.38~8.28(m, 4H), 8.11(d, 1H), 7.96~7.94(m, 3H), 7.75~7.35(m, 20H), 7.16(t, 1H) |
| 115 | δ=9.09(d, 1H), 8.55(d, 1H), 8.49(d, 1H), 8.28(d, 1H), 8.16~7.89(m, 9H), 7.75-7.51(m, 10H), 7.40~7.28(m, 6H), 7.16(t, 1H) |
| 119 | δ=8.55(d, 1H), 8.28(d, 1H), 8.11(d, 1H), 7.96~7.94(m, 6H), 7.75-7.25(m, 25H) |
| 120 | δ=8.55(d, 1H), 8.28(d, 1H), 8.11(d, 2H), 7.96~7.90(m, 6H), 7.78-7.28(m, 21H), 7.16(t, 1H) |
| 121 | δ=9.09(d, 1H), 8.55~8.49(m, 3H), 8.36(d, 2H), 8.16~7.94(m, 6H), 7.64~7.50(m, 12H), 7.39~7.31(m, 3H), 7.16(t, 1H) |
| 124 | δ=8.54(d, 2H), 8.45(d, 1H), 8.36(d, 2H), 8.20(d, 2H), 7.99~7.93(m, 5H), 7.65~7.31(m, 15H), 7.16(t, 1H) |
| 125 | δ=8.55(d, 1H), 8.36(d, 2H), 8.09(d, 1H), 7.98~7.89(m, 4H), 7.78(d, 1H), 7.64~7.26(m, 21H), 7.16(t, 2H) |
| 126 | δ=8.55(d, 2H), 8.36(d, 2H), 7.99~7.94(m, 5H), 7.75(d, 2H), 7.61~7.25(m, 18H), 7.16(t, 1H) |
| 135 | δ=9.09(d, 1H), 8.55~8.49(m, 3H), 8.16~7.89(m, 9H), 7.78(d, 1H), 7.65~7.53(m, 10H), 7.39~7.28(m, 5H), 7.16(t, 1H) |
| 140 | δ=8.55~8.51(m, 2H), 8.36(d, 2H), 8.12~8.09(m, 3H), 7.98~7.89(m, 4H), 7.78~7.50(m, 11H), 7.39~7.28(m, 5H), 7.16(t, 1H) |
| 145 | δ=9.08(d, 1H), 8.98(d, 1H), 8.55(d, 1H), 8.36(d, 2H), 8.17~8.11(m, 2H), 7.98~7.94(m, 4H), 7.75~7.16(m, 21H) |
| 147 | δ=8.36~8.30(m, 3H), 8.13(d, 1H), 7.98(d, 1H), 7.89(s, 1H), 7.75(d, 2H), 7.64~7.32(m, 17H), 7.17(d, 2H), 6.80(s, 1H), 6.71~6.65(m, 2H), 6.49(d, 1H) |
| 151 | δ=9.09 (s, 1H), 8.55(d, 1H), 8.49(d, 1H), 8.36(d, 2H), 8.16~7.94(m, 7H), 7.75~7.50(m, 10H), 7.40~7.31(m, 4H), 7.16(t, 1H). |
| 155 | δ=8.55(d, 1H), 8.28(d, 1H), 8.11~8.09(d, 2H), 7.96~7.89(m, 5H), 7.78~7.69(m, 3H), 7.55~7.50(m, 7H), 7.40~7.28(m, 6H), 7.16(t, 1H). |
| 157 | δ=8.95(d, 1H), 8.55(d, 2H), 8.36~8.28(d, 3H), 8.11~8.09(m, 2H), 7.98~7.94(m, 5H), 7.77~7.69(m, 3H), 7.55~7.50(m, 7H), 7.40~7.31(m, 7H), 7.16(t, 1H). |
| 158 | δ=8.55(d, 1H), 8.36(d, 2H), 8.18(s, 1H), 8.11(d, 1H), 7.98~7.90(m, 4H), 7.75~7.68(m, 4H), 7.55~7.50(m, 7H), 7.40~7.28(m, 6H), 7.16(t, 1H). |
| 160 | δ=8.55(d, 1H), 8.28(d, 1H), 8.11~8.08(d, 2H), 7.96~7.88(m, 7H), 1.75~7.69(m, 4H), 7.55~7.25(m, 16H),7.16(t, 1H). |
| 161 | δ=9.09(s, 1H), 8.55(d, 2H),8.28(d, 1H), 8.16~7.94(m, 9H), 7.75~7.16(m, 19H) |
| 162 | δ=8.55(d, 1H), 8.28(d, 1H), 8.11~7.94(m, 11H), 7.75~7.30(m, 22H), 7.16(t, 1H). |
| 163 | δ=8.55(d, 1H), 8.28(d, 1H), 8.11(d, 1H), 8.03~7.94(m, 7H), 7.82~7.69(m, 6H), 7.55~7.25(m, 15H), 7.16(t, 1H). |
| 169 | δ=8.54(d, 2H), 8.01(d, 2H), 7.91(s, 1H), 7.79~7.70(m, 9H), 7.65~7.60(m, 10H), 7.51~7.45(m, 6H), 7.18(d, 1H). |
| 170 | δ=8.55(d, 1H), 8.28(d, 1H), 8.11(d, 1H), 7.98~7.90(m, 7H), 1.78~7.69(m, 5H), 7.55~7.25(m, 15H), 7.16(t, 1H). |
| 172 | δ=8.55(d, 2H), 8.36(d, 2H), 8.06~7.94(m, 9H), 7.63~7.50(m, 12H), 7.39~7.25(m, 6H), 7.16(t, 1H). |
| 175 | δ=8.55 (d, 2H), 8.36(d, 2H), 8.09(d, 1H), 7.99~7.89(m, 6H), 7.78(d, 1H), 7.65~7.50(m, 10H), 7.39~7.28(m, 5H), 7.16(t, 1H). |
| 177 | δ=8.95 (d, 2H), 8.55~8.50(m, 3H), 8.36(d, 2H), 8.20(d, 1H), 8.09(d, 1H), 7.99~7.94(m, 6H), 7.77(t, 1H), 7.65~7.50(m, 10H), 7.39~7.25(m, 6H), 7.16(t, 1H). |
| 179 | δ=8.55 (d, 2H), 8.36(d, 2H), 8.18(s, 1H), 7.99~7.90(m, 5H), 7.74~7.50(m, 12H), 7.39~7.28(m, 5H), 7.16(t, 1H) |
| 187 | δ=8.55 (d, 1H), 8.45(d, 1H), 8.36~8.28(m, 3H), 8.12(d, 2H), 7.99~7.90(m, 5H), 7.75~7.69(m, 3H), 7.56~7.35(m, 12H), 7.16(t, 1H) |
| 194 | δ=8.55 (d, 1H), 8.36~8.28(m, 3H), 8.12~7.94(m, 9H), 7.75~7.16(m, 19H) |
| 198 | δ=8.55(d, 2H), 8.36(d, 2H), 8.12~8.09(m, 3H), 7.98~7.89(m, 4H), 7.77~7.67(m, 4H), 7.59~7.50(m, 7H),7.9~7.28(m, 5H), 7.16(t, 1H) |
| 200 | δ=8.54(d, 1H), 8.36(d, 3H), 8.13(d, 1H), 8.02~7.96(m, 5H),7.89(s, 1H), 7.75(d, 4H), 7.61~7.31(m, 19H) |
| 201 | δ=8.55(d, 1H), 8.36(d, 4H), 8.28(d, 1H), 8.11(d, 1H), 7.96(d, 4H), 7.75~7.50(m, 12H), 7.40~7.25(m, 6H), 7.16(t, 1H) |
| 202 | δ=8.55(d, 2H), 8.38(d, 1H), 7.98~7.94(m, 4H), 7.65~7.50(m, 15H), 7.39~7.31(m, 3H), 7.16(t, 1H) |
| 204 | δ=9.09(s, 1H), 8.55~8.49(m, 3H), 8.36(d, 2H), 8.16~7.94(m, 9H), 7.72~7.50(m, 11H), 7.39~7.16(m, 6H) |
| 206 | δ=8.54(d, 1H), 8.36(d, 4H), 8.30(d, 1H), 8.13(d, 1H), 7.99~7.96(m, 4H), 7.89(s, 1H), 7.75(d, 2H), 7.65~7.31(m, 20H) |
| 208 | δ=8.55(d, 1H), 8.38~8.28(m, 5H),8.31(d, 1H), 7.94(d, 3H), 7.75~7.35(m, 22H), 7.16(t, 1H) |
| 212 | δ=8.36~8.28(m, 6H), 8.11(d, 1H), 7.96(d, 3H), 7.75~7.69(m, 5H), 7.59~7.50(m, 9H), 7.40~7.25(m, 6H) |
| 215 | δ=8.55(d, 1H), 8.36(d, 4H), 8.28(d, 1H), 8.11(d, 1H), 7.98~7.94(m, 5H), 7.75(d, 1H), 7.55~7.50(m, 9H), 7.40~7.25(m, 6H), 7.16(t, 1H) |
| 220 | δ=8.55(d, 2H), 8.36(d, 2H), 7.99~7.94(m, 8H), 7.75(d, 2H), 7.65~7.25(m. 19H), 7.16(t, 1H) |
| 222 | δ=9.09(s, 1H), 8.55(d, 1H), 8.49(d, 1H), 8.36(d, 2H), 8.28(d, 1H), 8.31~7.94(m, 9H), 7.75~7.25(m, 17H) |
| 224 | δ=8.55(d, 1H), 8.36(d, 2H), 8.28(d, 1H), 8.11(d, 1H), 7.96~7.94(m, 6H), 7.75~7.16(m, 23H) |
| 301 | δ=8.55 (1H, dd), 8.28 (1H, dd), 8.24 (1H, s), 7.99-7.92 (5H, m), 7.85 (1H, dd), 7.75 (1H, ddd), 7.66-7.29 (15H, m), 7.17-7.09 (2H, m) |
| 304 | δ=8.54 (1H, dd), 8.37-8.22 (4H, m), 7.99-7.93 (3H, m), 7.85 (1H, dd), 7.74 (1H, ddd), 7.66-7.30 (15H, m), 7.17-7.10 (2H, m) |
| 307 | δ=8.56 (1H, dd), 8.27 (1H, dd), 8.13 (1H, dd), 7.99 (1H, dd), 7.85-7.30 (19H, m), 7.16-7.11 (2H, m) |
| 309 | δ=8.52 (1H, dd), 8.45 (1H, d), 8.29 (1H, dd), 8.07-7.96 (5H, m), 7.88-7.73 (4H, m), 7.66-7.30 (13H, m), 7.18-7.10 (2H, m) |
| 319 | δ=8.98 (1H, dd), 8.57 (1H, dd), 8.37-8.27 (4H, m), 8.01-7.92 (3H, m), 7.85-7.70 (5H, m), 7.58-7.39 (13H, m), 7.18-7.10 (2H, m) |
| 323 | δ=8.53 (1H, dd), 8.27 (1H, dd), 7.99 (1H, dd), 7.85-7.68 (7H, m), 7.55- 7.10 (15H, m) |
| 333 | δ=8.97 (1H, dd), 8.57 (1H, dd), 8.29 (1H, ddd), 8.15 (1H, dd), 8.01-7.93 (3H, m), 7.86-7.71 (7H, m), 7.59-7.30 (11H, m), 7.18-7.10 (2H, m) |
| 335 | δ=8.57 (1H, dd), 8.29 (1H, dd), 7.96 (1H, dd), 7.87-7.70 (7H, m), 7.58-7.30 (13H, m), 7.18-7.11 (2H, m) |
| 340 | δ=8.56 (2H, dd), 8.24 (1H, s), 7.90 (4H, ddd), 7.75 (1H, d), 7.63-7.32 (17H, m), 7.15 (1H, dd) |
| 342 | δ=8.99 (1H, dd), 8.57 (1H, dd), 8.29 (1H, ddd), 8.23 (1H, s), 8.02-7.91 (5H, m), 7.86-7.74 (5H, m), 7.57-7.30 (13H, m), 7.18-7.11 (2H, m) |
| 346 | δ=8.53 (1H, dd), 8.26 (1H, dd), 8.11 (1H, dd), 8.01-7.98 (2H, m), 7.85-7.29 (21H, m), 7.17 (1H, dd) |
| 402 | δ=9.10 (2H, dd), 8.50 (2H, dd), 8.26 (2H, dd), 8.20-7.99 (8H, m), 7.65-7.50 (10H, m), 7.41-7.10 (8H, m) |
| 411 | δ=8.56-8.52 (2H, m), 8.37 (2H, dd), 8.26-8.23 (4H, m), 8.00-7.96 (2H, m), 7.76-7.69 (3H, m), 7.62-7.10 (21H, m) |
| 413 | δ=8.56 (1H, dd), 8.38-8.23 (5H, m), 7.99-7.96 (2H, m), 7.85-7.69 (5H, m), 7.58-7.27 (17H, m), 7.16-7.10 (2H, m) |
| 419 | δ=8.56 (1H, dd), 8.37-8.24 (7H, m), 7.39 (1H, dd), 7.85 (1H, dd), 7.76-7.70 (4H, m), 7.57-7.11 (20H, m) |
| 430 | δ=9.10 (2H, dd), 8.48 (2H, dd), 8.26-7.96 (10H, m), 7.79-7.10 (18H, m) |
| 501 | δ=9.12 (1H, dd), 8.50 (1H, dd), 8.37-8.29 (3H, m), 8.10-7.96 (7H, m), 7.89-7.29 (22H, m) |
| 504 | δ=8.56 (1H, dd), 8.40-8.35 (3H, m), 8.01-7.85 (5H, m), 7.76-7.29 (24H, m) |
| 506 | δ=8.99-8.95 (3H, m), 8.37-8.29 (3H, m), 8.01-7.96 (3H, m), 7.86-7.74 (7H, m), 7.56-7.28 (18H, m) |
| 509 | δ=9.11 (2H, dd), 8.50 (2H, dd), 8.29 (1H, dd), 8.18-7.97 (8H, m), 7.85-7.70 (7H, m), 7.63-7.27 (14H, m) |
| 510 | δ=8.98 (1H, dd), 8.53 (1H, dd), 8.36 (2H, ddd), 8.20 (1H, d), 8.02-7.95 (4H, m), 7.86-7.70 (7H, m), 7.62-7.29 (15H, m), 7.16 (1H, dd) |
| 516 | δ=8.56 (1H, dd), 8.37 (2H, ddd), 8.25 (2H, dd), 8.01-7.97 (2H, m), 7.81-7.71 (6H, m), 7.63-7.15 (21H, m) |
| 601 | 5=8.57 (1H, dd), 8.27 (1H, dd), 7.99 (1H, dd), 7.85-7.73 (2H, m), 7.66-7.31 (9H, m), 7.18-7.14 (2H, m) |
| 613 | δ=8.54 (1H, dd), 8.29 (1H, dd), 7.99 (1H, dd), 7.86-7.69 (3H, m), 7.56-7.29 (8H, m), 7.18-7.10 (2H, m) |
| 617 | δ=8.57 (1H, dd), 8.29 (1H, dd), 7.97 (1H, dd), 7.82-7.71 (3H, m), 7.61-7.30 (8H, m), 7.18-7.14 (2H, m) |
| 621 | δ=8.57 (1H, dd), 8.27 (1H, dd), 7.97 (1H, dd), 7.83-7.74 (3H, m), 7.57-7.30 (8H, m), 7.17-7.11 (2H, m) |

### <Example>

### 1) Manufacture of Organic Light Emitting Device (Red Host)

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4'4"-tris[2-naphthyl(phenyl)amino] triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. The light emitting layer was deposited to 500 Å using a compound described in the following Table 8 as a host and (piq)₂(Ir) (acac) as a red phosphorescent dopant by doping the (piq)₂(Ir) (acac) to the host in a weight ratio of 3%. After that, BCP was deposited to 60 Å as a hole blocking layer, and Alq₃ was deposited to 200 Å thereon as an electron transfer layer.

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

### 2) Driving Voltage and Light Emission Efficiency of Organic Electroluminescent Device

For each of the organic electroluminescent devices manufactured as above, electroluminescent (EL) properties were measured using M7000 manufactured by McScience Inc., and with the measurement results, T90 was measured when standard luminance was 6,000 cd/m² through a lifetime measurement system (M6000) manufactured by McScience Inc.. HOMO, LUMO and band gap of the organic compounds of the present disclosure are as shown in the following Table 7.

**[Table 7]**

| Compound | HOMO (eV) | LUMO (eV) | Band Gap | T1 (eV) |
|---|---|---|---|---|
| Comparative Compound A | -5.33 | -2.07 | 3.26 | 2.50 |
| Comparative Compound B | -5.38 | -2.05 | 3.33 | 2.55 |
| Comparative Compound C | -5.27 | -1.91 | 3.36 | 2.69 |
| Comparative Compound D | -5.79 | -1.94 | 3.85 | 2.68 |
| Comparative Compound E | -5.03 | -1.76 | 3.27 | 2.58 |
| Comparative Compound F | -5.40 | -1.92 | 3.48 | 2.71 |
| Compound 1 of Present Application | -5.05 | -2.08 | 2.97 | 2.29 |
| Compound 51 of Present Application | -5.07 | -2.06 | 3.01 | 2.30 |
| Compound 201 of Present Application | -5.05 | -2.02 | 3.03 | 2.30 |

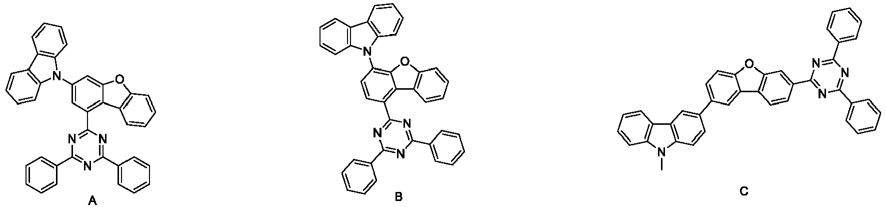

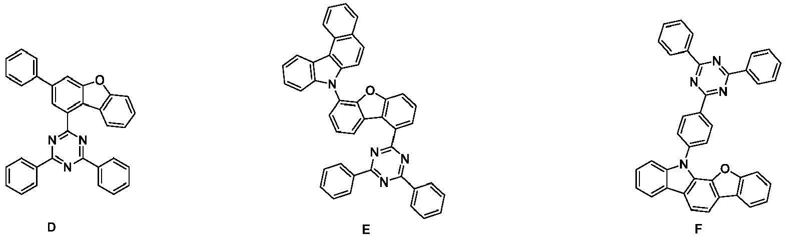

HOMO, LUMO and band gap of the comparative compounds and the compounds of the present application may be identified in Table 7. From the results, it was seen that the heterocyclic compound of Chemical Formula 1 according to the present application has an increased conjugation effect with the benzene ring of the carbazole being extended (fused) leading to a reduced band gap and a smaller T1 level compared to Comparative Compounds A to F, which was suitable as a red host of an organic light emitting device.

The following Table 8 shows examples of using a single host material, and Table 9 shows examples of employing the compound (acceptor (n-host)) corresponding to Chemical Formula 1 of the present application having a favorable electron transfer ability as a first host and the compound (donor (p-host)) corresponding to Chemical Formula 2 of the present application having a favorable hole transfer ability as a second host, and depositing the two host compounds as one source of supply.

**[Table 8]**

| | Compou nd | Driving Voltage (V) | Effici ency (cd/A) | Color Coordinate (x, y) | Lifetime (T₉₀) |
|---|---|---|---|---|---|
| Comparative Example 1 | A | 4.67 | 7.9 | (0.667, 0.358) | 50 |
| Comparative Example 2 | B | 4.77 | 7.1 | (0.664, 0.359) | 45 |
| Comparative Example 3 | C | 4.87 | 6.7 | (0.665, 0.359) | 21 |
| Comparative Example 4 | D | 4.69 | 5.5 | (0.666, 0.371) | 62 |
| Comparative Example 5 | E | 4.66 | 5.2 | (0.667, 0.352) | 55 |
| Comparative Example 6 | F | 4.24 | 7.5 | (0.668, 0.351) | 13 |
| Example 1 | 1 | 3.78 | 25.4 | (0.668, 0.352) | 140 |
| Example 2 | 2 | 3.89 | 21.7 | (0.669, 0.351) | 118 |
| Example 3 | 3 | 3.92 | 24.7 | (0.667, 0.349) | 115 |
| Example 4 | 6 | 3.88 | 22.5 | (0.667, 0.352) | 110 |
| Example 5 | 11 | 4.02 | 21.2 | (0.668, 0.351) | 121 |
| Example 6 | 14 | 4.05 | 22.8 | (0.668, 0.351) | 107 |
| Example 7 | 21 | 3.83 | 24.8 | (0.669, 0.353) | 111 |
| Example 8 | 22 | 3.97 | 22.5 | (0.669, 0.351) | 119 |
| Example 9 | 34 | 4.06 | 21.5 | (0.668, 0.351) | 115 |
| Example 10 | 37 | 4.10 | 20.9 | (0.668, 0.352) | 108 |
| Example 11 | 41 | 4.13 | 20.1 | (0.668, 0.352) | 124 |
| Example 12 | 51 | 3.79 | 19.5 | (0.669, 0.351) | 100 |
| Example 13 | 52 | 3.81 | 18.5 | (0.668, 0.351) | 95 |
| Example 14 | 53 | 4.00 | 20.5 | (0.669, 0.352) | 85 |
| Example 15 | 57 | 4.03 | 19.8 | (0.667, 0.351) | 88 |
| Example 16 | 62 | 3.99 | 18.5 | (0.668, 0.351) | 101 |
| Example 17 | 65 | 4.05 | 18.9 | (0.666, 0.350) | 87 |
| Example 18 | 72 | 4.10 | 19.9 | (0.667, 0.351) | 88 |
| Example 19 | 76 | 4.02 | 20.5 | (0.668, 0.350) | 87 |
| Example 20 | 77 | 4.09 | 19.9 | (0.669, 0.352) | 95 |
| Example 21 | 79 | 4.10 | 19.5 | (0.667, 0.351) | 79 |
| Example 22 | 81 | 4.02 | 18.1 | (0.668, 0.352) | 84 |
| Example 23 | 88 | 4.12 | 20.5 | (0.669, 0.351) | 79 |
| Example 24 | 95 | 4.00 | 19.4 | (0.668, 0.351) | 97 |
| Example 25 | 96 | 4.19 | 20.5 | (0.667, 0.352) | 88 |
| Example 26 | 102 | 3.80 | 23.5 | (0.667, 0.350) | 135 |
| Example 27 | 103 | 3.81 | 22.6 | (0.668, 0.351) | 121 |
| Example 28 | 105 | 3.98 | 23.1 | (0.669, 0.352) | 138 |
| Example 29 | 106 | 3.94 | 24.5 | (0.668, 0.352) | 135 |
| Example 30 | 108 | 3.87 | 23.5 | (0.669, 0.351) | 120 |
| Example 31 | 113 | 3.98 | 22.4 | (0.668, 0.351) | 117 |
| Example 32 | 115 | 4.09 | 23.9 | (0.667, 0.350) | 119 |
| Example 33 | 119 | 3.95 | 24.6 | (0.667, 0.349) | 133 |
| Example 34 | 120 | 4.05 | 25.5 | (0.669, 0.351) | 128 |
| Example 35 | 121 | 3.99 | 23.5 | (0.668, 0.350) | 128 |
| Example 36 | 124 | 3.84 | 22.1 | (0.667, 0.351) | 111 |
| Example 37 | 125 | 4.08 | 23.2 | (0.668, 0.350) | 120 |
| Example 38 | 126 | 3.89 | 24.5 | (0.669, 0.352) | 136 |
| Example 39 | 135 | 4.15 | 21.5 | (0.668, 0.351) | 110 |
| Example 40 | 140 | 4.08 | 22.6 | (0.667, 0.352) | 104 |
| Example 41 | 145 | 4.22 | 20.0 | (0.667, 0.351) | 112 |
| Example 42 | 147 | 4.02 | 22.5 | (0.669, 0.353) | 129 |
| Example 43 | 151 | 3.91 | 21.5 | (0.668, 0.351) | 85 |
| Example 44 | 155 | 3.89 | 20.6 | (0.668, 0.353) | 98 |
| Example 45 | 157 | 4.02 | 17.8 | (0.667, 0.351) | 79 |
| Example 46 | 158 | 3.88 | 21.9 | (0.669, 0.352) | 93 |
| Example 47 | 160 | 4.02 | 18.2 | (0.667, 0.351) | 81 |
| Example 48 | 161 | 4.05 | 19.8 | (0.668, 0.352) | 84 |
| Example 49 | 162 | 4.00 | 17.5 | (0.669, 0.351) | 95 |
| Example 50 | 163 | 4.08 | 18.4 | (0.667, 0.350) | 99 |
| Example 51 | 169 | 4.01 | 19.1 | (0.668, 0.352) | 100 |
| Example 52 | 170 | 3.99 | 20.7 | (0.669, 0.350) | 91 |
| Example 53 | 172 | 3.90 | 21.4 | (0.667, 0.351) | 85 |
| Example 54 | 175 | 3.98 | 20.7 | (0.668, 0.353) | 74 |
| Example 55 | 177 | 4.00 | 18.0 | (0.669, 0.353) | 86 |
| Example 56 | 179 | 4.02 | 19.5 | (0.667, 0.351) | 79 |
| Example 57 | 187 | 4.08 | 17.4 | (0.668, 0.351) | 81 |
| Example 58 | 194 | 3.84 | 21.1 | (0.667, 0.352) | 96 |
| Example 59 | 198 | 4.07 | 17.4 | (0.668, 0.351) | 84 |
| Example 60 | 200 | 4.09 | 19.1 | (0.667, 0.351) | 95 |
| Example 61 | 201 | 4.02 | 18.0 | (0.668, 0.351) | 115 |
| Example 62 | 202 | 4.08 | 18.6 | (0.669, 0.350) | 119 |
| Example 63 | 204 | 4.21 | 17.1 | (0.668, 0.350) | 105 |
| Example 64 | 206 | 3.99 | 18.0 | (0.669, 0.350) | 95 |
| Example 65 | 208 | 3.88 | 19.7 | (0.668, 0.350) | 108 |
| Example 66 | 212 | 4.10 | 18.9 | (0.669, 0.352) | 89 |
| Example 67 | 215 | 3.88 | 20.6 | (0.667, 0.349) | 82 |
| Example 68 | 220 | 4.01 | 20.1 | (0.668, 0.351) | 85 |
| Example 69 | 222 | 3.99 | 19.7 | (0.667, 0.351) | 88 |
| Example 70 | 224 | 3.83 | 20.4 | (0.668, 0.351) | 79 |

As seen from Table 8, it was identified that, when comprising the compound of Chemical Formula 1 in the organic material layer of the organic light emitting device, driving voltage, efficiency and lifetime were significantly improved. This is due to the fact that electrons and holes are efficiently transferred due to bipolar properties having a donor with a favorable hole transfer ability and an acceptor with a favorable electron transfer ability in one molecule, which helps with enhancement in the light emission properties. In addition, it was identified that, by extending the benzene ring with the donor with a favorable hole transfer ability, a phenomenon of trap occurring at an adjoining interface was minimized by adjusting the HOMO level, and the lifetime was improved by effectively forming a light emitting zone in the light emitting layer.

**[Table 9]**

| | First Host | Second Host | Driving Voltage (V) | Efficie ncy (cd/A) | Color Coordinate (x, y) | Lifet ime (T₉₀) |
|---|---|---|---|---|---|---|
| Comparative Example 7 | A | 1-1 | 4.13 | 8.5 | (0.667, 0.358) | 70 |
| Comparative Example 8 | B | 1-7 | 4.04 | 8.1 | (0.664, 0.359) | 80 |
| Comparative Example 9 | C | 1-11 | 4.45 | 7.3 | (0.665, 0.359) | 64 |
| Comparative Example 10 | D | 1-18 | 4.31 | 7.1 | (0.666, 0.371) | 87 |
| Comparative Example 11 | E | 1-2 | 4.25 | 6.9 | (0.667, 0.352) | 76 |
| Comparative Example 12 | F | 1-14 | 4.35 | 8.1 | (0.668, 0.351) | 50 |
| Example 71 | 1 | 1-5 | 3.70 | 27.6 | (0.668, 0.352) | 200 |
| Example 72 | 2 | 1-12 | 3.80 | 24.8 | (0.669, 0.351) | 180 |
| Example 73 | 3 | 1-7 | 3.81 | 25.9 | (0.667, 0.349) | 145 |
| Example 74 | 14 | 1-18 | 3.98 | 24.8 | (0.668, 0.351) | 155 |
| Example 75 | 21 | 1-8 | 3.74 | 26.8 | (0.669, 0.353) | 120 |
| Example 76 | 22 | 1-3 | 3.80 | 24.6 | (0.669, 0.351) | 130 |
| Example 77 | 41 | 1-1 | 4.07 | 23.2 | (0.668, 0.352) | 140 |
| Example 78 | 51 | 1-7 | 3.70 | 21.8 | (0.669, 0.351) | 154 |
| Example 79 | 53 | 1-11 | 3.94 | 21.9 | (0.669, 0.352) | 111 |
| Example 80 | 76 | 1-5 | 3.89 | 22.9 | (0.668, 0.350) | 102 |
| Example 81 | 102 | 1-15 | 3.66 | 25.7 | (0.667, 0.350) | 150 |
| Example 82 | 105 | 1-9 | 3.82 | 25.8 | (0.669, 0.352) | 148 |
| Example 83 | 106 | 1-4 | 3.81 | 26.0 | (0.668, 0.352) | 150 |
| Example 84 | 119 | 1-2 | 3.83 | 26.9 | (0.667, 0.349) | 160 |
| Example 85 | 120 | 1-6 | 3.94 | 26.0 | (0.669, 0.351) | 156 |
| Example 86 | 126 | 1-9 | 3.62 | 25.5 | (0.669, 0.352) | 148 |
| Example 87 | 145 | 1-14 | 4.09 | 23.1 | (0.667, 0.351) | 120 |
| Example 88 | 151 | 1-18 | 3.88 | 23.7 | (0.668, 0.351) | 99 |
| Example 89 | 155 | 1-16 | 3.84 | 22.9 | (0.668, 0.353) | 107 |
| Example 90 | 158 | 1-12 | 3.82 | 21.9 | (0.669, 0.352) | 106 |
| Example 91 | 169 | 1-13 | 3.94 | 22.1 | (0.668, 0.352) | 111 |
| Example 92 | 194 | 1-5 | 3.82 | 21.2 | (0.667, 0.352) | 103 |
| Example 93 | 198 | 1-9 | 4.01 | 20.4 | (0.668, 0.351) | 93 |
| Example 94 | 201 | 1-13 | 4.00 | 22.2 | (0.668, 0.351) | 138 |
| Example 95 | 215 | 1-2 | 3.85 | 21.7 | (0.667, 0.349) | 97 |
| Example 96 | 220 | 1-7 | 3.96 | 21.2 | (0.668, 0.351) | 94 |
| Example 97 | 222 | 1-6 | 3.94 | 22.1 | (0.667, 0.351) | 96 |
| Example 98 | 224 | 1-5 | 3.79 | 22.9 | (0.668, 0.351) | 89 |

From Table 9, it was identified that driving voltage, efficiency and lifetime were improved when comprising the heterocyclic compound of Chemical Formula 1 and the heterocyclic compound of Chemical Formula 2 at the same time in the organic material layer of the organic light emitting device. Such results may lead to a forecast that an exciplex phenomenon occurs when comprising the two compounds at the same time.

The exciplex phenomenon refers to forming a bicomplex in an excited state due to electron exchanges between a molecule having strong donor properties and a molecule having strong acceptor properties.

FIG. 4 is a diagram explaining the exciplex phenomenon. When the exciplex phenomenon occurs as in FIG. 4, new S₁ energy level and T₁ energy level are formed, and red shifted changes in PL may be identified compared to in each of the molecules.

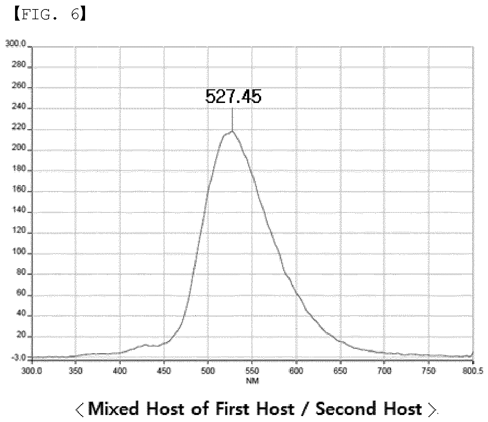

Specifically, FIG. 5 shows data measuring photoluminescence (PL) of each of the first host and the second host according to Example 71 of the present application, and FIG. 6 shows data measuring photoluminescence (PL) when comprising both the first host and the second host according to Example 71 of the present application.

As identified in FIG. 6 and FIG. 7, a red shift was identified when using the compound corresponding to Chemical Formula 1 of the present application (first host) and the compound corresponding to Chemical Formula 2 of the present application (second host) at the same time compared to when using each of the hosts alone.

When the exciplex phenomenon occurs between two molecules as above, reverse intersystem crossing (RISC) occurs, and as a result, internal quantum efficiency may increase up to 100%. As a result, it was identified that the mixed host has an advantage of increasing internal quantum efficiency through forming an exciplex compared to the single host.

Particularly, the compound of Chemical Formula 1 is a bipolar compound and does not have a strong acceptor ability, however, by introducing a donor (p-host) that is the heterocyclic compound of Chemical Formula 2 having a favorable hole transfer ability, exciplex may be formed based on the observation of red shifted changes in the PL, which resultantly helps with enhancement in light emission properties. In addition, it was identified that, by introducing the compound (donor(p-host)) corresponding to Chemical Formula 2 of the present application having a favorable hole transfer ability, the lifetime was significantly improved due to a proper movement of a light emitting zone in the light emitting layer.

### <Experimental Example 1-1>-Manufacture of Organic Light Emitting Device

### 1) Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4, 4 ', 4 "-tris[2-naphthyl(phenyl)amino] triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. Specifically, each of the compounds of Examples 1 to 26 of the following Table 10 was used as a red host of the light emitting layer, and (piq)₂(Ir) (acac), a red phosphorescent dopant, was doped to the red host by 3 wt% to deposit the light emitting layer having a thickness of 500 Å. After that, bathocuproine (hereinafter, BCP) was deposited to 60 Å as a hole blocking layer, and Alq₃ was deposited to 200 Å thereon as an electron transfer layer.

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic light emitting device was manufactured.

Meanwhile, all the organic compounds required to manufacture the OLED were vacuum sublimation purified under 10⁻⁸ torr to 10⁻⁶ torr for each material to be used in the OLED manufacture.

### 2) Driving Voltage and Light Emission Efficiency of Organic Light Emitting Device

For each of the organic light emitting devices of Examples 1 to 26 manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc.. T₉₀ means a lifetime (unit: h, hour), a time taken to become 90% with respect to initial luminance.

Properties of the organic light emitting devices measured are as shown in the following Table 10.

**[Table 10]**

| | Compo und | Threshold Voltage | Driving Voltage (Vₒₚ) | Efficien cy (cd/A) | Color Coordinate (x, y) | Lifet ime (T₉₀) |
|---|---|---|---|---|---|---|
| Example 1 | 301 | 2.54 | 3.43 | 48.2 | (0.680, 0.320) | 190 |
| Example 2 | 304 | 2.68 | 3.48 | 47.1 | (0.676, 0.323) | 185 |
| Example 3 | 307 | 2.49 | 3.47 | 48.7 | (0.681, 0.319) | 197 |
| Example 4 | 309 | 2.51 | 3.57 | 49.7 | (0.678, 0.321) | 192 |
| Example 5 | 319 | 2.66 | 3.61 | 43.2 | (0.676, 0.323) | 175 |
| Example 6 | 323 | 2.50 | 3.55 | 44.7 | (0.683, 0.316) | 178 |
| Example 7 | 333 | 2.49 | 3.57 | 43.6 | (0.680, 0.319) | 168 |
| Example 8 | 335 | 2.53 | 3.51 | 44.7 | (0.680, 0.320) | 171 |
| Example 9 | 340 | 2.47 | 3.62 | 43.2 | (0.678, 0.321) | 181 |
| Example 10 | 342 | 2.44 | 3.57 | 44.1 | (0.680, 0.319) | 175 |
| Example 11 | 346 | 2.53 | 3.55 | 41.1 | (0.679, 0.320) | 170 |
| Example 12 | 402 | 2.38 | 3.77 | 38.2 | (0.682, 0.318) | 157 |
| Example 13 | 411 | 2.35 | 3.73 | 32.5 | (0.681, 0.319) | 146 |
| Example 14 | 413 | 2.39 | 3.77 | 33.5 | (0.680, 0.319) | 141 |
| Example 15 | 419 | 2.33 | 3.72 | 32.7 | (0.677, 0.322) | 139 |
| Example 16 | 430 | 2.35 | 3.69 | 33.1 | (0.679, 0.321) | 135 |
| Example 17 | 501 | 2.36 | 3.90 | 28.9 | (0.680, 0.320) | 112 |
| Example 18 | 504 | 2.35 | 3.95 | 27.7 | (0.681, 0.318) | 108 |
| Example 19 | 506 | 2.33 | 3.91 | 24.3 | (0.680 0.320) | 118 |
| Example 20 | 509 | 2.29 | 3.95 | 23.2 | (0.678, 0.321) | 116 |
| Example 21 | 510 | 2.35 | 3.89 | 23.6 | (0.682, 0.317) | 121 |
| Example 22 | 516 | 2.36 | 3.94 | 22.7 | (0.676, 0.323) | 109 |
| Example 23 | 601 | 2.31 | 3.42 | 51.7 | (0.679, 0.320) | 210 |
| Example 24 | 613 | 2.33 | 3.39 | 54.2 | (0.680, 0.319) | 207 |
| Example 25 | 617 | 2.38 | 3.41 | 53.6 | (0.683, 0.316) | 212 |
| Example 26 | 621 | 2.28 | 3.38 | 52.7 | (0.681, 0.318) | 206 |

As seen from Table 10, it was identified that driving voltage, efficiency and lifetime were improved when comprising the heterocyclic compound of Chemical Formula 1 in the organic material layer of the organic light emitting device. Weakening an acceptor that involves in an electron transfer ability as in Examples 1 to 11 may help with forming a proper threshold voltage by adjusting the HOMO and LUMO levels. In addition, it was identified that efficiency and lifetime were further enhanced by effectively matching a charge balance in the device through adjusting acceptor propensity of the bipolar host as in Examples 1 to 11.

Increasing conjugation in the molecule as in Examples 12 to 22 of Table 10 affects electron transfer ability and hole transfer ability. In addition, it was identified that a phenomenon of trap occurring at an adjoining interface was minimized by adjusting the HOMO and LUMO levels, and efficiency and lifetime were improved by effectively forming a light emitting zone in the light emitting layer.

It was identified that, when substituting hydrogen in the molecule with deuterium as in Examples 23 to 26 of Table 10, kinetic energy of the hydrogen atom decreased as the molecular weight of the hydrogen atom increased, and efficiency and lifetime were improved by increasing molecular stability.

### <Experimental Example 2-1>-Manufacture of Organic Light Emitting Device

### 1) Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4, 4', 4 "-tris[2-naphthyl(phenyl)amino] triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. Specifically, each of the compounds of Examples 1 to 26 of the following Table 11 was used as a red host of the light emitting layer, and (piq)₂(Ir) (acac), a red phosphorescent dopant, was doped to the red host by 3 wt% to deposit the light emitting layer having a thickness of 500 Å. After that, bathocuproine (hereinafter, BCP) was deposited to 60 Å as a hole blocking layer, and Alq₃ was deposited to 200 Å thereon as an electron transfer layer.

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic light emitting device was manufactured.

Meanwhile, all the organic compounds required to manufacture the OLED were vacuum sublimation purified under 10⁻⁸ torr to 10⁻⁶ torr for each material to be used in the OLED manufacture.

### 2) Driving Voltage and Light Emission Efficiency of Organic Light Emitting Device

For each of the organic light emitting devices of Examples 1 to 26 of Table 11 manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc.. T₉₀ means a lifetime (unit: h, hour), a time taken to become 90% with respect to initial luminance.

Properties of the organic light emitting devices measured are as shown in the following Table 11.

**[Table 11]**

| | Firs t Host | Seco nd Host | Thresho ld Voltage (V) | Driving Voltage (V) | Effic iency (cd/A ) | Color Coordinate (x, y) | Life time (T₉₀) |
|---|---|---|---|---|---|---|---|
| Example 1 | 301 | 1-7 | 2.47 | 3.42 | 58.7 | (0.679, 0.320) | 257 |
| Example 2 | 304 | 1-18 | 2.63 | 3.47 | 57.6 | (0.680, 0.320) | 254 |
| Example 3 | 307 | 1-20 | 2.45 | 3.52 | 59.2 | (0.678, 0.322) | 248 |
| Example 4 | 309 | 1-27 | 2.44 | 3.48 | 56.9 | (0.681, 0.319) | 256 |
| Example 5 | 319 | 1-11 | 2.59 | 3.46 | 53.2 | (0.678, 0.322) | 232 |
| Example 6 | 323 | 1-4 | 2.53 | 3.51 | 51.7 | (0.680, 0.320) | 228 |
| Example 7 | 333 | 1-30 | 2.50 | 3.45 | 52.6 | (0.681, 0.319) | 231 |
| Example 8 | 335 | 1-21 | 2.55 | 3.46 | 54.1 | (0.677, 0.323) | 225 |
| Example 9 | 340 | 1-15 | 2.44 | 3.52 | 50.5 | (0.682, 0.318) | 236 |
| Example 10 | 342 | 1-11 | 2.43 | 3.48 | 51.6 | (0.683, 0.317) | 233 |
| Example 11 | 346 | 1-7 | 2.50 | 3.50 | 51.2 | (0.676, 0.323) | 227 |
| Example 12 | 402 | 1-20 | 2.37 | 3.62 | 47.6 | (0.680, 0.319) | 216 |
| Example 13 | 411 | 1-14 | 2.33 | 3.67 | 43.2 | (0.679, 0.320) | 205 |
| Example 14 | 413 | 1-8 | 2.36 | 3.61 | 41.7 | (0.682, 0.317) | 208 |
| Example 15 | 419 | 1-3 | 2.32 | 3.62 | 42.1 | (0.677, 0.323) | 201 |
| Example 16 | 430 | 1-7 | 2.32 | 3.63 | 40.9 | (0.681, 0.319) | 209 |
| Example 17 | 501 | 1-19 | 2.34 | 3.74 | 37.2 | (0.683, 0.317) | 195 |
| Example 18 | 504 | 1-29 | 2.33 | 3.82 | 36.9 | (0.681, 0.319) | 182 |
| Example 19 | 506 | 1-16 | 2.36 | 3.76 | 30.7 | (0.676, 0.323) | 178 |
| Example 20 | 509 | 1-6 | 2.25 | 3.75 | 33.5 | (0.678, 0.321) | 168 |
| Example 21 | 510 | 1-5 | 2.37 | 3.71 | 31.7 | (0.680, 0.319) | 172 |
| Example 22 | 516 | 1-23 | 2.33 | 3.81 | 32.1 | (0.677, 0.322) | 165 |
| Example 23 | 601 | 1-24 | 2.26 | 3.35 | 68.5 | (0.679, 0.320) | 279 |
| Example 24 | 613 | 1-28 | 2.35 | 3.38 | 61.7 | (0.681, 0.319) | 268 |
| Example 25 | 617 | 1-12 | 2.35 | 3.41 | 62.2 | (0.680, 0.320) | 263 |
| Example 26 | 621 | 1-9 | 2.31 | 3.39 | 63.8 | (0.677, 0.323) | 267 |

Table 11 shows cases of comprising the heterocyclic compound of Chemical Formula 1 and the heterocyclic compound of Chemical Formula 2 at the same time in the organic material layer of the organic light emitting device, and as described above, an exciplex phenomenon may be expected to occur by comprising the two compounds at the same time.

In addition, the heterocyclic compound of Chemical Formula 2 serves as an electron blocking layer (EBL) based on a high LUMO level, which creates an effective light emitting area by helping excited electrons to stay in the light emitting layer area. As a result, significant improvements in efficiency and lifetime were identified.

## Claims

1. A heterocyclic compound represented by the following Chemical Formula 1: 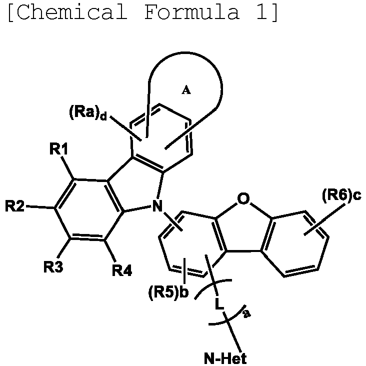 wherein, in Chemical Formula 1,
N-Het is a C2 to C60 monocyclic or polycyclic heterocyclic group substituted or unsubstituted, and comprising one or more Ns;
L is a direct bond; a substituted or unsubstituted C6 to C60 arylene group; or a substituted or unsubstituted C2 to C60 heteroarylene group, a is an integer of 1 to 3, and when a is 2 or greater, Ls are the same as or different from each other;
A is a substituted or unsubstituted C6 to C60 aryl ring; or a substituted or unsubstituted C2 to C60 heteroaryl ring;
Ra is selected from the group consisting of hydrogen; deuterium; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, d is an integer of 0 to 2, and when d is 2, the two Ras are the same as or different from each other; and
R1 to R6 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted phosphine oxide group; a substituted or unsubstituted silyl group; and a substituted or unsubstituted amine group, or two or more groups adjacent to each other bond to each other to form a substituted or unsubstituted C6 to C60 aromatic hydrocarbon ring or a substituted or unsubstituted C2 to C60 heteroring, b is an integer of 0 to 2, c is an integer of 0 to 4, and when b is 2, R5s are the same as or different from each other, and when c is 2 or greater, R6s are the same as or different from each other.

2. The heterocyclic compound of Claim 1, wherein Chemical Formula 1 is represented by one of the following Chemical Formulae 3 to 6: 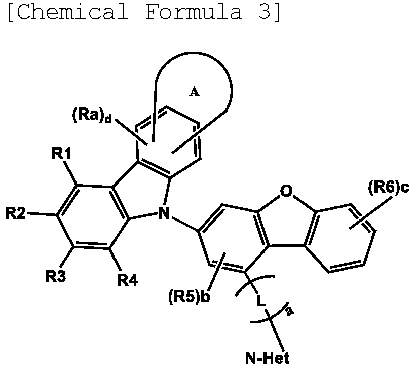 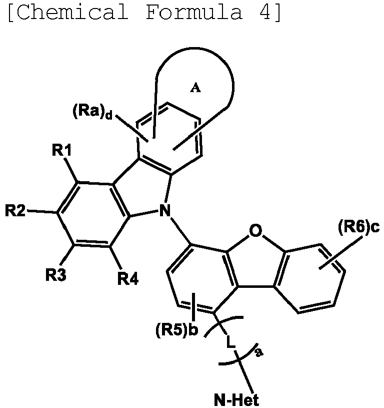 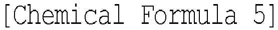 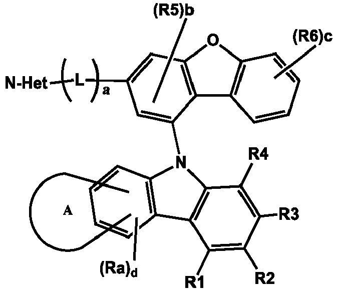 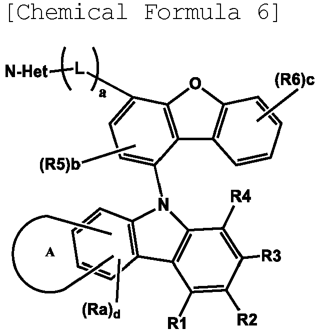 in Chemical Formulae 3 to 6,
N-Het, L, A, Ra, R1 to R6, a, b, c and d have the same definitions as in Chemical Formula 1.

3. The heterocyclic compound of Claim 1, wherein 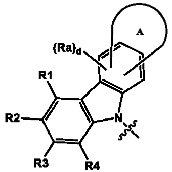 is represented by any one of the following Chemical Formulae 1-1 to 1-6: 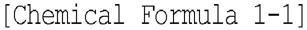 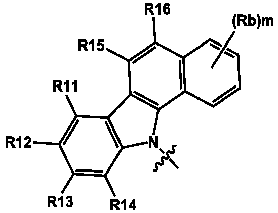 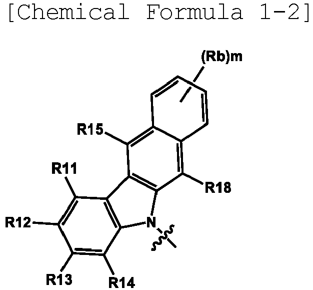 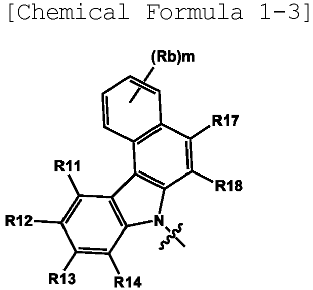 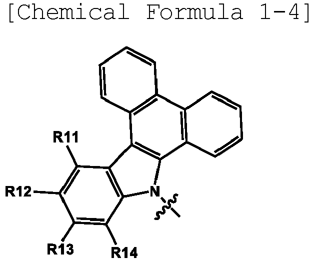 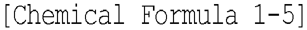 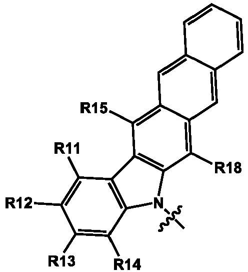 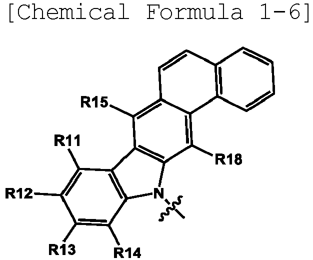 in Chemical Formulae 1-1 to 1-6,
R11 to R14 are the same as or different from each other, and each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring;
R15 to R18 are the same as or different from each other, and each independently hydrogen; deuterium; a substituted or unsubstituted C1 to C60 alkyl group; a substituted or unsubstituted C6 to C60 aryl group; or a substituted or unsubstituted C2 to C60 heteroaryl group; and
Rb is hydrogen; deuterium; or a substituted or unsubstituted C6 to C60 aryl group, m is an integer of 0 to 4, and when m is 2 or greater, Rbs are the same as or different from each other.

4. The heterocyclic compound of Claim 3, wherein, when R12 and R13 of Chemical Formula 1-1 and Chemical Formula 1-3 bond to each other to form an unsubstituted C6 to C60 aromatic hydrocarbon ring or an unsubstituted C2 to C60 heteroring, at least one of R11, R14, R15 to R18 and Rb of Chemical Formula 1-1 and Chemical Formula 1-3 is selected from the group consisting of deuterium; a substituted or unsubstituted C1 to C60 alkyl group; a substituted or unsubstituted C6 to C60 aryl group; and a substituted or unsubstituted C2 to C60 heteroaryl group.

5. The heterocyclic compound of Claim 3, wherein, when R11 to R15, R18 and Rb of Chemical Formula 1-2 are all hydrogen or adjacent two groups among R11 to R14 of Chemical Formula 1-2 bond to each other to form an unsubstituted C6 to C60 aromatic hydrocarbon ring or an unsubstituted C2 to C60 heteroring, N-Het of Chemical Formula 1 is a C2 to C60 monocyclic or polycyclic heteroring substituted or unsubstituted and comprising one or more and two or less Ns, or a deuterium content in Chemical Formula 1 is greater than or equal to 10% and less than or equal to 100%, or L of Chemical Formula 1 is a substituted or unsubstituted C6 to C60 arylene group; or a substituted or unsubstituted C2 to C60 heteroarylene group.

6. The heterocyclic compound of Claim 1, wherein N-Het is represented by the following Chemical Formula 2-1: 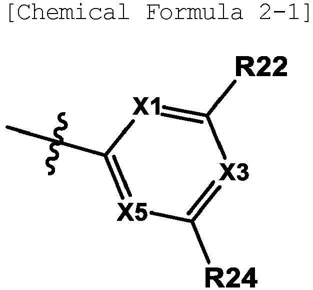 in Chemical Formula 2-1,
X1 is N or CR21, X3 is N or CR23, and X5 is N or CR25;
at least one of X1, X3 and X5 is N; and
R21 to R25 are the same as or different from each other, and each independently hydrogen; deuterium; a substituted or unsubstituted C1 to C60 alkyl group; a substituted or unsubstituted C6 to C60 aryl group; or a substituted or unsubstituted C2 to C60 heteroaryl group.

7. The heterocyclic compound of Claim 6, wherein Chemical Formula 2-1 is any one of the following structural formulae: 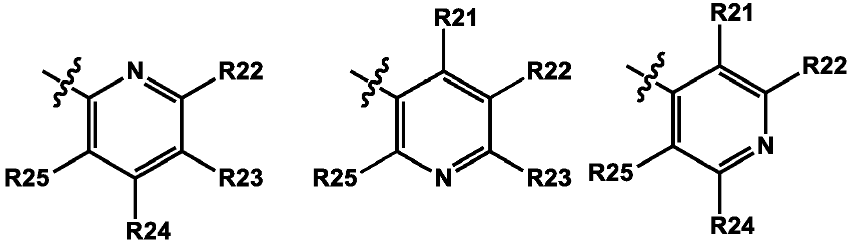 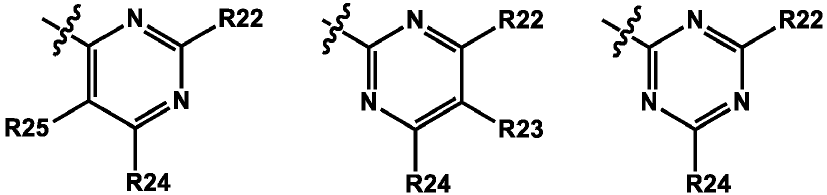 in the structural formulae,
R21 to R25 have the same definitions as in Chemical Formula 2-1.

8. The heterocyclic compound of Claim 1, wherein Chemical Formula 1 is represented by any one of the following compounds:

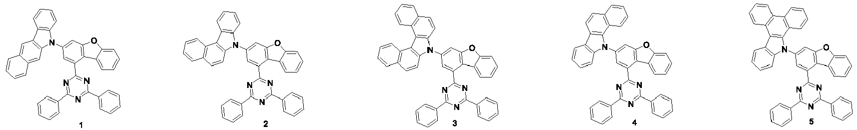

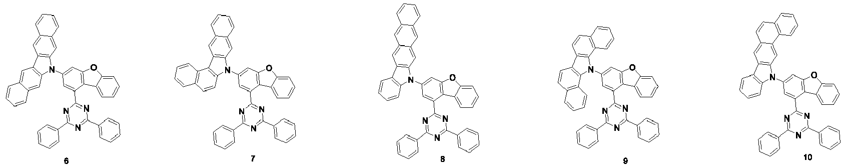

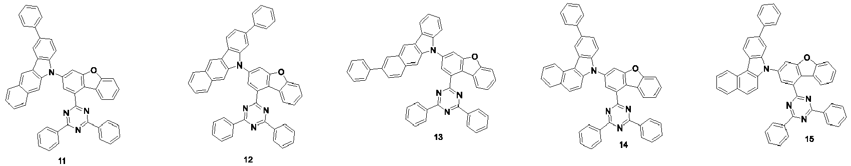

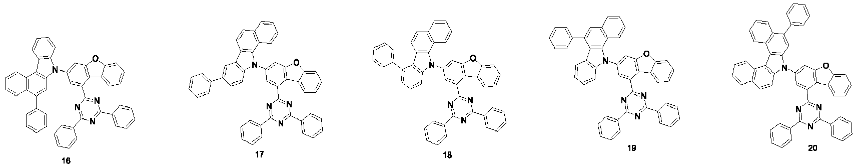

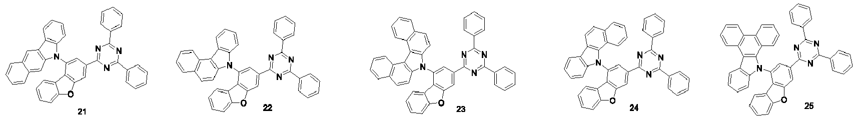

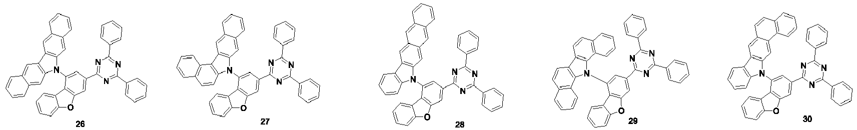

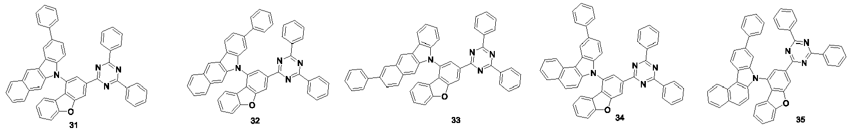

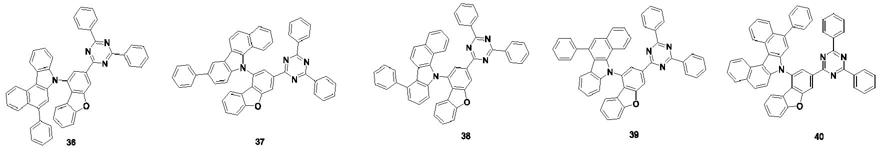

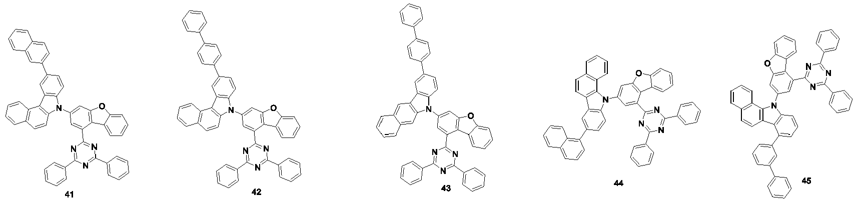

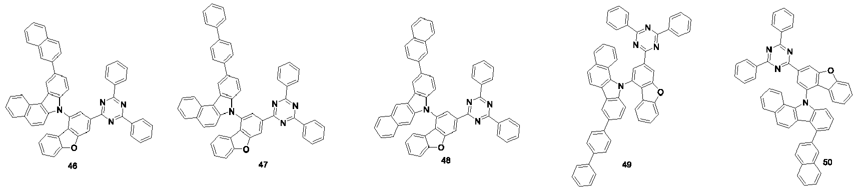

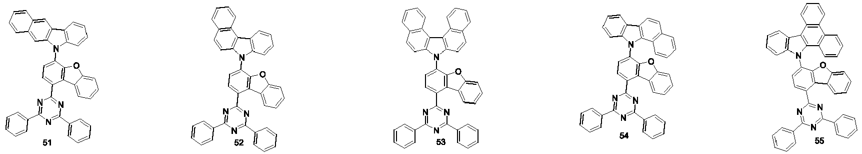

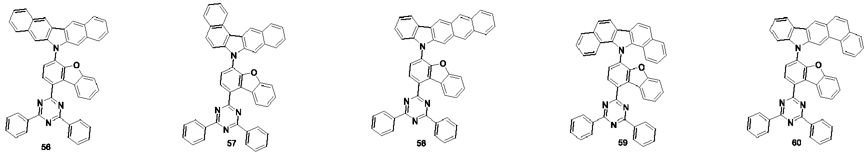

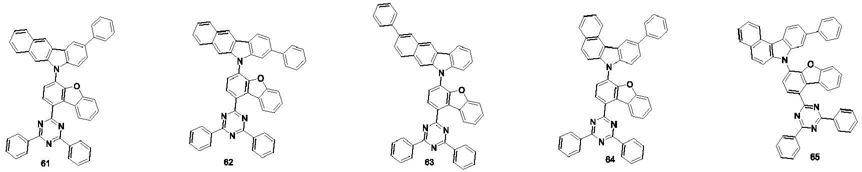

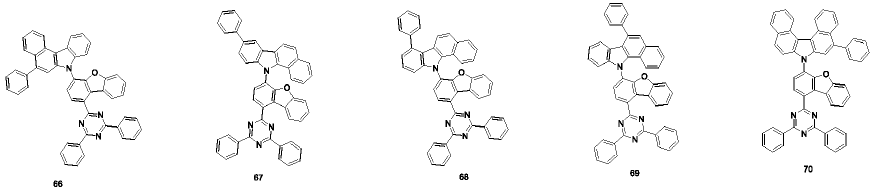

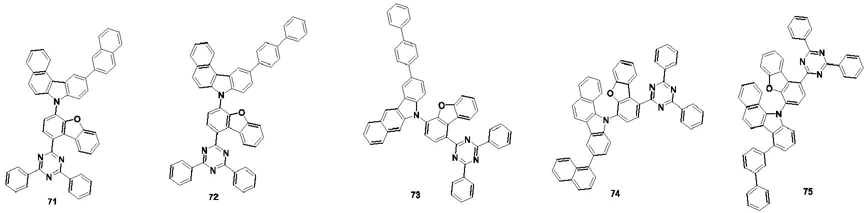

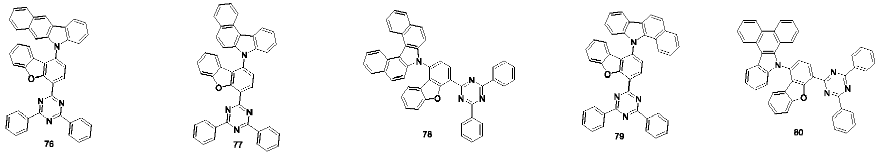

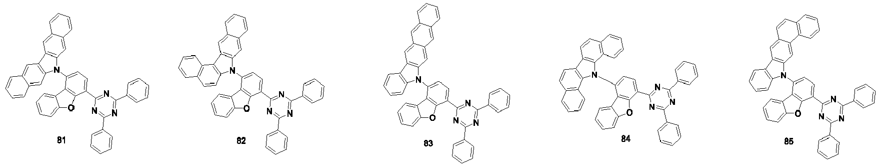

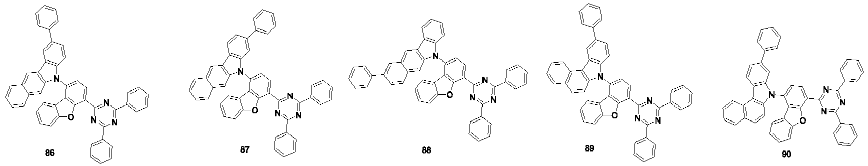

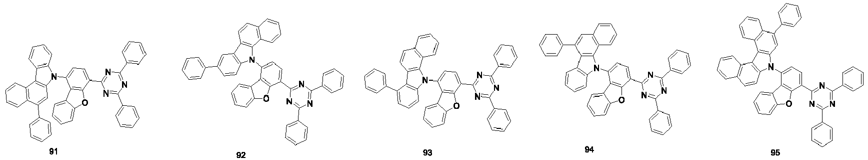

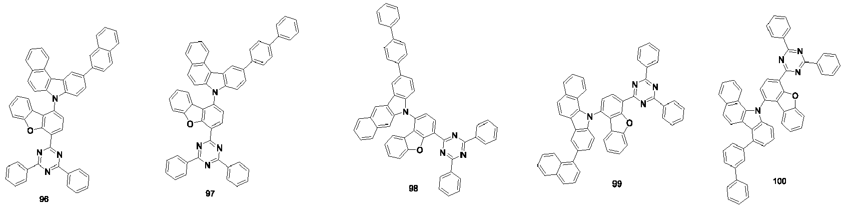

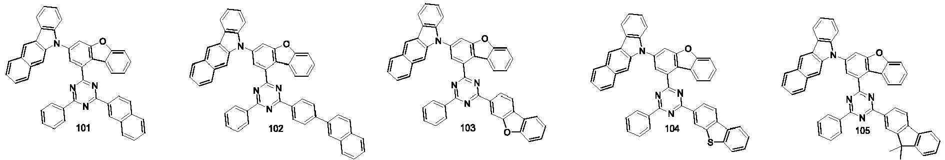

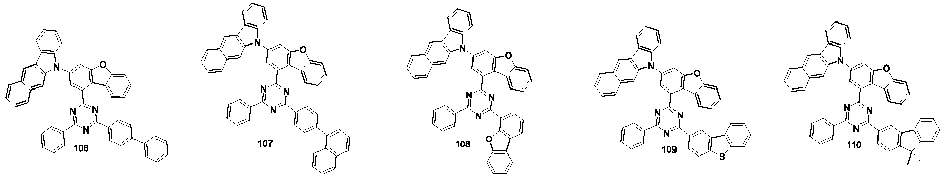

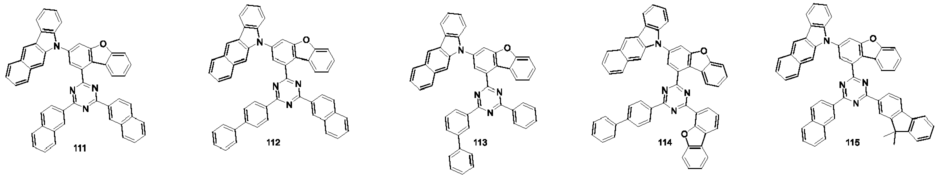

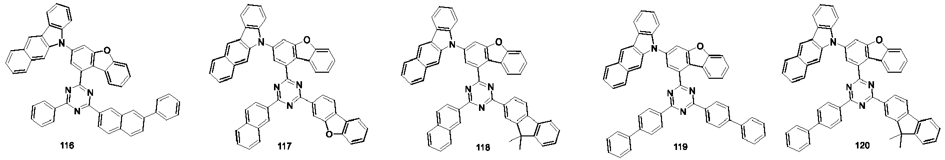

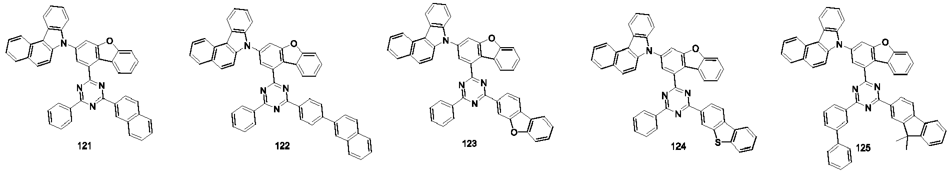

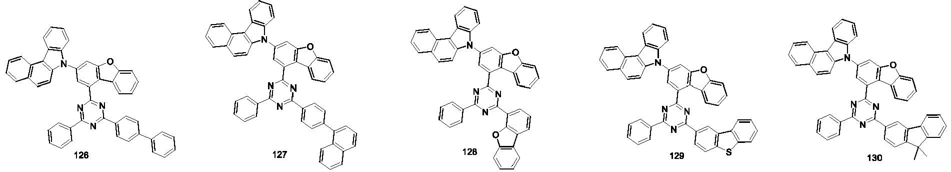

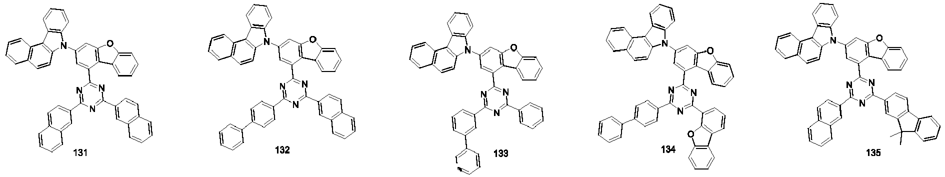

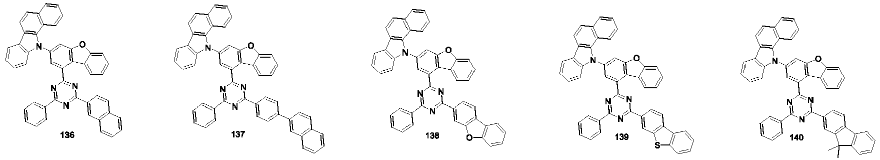

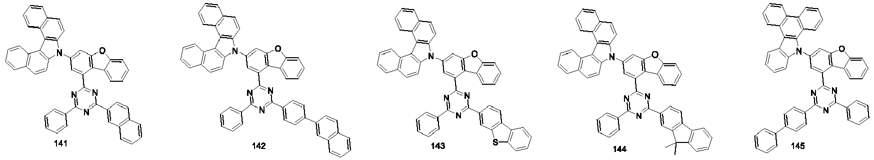

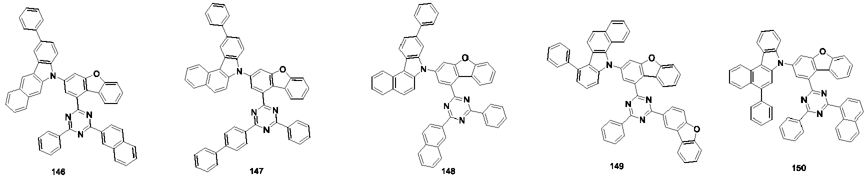

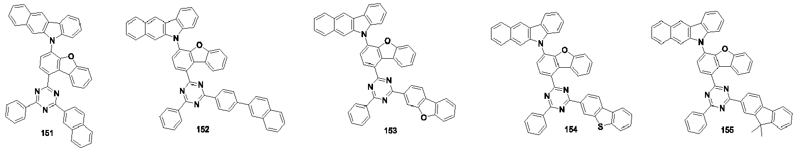

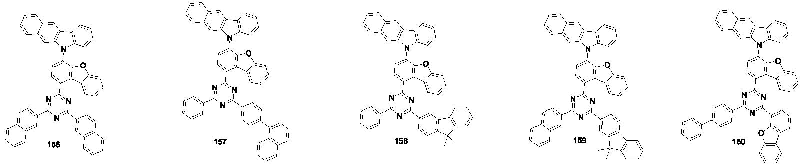

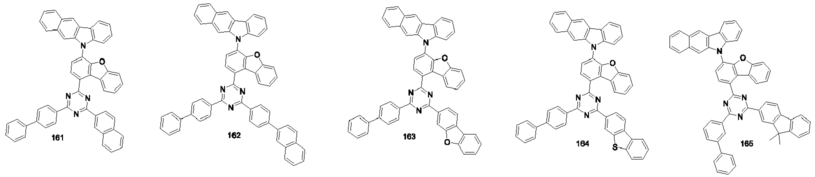

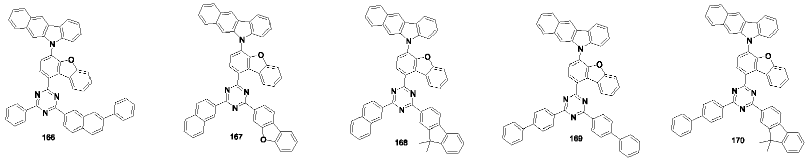

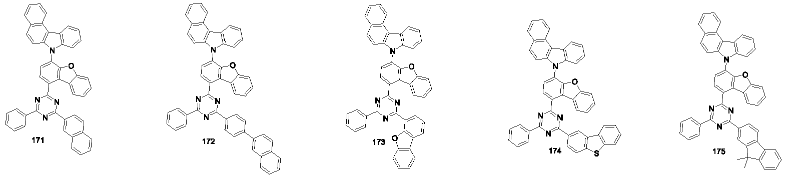

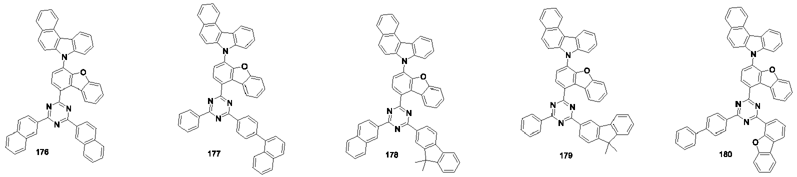

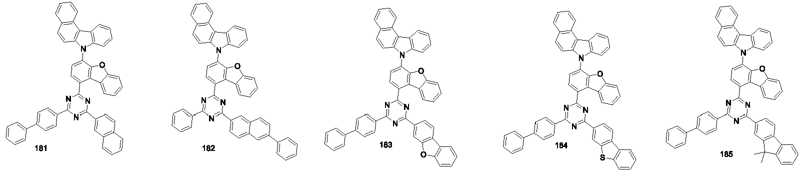

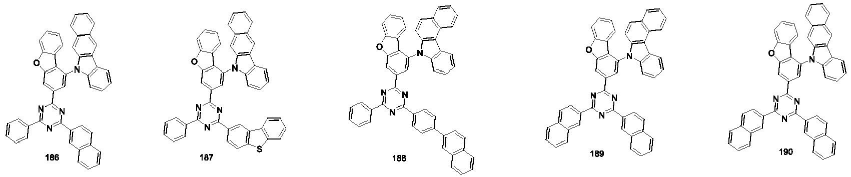

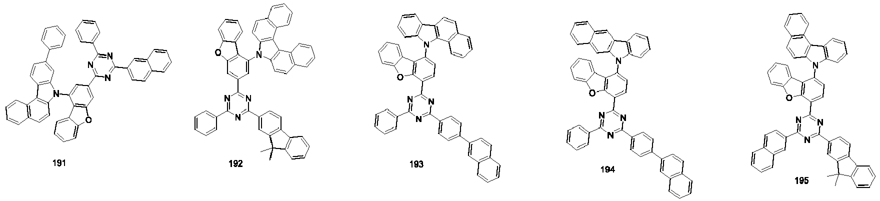

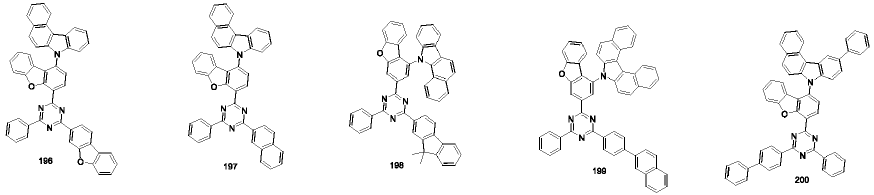

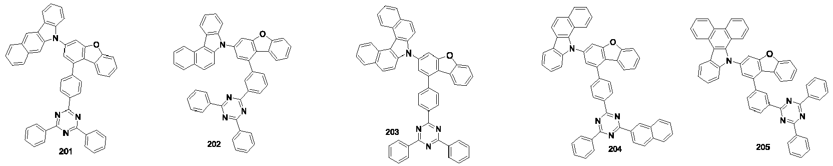

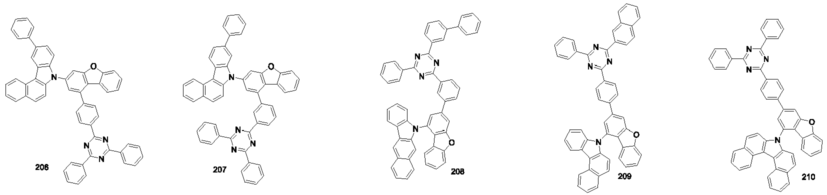

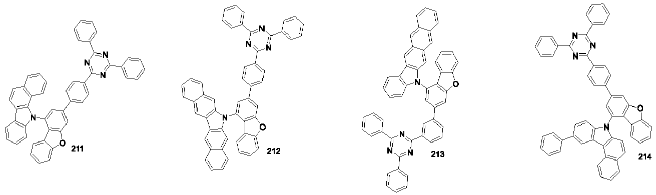

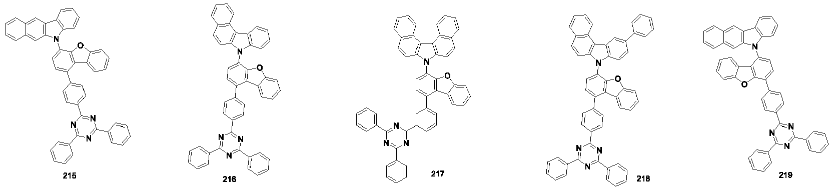

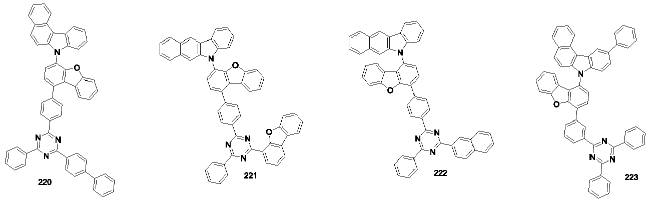

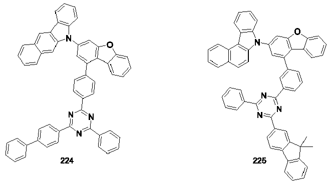

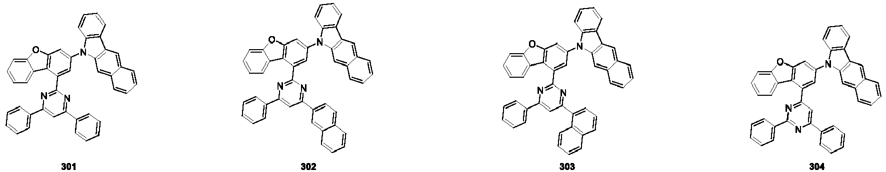

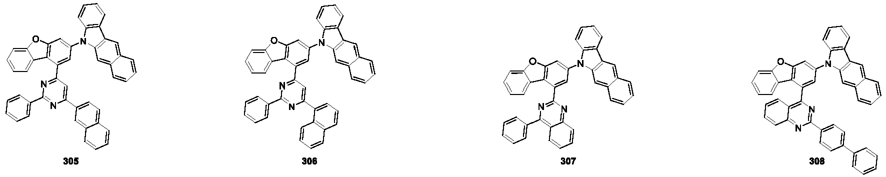

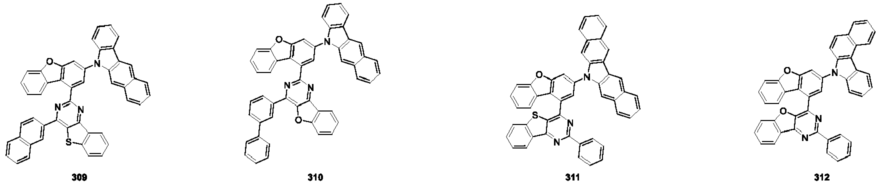

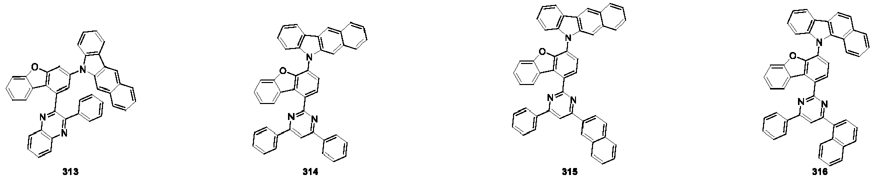

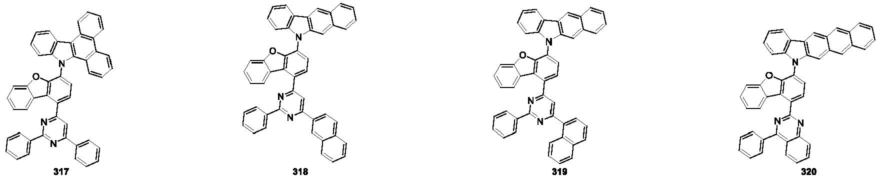

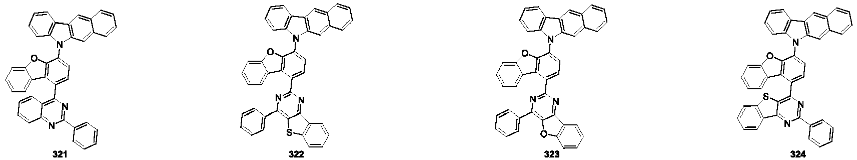

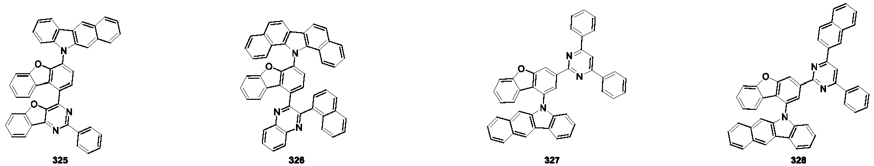

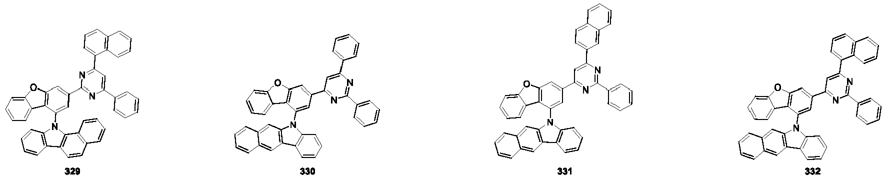

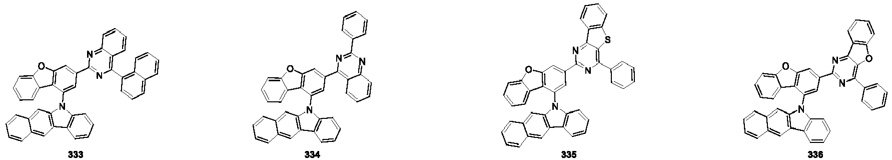

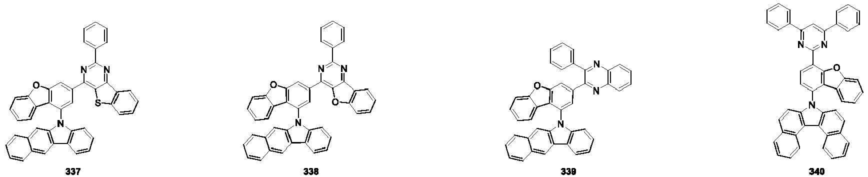

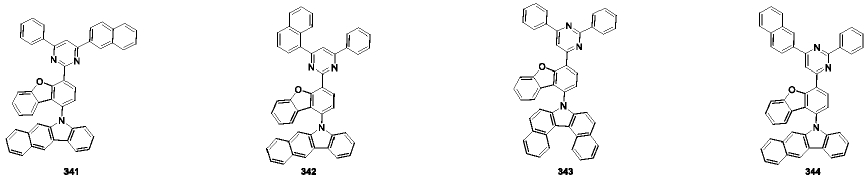

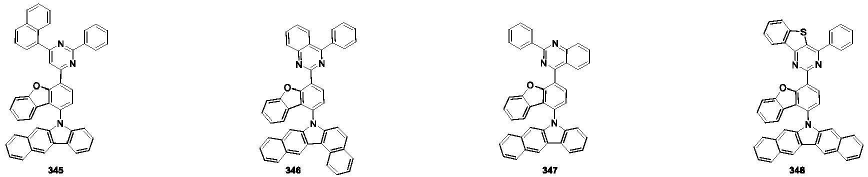

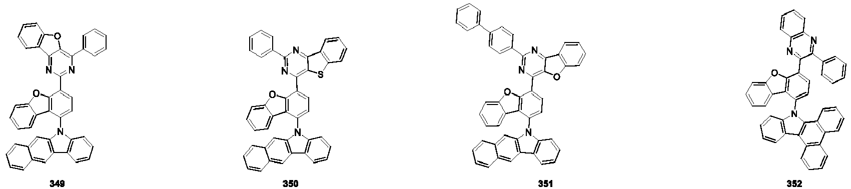

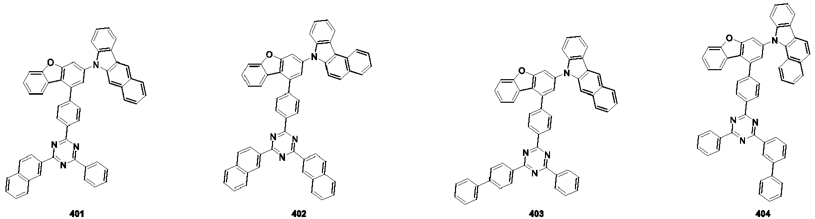

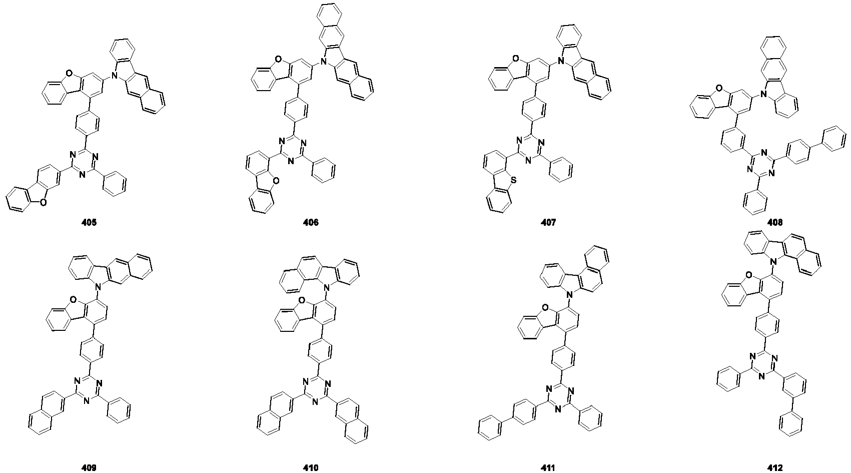

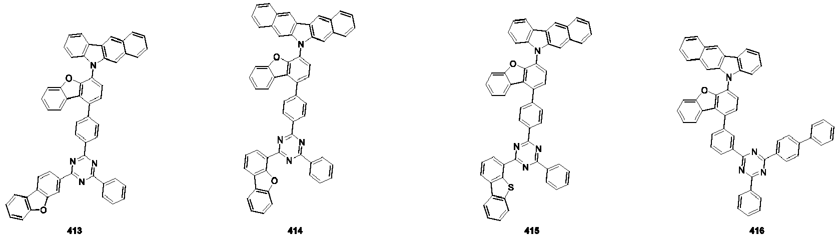

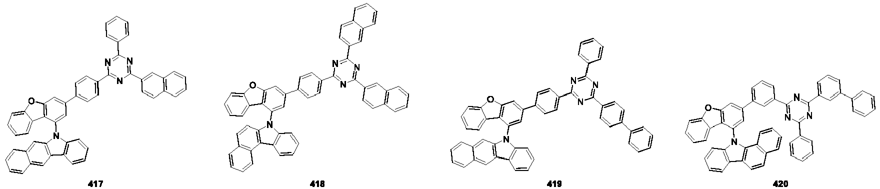

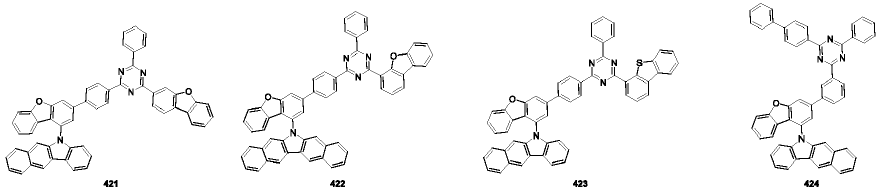

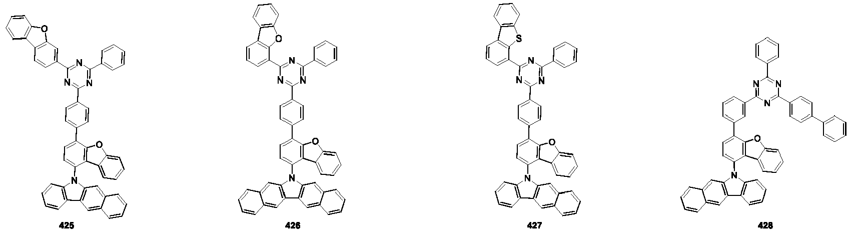

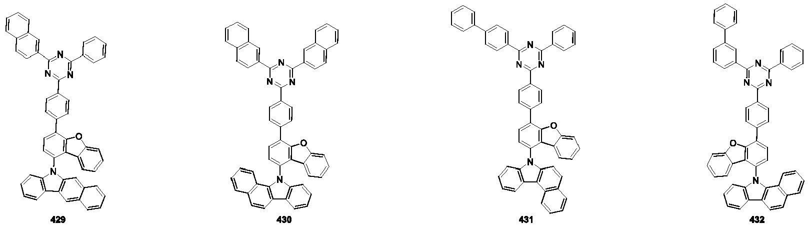

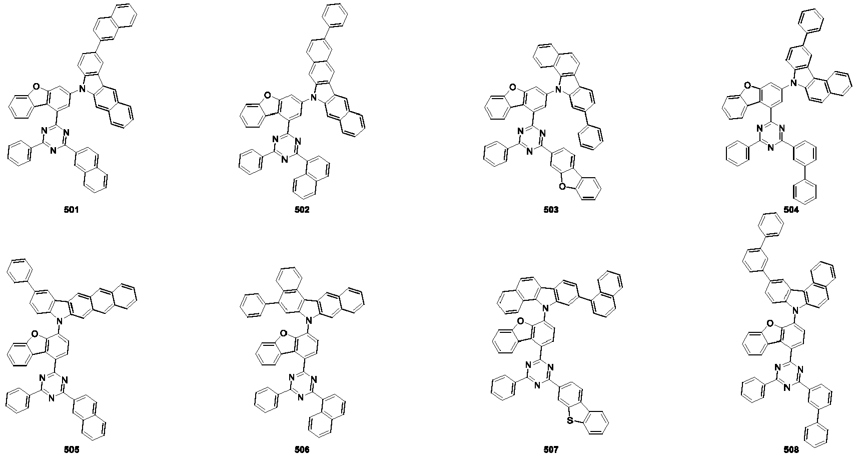

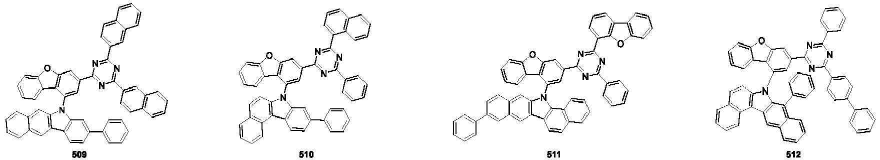

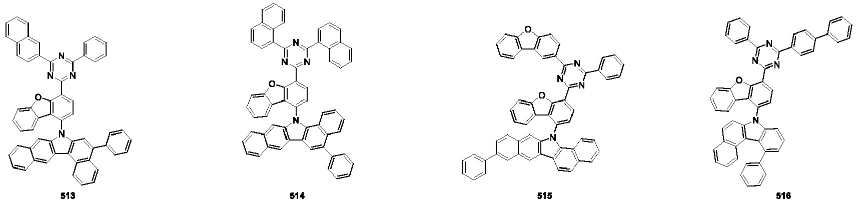

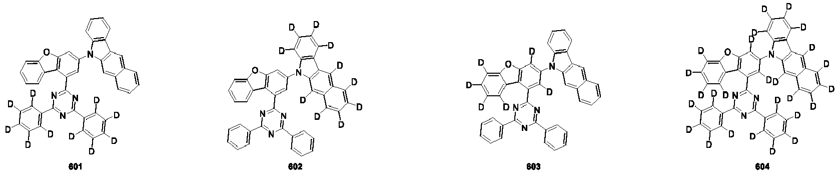

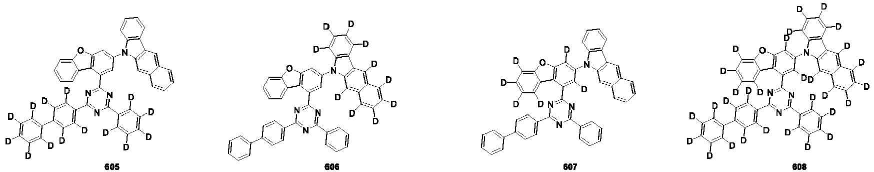

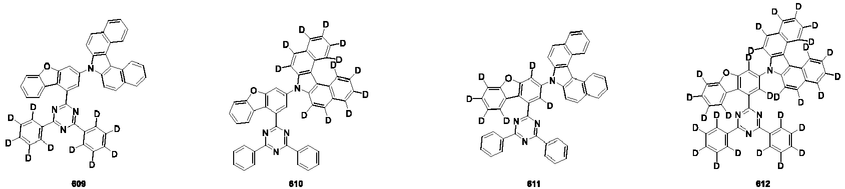

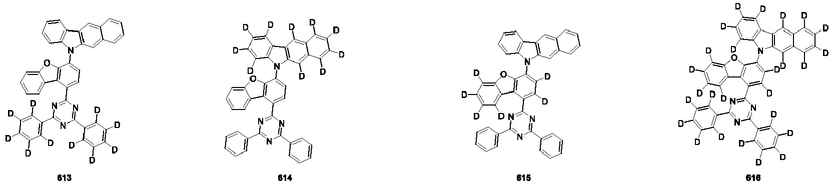

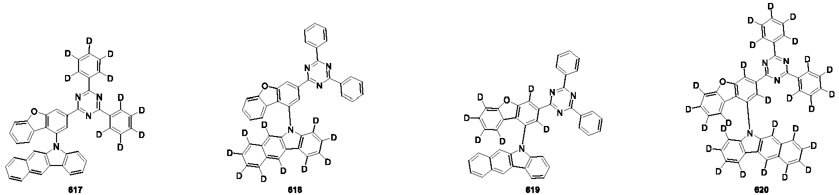

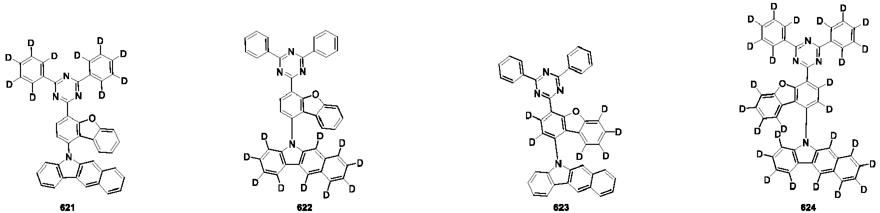

9. An organic light emitting device comprising:
a first electrode;
a second electrode provided opposite to the first electrode; and
one or more organic material layers provided between the first electrode and the second electrode,
wherein one or more layers of the organic material layers comprise one or more of the heterocyclic compound of any one of Claims 1 to 8.

10. The organic light emitting device of Claim 9, wherein the organic material layer comprises a light emitting layer, and the light emitting layer comprises the heterocyclic compound.

11. The organic light emitting device of Claim 9, wherein the organic material layer comprises a light emitting layer, the light emitting layer comprises a host material, and the host material comprises the heterocyclic compound.

12. The organic light emitting device of Claim 9, wherein the organic material layer comprises an electron injection layer or an electron transfer layer, and the electron injection layer or the electron transfer layer comprises the heterocyclic compound.

13. The organic light emitting device of Claim 9, wherein the organic material layer comprises an electron blocking layer or a hole blocking layer, and the electron blocking layer or the hole blocking layer comprises the heterocyclic compound.

14. The organic light emitting device of Claim 9, further comprising one, two or more layers selected from the group consisting of a light emitting layer, a hole injection layer, a hole transfer layer, an electron injection layer, an electron transfer layer, an electron blocking layer and a hole blocking layer.

15. The organic light emitting device of Claim 9, wherein the organic material layer further comprises a heterocyclic compound of the following Chemical Formula 2: 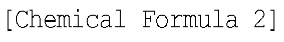 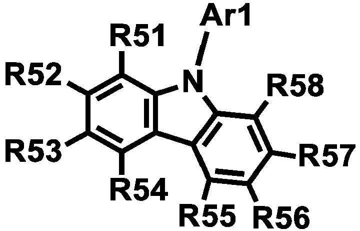 in Chemical Formula 2,
Ar1 is hydrogen; deuterium; a substituted or unsubstituted C1 to C60 alkyl group; a substituted or unsubstituted C6 to C60 aryl group; or a substituted or unsubstituted C2 to C60 heteroaryl group; and
R51 to R58 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted phosphine oxide group; and a substituted or unsubstituted amine group, or two or more groups adjacent to each other bond to each other to form a substituted or unsubstituted C6 to C60 aromatic hydrocarbon ring or a substituted or unsubstituted C2 to C60 heteroring.

16. The organic light emitting device of Claim 15, wherein Chemical Formula 2 is represented by any one of the following Chemical Formulae 10 to 12: 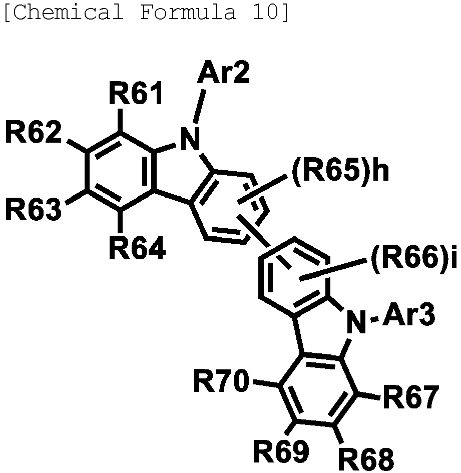 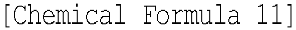 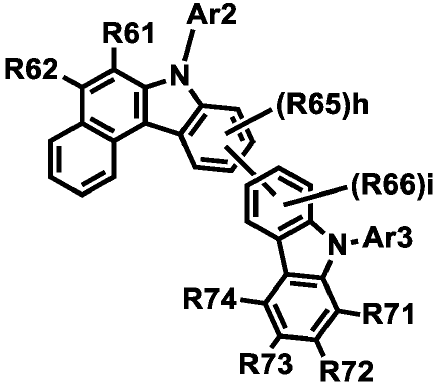 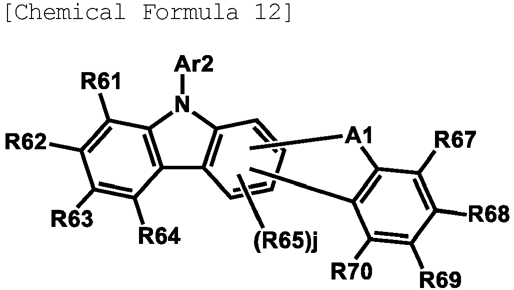 in Chemical Formulae 10 to 12,
R61 to R70 are the same as or different from each other, and each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group;
R71 to R74 are the same as or different from each other, and each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring;
Ar2 and Ar3 are the same as or different from each other, and each independently a substituted or unsubstituted C1 to C60 alkyl group; a substituted or unsubstituted C6 to C60 aryl group; or a substituted or unsubstituted C2 to C60 heteroaryl group;
A1 is O; S; NAr4; or CRdRe;
Rd and Re are the same as or different from each other, and each independently a substituted or unsubstituted C1 to C60 alkyl group; or a substituted or unsubstituted C6 to C60 aryl group;
Ar4 is a substituted or unsubstituted C1 to C60 alkyl group; a substituted or unsubstituted C6 to C60 aryl group; or a substituted or unsubstituted C2 to C60 heteroaryl group;
h and i are an integer of 0 to 3; and
j is an integer of 0 to 2.

17. The organic light emitting device of Claim 15, wherein the heterocyclic compound represented by Chemical Formula 2 is any one of the following compounds:

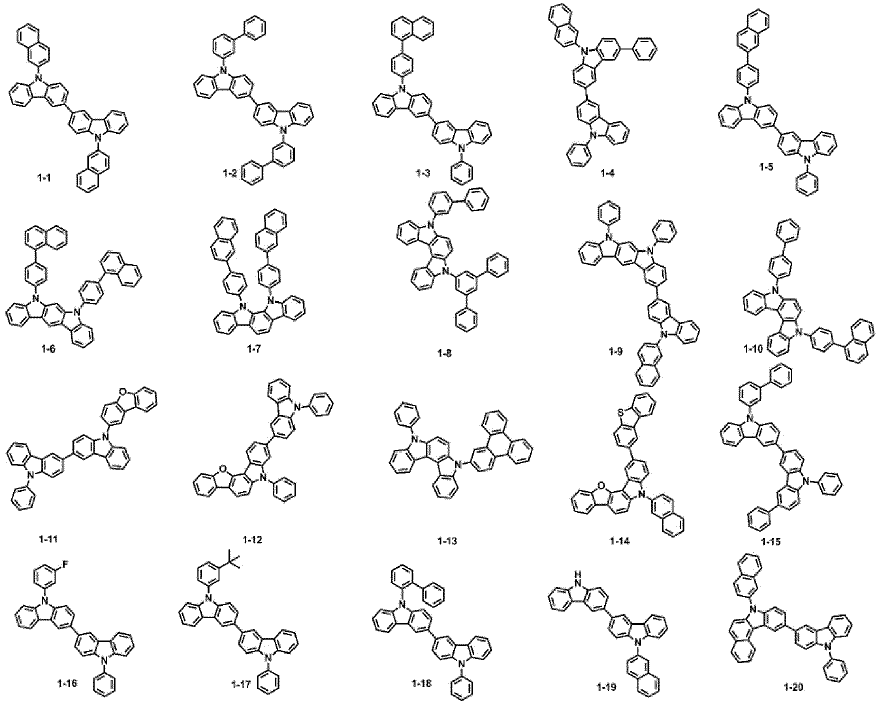

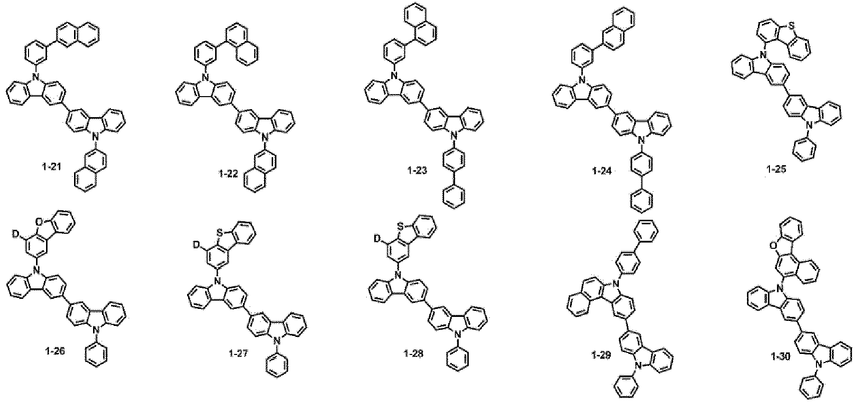

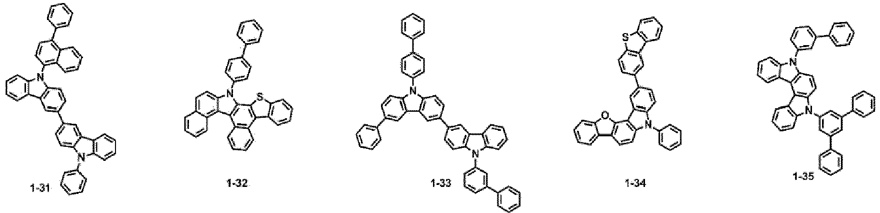

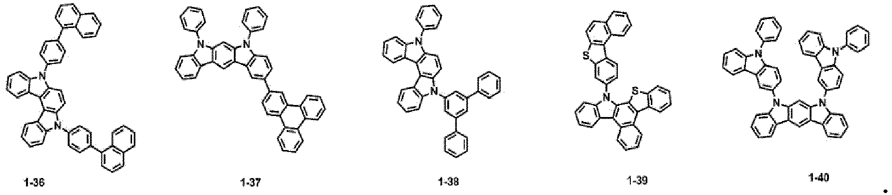

18. A composition for an organic material layer of an organic light emitting device, the composition comprising:
the heterocyclic compound represented by Chemical Formula 1 of any one of Claims 1 to 8; and
a heterocyclic compound represented by the following Chemical Formula 2: 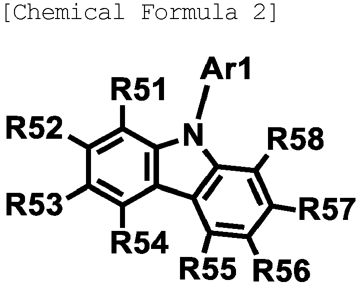 wherein, in Chemical Formula 2,
Ar1 is a substituted or unsubstituted C1 to C60 alkyl group; a substituted or unsubstituted C6 to C60 aryl group; or a substituted or unsubstituted C2 to C60 heteroaryl group; and
R51 to R58 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted phosphine oxide group; and a substituted or unsubstituted amine group, or two or more groups adjacent to each other bond to each other to form a substituted or unsubstituted C6 to C60 aromatic hydrocarbon ring or a substituted or unsubstituted C2 to C60 heteroring.

19. The composition for an organic material layer of an organic light emitting device of Claim 18, wherein, in the composition, the heterocyclic compound represented by Chemical Formula 1 and the heterocyclic compound represented by Chemical Formula 2 have a weight ratio of 1:10 to 10:1.

20. A method for manufacturing an organic light emitting device, the method comprising:
preparing a substrate;
forming a first electrode on the substrate;
forming one or more organic material layers on the first electrode; and
forming a second electrode on the organic material layer,
wherein the forming of organic material layers comprises forming one or more organic material layers using the composition for an organic material layer of Claim 18.

21. The method for manufacturing an organic light emitting device of Claim 20, wherein the forming of organic material layers is forming using a thermal vacuum deposition method after premixing the heterocyclic compound of Chemical Formula 1 and the heterocyclic compound of Chemical Formula 2.
